(19) Europäisches Patentamt
European Patent Office
Office européen des brevets

(11) Publication number: **0 411 912 B1**

# (12) EUROPEAN PATENT SPECIFICATION

(45) Date of publication of patent specification: **19.04.95**

(51) Int. Cl.[6]: **C07D 311/10**, C07D 311/16, C07D 413/12, A61K 31/35, //(C07D413/12,311:00,265:00)

(21) Application number: **90308441.6**

(22) Date of filing: **31.07.90**

## (54) Coumarin derivatives, their preparation and their use in the treatment of cerebrovascular disorders.

(30) Priority: **31.07.89 JP 198817/89**
**28.11.89 JP 309558/89**

(43) Date of publication of application:
**06.02.91 Bulletin 91/06**

(45) Publication of the grant of the patent:
**19.04.95 Bulletin 95/16**

(84) Designated Contracting States:
**AT BE CH DE DK ES FR GB GR IT LI LU NL SE**

(56) References cited:
**EP-A- 0 171 645**
**EP-A- 0 175 541**
**EP-A- 0 255 980**

(73) Proprietor: **Sankyo Company Limited**
**5-1 Nihonbashi Honcho 3-chome**
**Chuo-ku**
**Tokyo (JP)**

(72) Inventor: **Yanagisawa, Hiroaki c/o Sankyo Company Limited**
**2-58, 1-chome**
**Hiromachi**
**Shinagawa-ku**
**Tokyo (JP)**
Inventor: **Fujimoto, Koichi c/o Sankyo Company Limited**
**2-58, 1-chome**
**Hiromachi**
**Shinagawa-ku**
**Tokyo (JP)**
Inventor: **Shimoji, Yasuo c/o Sankyo Company Limited**
**2-58, 1-chome**
**Hiromachi**
**Shinagawa-ku**
**Tokyo (JP)**
Inventor: **Kanazaki, Takuro c/o Sankyo Company Limited**
**2-58, 1-chome**
**Hiromachi**
**Shinagawa-ku**
**Tokyo (JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

Inventor: **Iwata, Nobuyoshi C/o Sankyo Company Limited**
**2-58, 1-chome**
**Hiromachi**
**Shinagawa-ku**
**Tokyo (JP)**
Inventor: **Kubo, Yoshiko c/o Sankyo Company Limited**
**2-58, 1-chome**
**Hiromachi**
**Shinagawa-ku**
**Tokyo (JP)**

(74) Representative: **Gibson, Christian John Robert et al**
**MARKS & CLERK,**
**57/60 Lincoln's Inn Fields**
**London WC2A 3LS (GB)**

## Description

The present invention relates to the use of a certain series of 7-(substituted alkoxy)coumarin derivatives for the manufacture of a medicament for the treatment of cerebrovascular disorders and provides pharmaceutical compositions containing these compounds; it also provides, as new compounds, certain new and useful members of this series and provides processes for their preparation.

The compounds which are presently known for the treatment of cerebrovascular disorders can be classified into two groups; one group consists of compounds which improve cerebral circulation; and the other group consists of compounds which activate the cerebral metabolism. Compounds which improve the cerebral circulation, such as nicardipine and pentoxifylline, increase the cerebral blood flow by relaxing the smooth muscles of the cerebral blood vessels or by improving the cerebral microcirculation, which results in an improvement in any cerebral dysfunction. Compounds which activate the cerebral metabolism, such as tiapride and indeloxazine, improve mental symptoms by the activation of neurotransmitting monoamine compounds, such as serotonin, norepinephrine and dopamine. Some compounds having both of the activities mentioned above have been developed; however, no such compound has yet been discovered which exhibits sufficient clinical activity.

As a class, the 7-(substituted alkoxy)coumarin derivatives are known, and these compounds have been found to have certain limited therapeutic activities, although they have not hitherto been proposed for the treatment of cerebrovascular disorders. Known compounds include those disclosed in: Indian J. Appl. Chem., 32(1), 65 - 68 (1969) [see Chem. Abs., 75, 20107s (1971)]; Res. Commun. Chem. Pathol. Pharmacol., 9(3), 555 - 560 (1974) [see Chem. Abs., 82, 93490r (1975)]; UK Patent No. 1 524 260; Japanese Patent Application Kokai No. Sho. 45-23145; Indian J. Chem. Sect. B, 20B(2), 171 - 174 (1981) [see Chem. Abs., 95, 6981d (1981)]; Japanese Patent Application Kokai No. Sho. 42-4340; and PCT Publication No. WO 89/05289. Examples of compounds which are disclosed in these prior documents include those shown below as Compounds A to F.

Compound A:

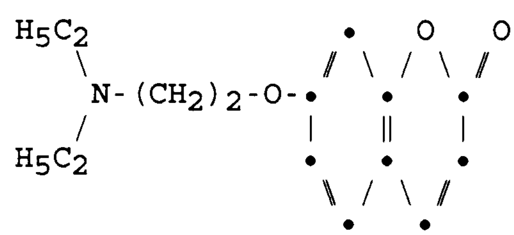

Compound B:

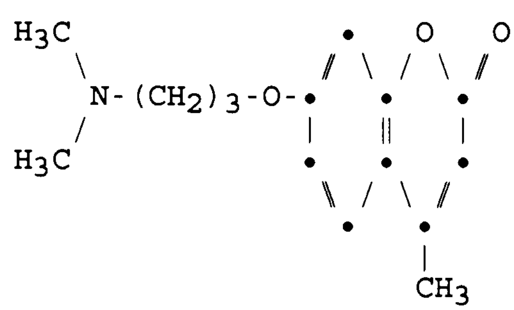

Compound C:

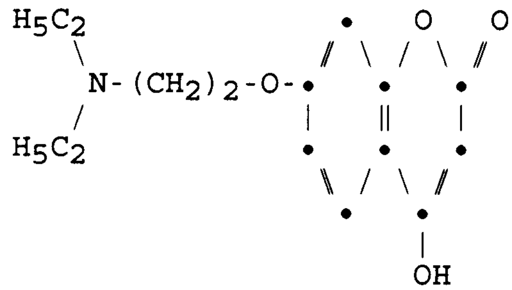

Compound D:

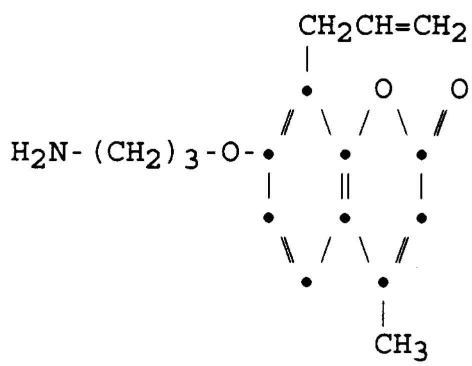

Compound E:

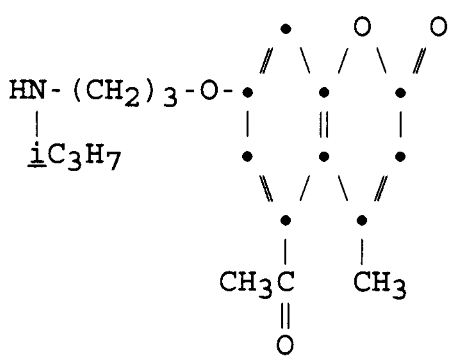

Compound F:

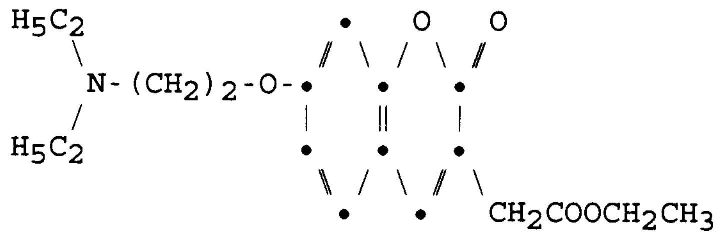

Of these prior documents, the most relevant is thought to be Res. Commun. Chem. Pathol. Pharmacol., 9(3), 555 - 560 (1974), which discloses certain compounds which we have now found to be useful in the treatment of cerebrovascular disorders, and whose use forms a part of the present invention. However, none of the prior art compounds has been disclosed to have any activity related to the activity with which the present invention is concerned.

We have now discovered that a series of 7-(substituted alkoxy)coumarin derivatives have the ability to improve ischaemia-induced hyperviscosity of the blood and to show antireserpine activity and can therefore be expected to be of value in the treatment of patients with cerebrovascular disorders, including, for example, senile dementia.

Thus, the compounds to which the present invention relates are those compounds of formula (I):

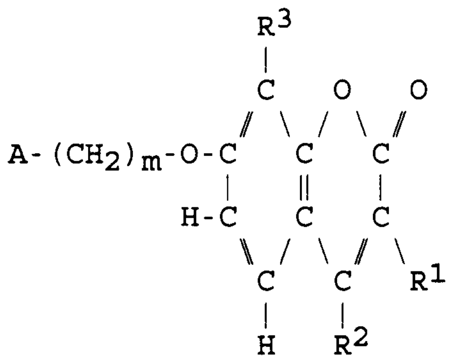

(I)

in which:

A represents a group of formula (II) or (III):

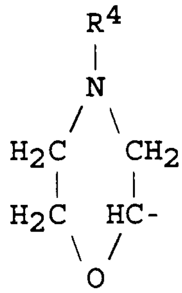

(II)

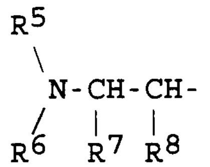

(III);

$R^1$, $R^2$ and $R^3$ are the same or different and each represents a hydrogen atom, an alkyl group having from 1 to 4 carbon atoms or a halogen atom;

$R^4$ represents a hydrogen atom or an alkyl group having from 1 to 4 carbon atoms, said alkyl group being unsubstituted or having at least one substituent selected from aryl groups having from 6 to 10 carbon atoms, where said aryl group is unsubstituted or is substituted by at least one of substituents (a), defined below;

$R^5$ and $R^6$ are the same or different and each represents a hydrogen atom or an alkyl group having from 1 to 4 carbon atoms;

$R^7$ and $R^8$ are the same or different and each represents a hydrogen atom or an alkyl group having from 1 to 4 carbon atoms; or $R^7$ and $R^8$ together represent an alkylene group having 3 or 4 carbon atoms; and

$\underline{m}$ is 1 or 2;

substituents (a):

alkyl groups having from 1 to 4 carbon atoms, alkoxy groups having from 1 to 4 carbon atoms, trifluoromethyl groups, nitro groups, cyano groups and halogen atoms;

and pharmaceutically acceptable salts thereof.

In its broadest aspect, the present invention provides the use of the compounds of formula (I) defined above and salts thereof for the manufacture of a medicament for the treatment of cerebrovascular disorders.

All of the compounds of formula (I) defined above are new except certain of those in which A represents a group of formula (III). Accordingly, the invention also provides as new compounds those compounds of formula (I) defined above, and pharmaceutically acceptable salts thereof, provided that $R^5$ and $R^6$ are not identical and either a methyl or an ethyl group when: A represents a group of formula (III), and $R^1$, $R^3$, $R^7$ and $R^8$ all represent hydrogen atoms, and $R^2$ represents a methyl group and $\underline{m}$ is 1.

The invention also provides a pharmaceutical composition for the treatment of cerebrovascular disorders comprising a therapeutically effective amount of at least one compound of formula (I) or a pharmaceutically acceptable salt thereof in admixture with a pharmaceutically acceptable carrier, diluent or excipient.

The invention also provides a process for preparing the novel compounds of the present invention, which process is described more fully hereafter.

Of the compounds of the present invention, one class of compounds may be represented by the formula (Ia):

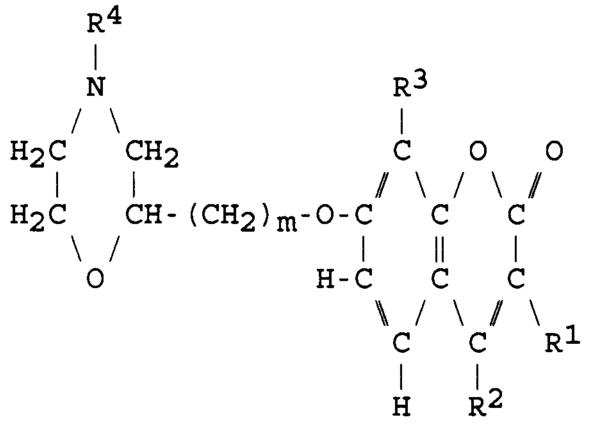

(Ia)

in which $R^1$, $R^2$, $R^3$, $R^4$ and m are as defined above, and m may be, for example, 1.

Another class of compounds of the present invention are those compounds having the formula (Ib):

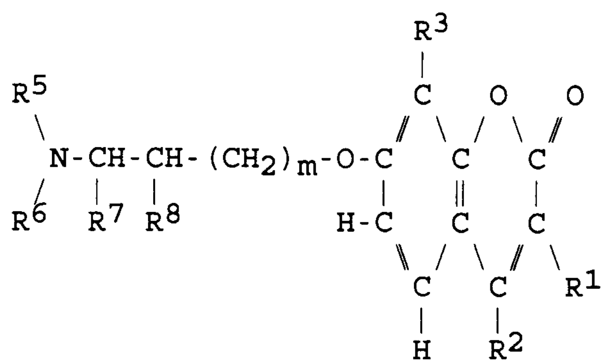

(Ib)

in which $R^1$, $R^2$, $R^3$, $R^5$, $R^6$, $R^7$, $R^8$ and m are as defined above, and $R^7$ and $R^8$ may be, for example, hydrogen atoms.

In the compounds of the present invention, where $R^1$, $R^2$, $R^3$, $R^4$, $R^5$, $R^6$, $R^7$ or $R^8$ represents an alkyl group, this has from 1 to 4 carbon atoms and may be a straight or branched chain group. Examples of such groups include the methyl, ethyl, propyl, isopropyl, butyl, isobutyl, sec-butyl and t-butyl groups, of which the methyl group is preferred.

Where $R^1$, $R^2$ or $R^3$ represents a halogen atom, this may be a fluorine, chlorine, bromine or iodine atom, preferably a fluorine or chlorine atom.

Where $R^4$ represents an alkyl group having an aryl substituent, i.e. an aralkyl group, the alkyl part may be any of those alkyl groups listed above, but is preferably a methyl, ethyl, propyl or isopropyl group, more preferably a methyl or ethyl group and most preferably a methyl group. The aryl part has from 6 to 10 ring atoms and is preferably a phenyl, $\alpha$-naphthyl or $\beta$-naphthyl group, more preferably a phenyl group. Especially where the aryl part is a phenyl group, there may be 2 or more such aryl parts, but 1 such aryl part is preferred. Examples of the unsubstituted aralkyl groups include the benzyl, phenethyl, 1-phenylethyl, 3-phenylpropyl, 2-phenylpropyl, benzhydryl and $\alpha$-naphthylmethyl groups, of which the benzyl, phenethyl and 3-phenylpropyl groups are preferred and the benzyl group is most preferred. Such groups may be unsubstituted or they may be substituted by at least one of substituents (a), defined above. Examples of substituents (a) include the alkyl groups exemplified above, alkoxy groups corresponding to those exemplified alkyl groups, trifluoromethyl groups, nitro groups, cyano groups and halogen atoms. However, the unsubstituted groups are preferred.

Where $R^7$ and $R^8$ together represent an alkylene group having 3 or 4 carbon atoms, the group of formula (III) thus represents a cyclopentyl ($C_3$ alkylene) or cyclohexyl ($C_4$ alkylene) group having a 2-amino substituent.

All of the compounds of the present invention where A represents a group of formula (II) are believed to be novel and these compounds, which include those of formula (Ia), form per se part of the present invention. Those compounds of formula (I) where A represents a group of formula (III) and $R^5$ and $R^6$ are

the same and either a methyl or an ethyl group, $R^1$, $R^3$, $R^7$ and $R^8$ all represent hydrogen atoms, $R^2$ represents a methyl group and m is 1 are not claimed per se, but have now been found for the first time to be useful for the treatment of cerebrovascular disorders.

Of the compounds of formula (Ia), the preferred compounds are:

(A) those compounds in which $R^1$ represents a hydrogen atom, a methyl group, an ethyl group, a propyl group, an isopropyl group, a fluorine atom or a chlorine atom;

(B) those compounds in which $R^2$ represents a hydrogen atom, a methyl group, an ethyl group, a propyl group or an isopropyl group;

(C) those compounds in which $R^3$ represents a hydrogen atom, a methyl group, an ethyl group or an isopropyl group;

(D) those compounds in which $R^4$ represents a hydrogen atom;

and especially preferred of these compounds are those in which:

(E) $R^1$ represents a hydrogen atom, a methyl group, a fluorine atom or a chlorine atom; $R^2$ and $R^3$ are the same or different and each represents a hydrogen atom or a methyl group; and $R^4$ represents a hydrogen atom;

(F) m is 1.

Of the compounds of formula (Ib), the preferred compounds are:

(G) those compounds in which both $R^5$ and $R^6$ represent hydrogen atoms;

(H) those compounds in which $R^5$ represents a hydrogen atom and $R^6$ represents an alkyl group containing from 1 to 4 carbon atoms; more preferred are those compounds in which $R^5$ represents a hydrogen atom and $R^6$ represents a methyl group;

(I) those compounds in which $R^5$ and $R^6$ are the same or different and each represents a methyl or ethyl group;

(J) those compounds in which $R^1$ represents a hydrogen atom, a chlorine atom or a methyl group;

(K) those compounds in which $R^2$ represents a hydrogen atom or a methyl group;

(L) those compounds in which $R^3$ represents a hydrogen atom or a methyl group.

More preferred compounds of formula (Ib) are:

(M) those compounds wherein:

$R^1$, $R^2$ and $R^3$ all represent methyl groups;

$R^5$ and $R^6$ both represent hydrogen atoms; and

$R^7$ and $R^8$ are the same or different and each represents a hydrogen atom, a methyl group, an ethyl group or an isopropyl group.

(N) those compounds wherein:

$R^1$, $R^2$ and $R^3$ all represent methyl groups;

$R^5$ represents a hydrogen atom;

$R^6$ represents a methyl group; and

$R^7$ and $R^8$ are the same or different and each represents a hydrogen atom, a methyl group, an ethyl group or an isopropyl group.

(O) those compounds wherein:

$R^1$, $R^2$ and $R^3$ all represent methyl groups;

$R^5$ and $R^6$ both represent methyl groups; and

$R^7$ and $R^8$ are the same or different and each represents a hydrogen atom, a methyl group, an ethyl group or an isopropyl group.

Although the compounds of the present invention are all represented herein by a single general formula (I), the compounds may exist in the form of various isomers, depending on the nature of the substituent groups thereon. In particular, the compounds of formula (Ia) may exist as two kinds of optical isomers, the (R) form and the (S) form, because of the asymmetric carbon atom contained in the 2-morpholinylmethoxy group; also, the compounds of formula (Ib) may exist as two or four kinds of optical isomers, e.g. the (R) form and the (S) form, because of the asymmetric carbon atom or atoms when $R^7$ and/or $R^8$ represents an alkyl group. The present invention embraces both the individual isolated isomers as well as mixtures thereof, including racemic mixtures (which may be formed from equimolar mixtures of reagents). As is well known in the art, sometimes one of a pair of isomers exhibits greater activity or other more desirable characteristics than another of the pair, and, in such a case, use of the better isomer alone may be desirable. Otherwise, if desired, the compounds may be employed as a mixture of two or more isomers, and no disadvantage will thereby result. Where individual isomers are required, these may be formed by stereospecific synthesis techniques as explained hereafter and illustrated in the following Examples. Alternatively, mixtures of the compounds of the present invention may be formed and then separated by conventional resolution techniques, such as are well understood in the art.

The compounds of the present invention contain a basic nitrogen atom and can, therefore form salts with suitable acids. There is, in principle, no restriction on the nature of the acids used to form such salts. However, where the resulting salt is intended for therapeutic use, it is necessary that the salt should be pharmaceutically acceptable, which, as is well known in the art means that it should not have reduced activity (or unacceptably reduced activity) or increased toxicity (or unacceptably increased toxicity) as compared to the free base. Where, however, the salt is to be used for some other purpose, e.g. as an intermediate in the production of another, and possibly more active, compound, even this restriction does not apply. Examples of suitable acids include: inorganic acids, such as hydrochloric acid, hydrobromic acid, sulphuric acid, nitric acid and phosphoric acid; organic carboxylic acids, such as oxalic acid, lactic acid, citric acid, tartaric acid, succinic acid, maleic acid and fumaric acid; and organic sulphonic acids, such as methanesulphonic acid.

Examples of specific compounds of the invention are given in the following formulae (I-1) to (I-3), in which the substituents are as defined in the corresponding one of Tables 1 to 3, respectively [i.e. Table 1 relates to formula (I-1), Table 2 relates to formula (I-2) and Table 3 relates to formula (I-3).

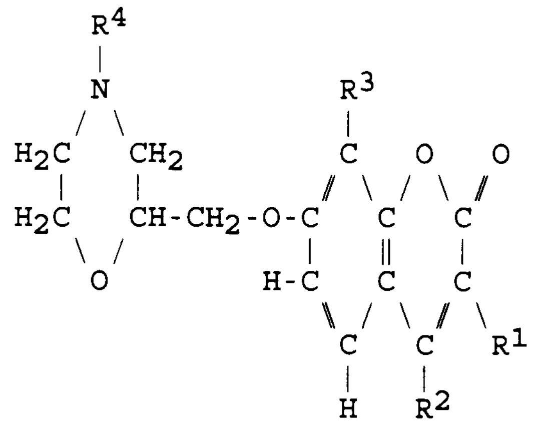

(I-1)

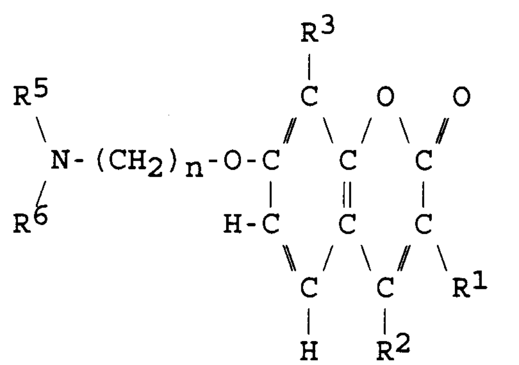

(I-2)

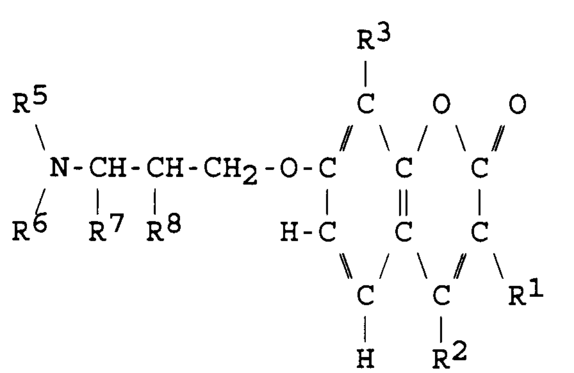

(I-3)

In Table 3, Compounds 3-11 and 3-12, $R^7$ and $R^8$ together represent either a trimethylene or tetramethylene group, respectively, to form with the carbon atoms to which they are attached either a cyclopentane or cyclohexane ring.

In the following Tables, certain abbreviations are used for the sake of brevity, and these abbreviations have the following meanings:

| | |
|---|---|
| Bu | butyl |
| Et | ethyl |
| Me | methyl |
| iPr | isopropyl |

Table 1

| Cpd No. | $R^1$ | $R^2$ | $R^3$ | $R^4$ |
|---|---|---|---|---|
| 1-1 | H | H | H | H |
| 1-2 | H | Me | H | H |
| 1-3 | H | Me | Me | H |
| 1-4 | Me | Me | Me | H |
| 1-5 | Me | Me | H | H |
| 1-6 | Cℓ | Me | H | H |
| 1-7 | Me | Me | Me | Me |
| 1-8 | Et | Me | Me | H |
| 1-9 | Et | Me | H | H |
| 1-10 | iPr | Me | Me | H |
| 1-11 | iPr | Me | H | H |
| 1-12 | Bu | Me | Me | H |
| 1-13 | Bu | Me | H | H |
| 1-14 | H | Pr | H | H |

Table 2

| Cpd No. | $R^1$ | $R^2$ | $R^3$ | $R^5$ | $R^6$ | n |
|---|---|---|---|---|---|---|
| 2-1 | Me | Me | Me | H | Me | 3 |
| 2-2 | Me | Me | Me | H | Me | 4 |
| 2-3 | Cℓ | Me | Me | H | Me | 3 |
| 2-4 | Cℓ | Me | Me | H | Me | 4 |
| 2-5 | Me | Me | Me | H | Et | 3 |
| 2-6 | Me | Me | Me | H | Et | 4 |
| 2-7 | Me | Me | H | H | Me | 3 |
| 2-8 | Me | Me | H | H | Me | 4 |
| 2-9 | H | Me | Me | H | Me | 3 |
| 2-10 | H | Me | Me | H | Me | 4 |
| 2-11 | H | Me | H | H | Me | 3 |
| 2-12 | H | Me | H | H | Me | 4 |
| 2-13 | H | H | Me | H | Me | 3 |
| 2-14 | H | H | Me | H | Me | 4 |
| 2-15 | Me | H | H | H | Me | 3 |
| 2-16 | Me | H | H | H | Me | 4 |
| 2-17 | H | H | H | H | Me | 3 |
| 2-18 | H | H | H | H | Me | 4 |
| 2-19 | Me | Me | Me | H | iPr | 3 |
| 2-20 | Me | Me | Me | H | iPr | 4 |
| 2-21 | F | Me | Me | H | Me | 3 |
| 2-22 | F | Me | H | H | Me | 3 |
| 2-23 | Me | Me | Me | Me | Me | 3 |
| 2-24 | Me | Me | Me | Me | Me | 4 |
| 2-25 | F | Me | Me | Me | Me | 3 |
| 2-26 | Me | Me | Me | Me | Et | 3 |
| 2-27 | Me | Me | Me | Et | Et | 3 |
| 2-28 | Me | Me | H | Me | Me | 3 |

Table 2 (cont)

| Cpd No. | $R^1$ | $R^2$ | $R^3$ | $R^5$ | $R^6$ | *n* |
|---|---|---|---|---|---|---|
| 2-29 | Me | Me | H | Me | Me | 4 |
| 2-30 | Me | Me | Me | H | H | 3 |
| 2-31 | Me | Me | Me | H | H | 4 |
| 2-32 | Me | Me | H | H | H | 3 |
| 2-33 | Me | Me | H | H | H | 4 |

Table 3

| Cpd No. | $R^1$ | $R^2$ | $R^3$ | $R^5$ | $R^6$ | $R^7$ | $R^8$ |
|---|---|---|---|---|---|---|---|
| 3-1 | Me | Me | Me | H | H | Me | H |
| 3-2 | Me | Me | Me | H | H | H | Me |
| 3-3 | Me | Me | Me | H | H | Et | H |
| 3-4 | Me | Me | Me | H | H | Et | Et |
| 3-5 | Me | Me | Me | H | H | iPr | H |
| 3-6 | Me | Me | Me | H | H | H | iPr |
| 3-7 | Me | Me | Me | H | Me | Me | H |
| 3-8 | Me | Me | Me | Me | Me | Me | H |
| 3-9 | Me | Me | Me | H | Me | Me | Me |
| 3-10 | Me | Me | Me | H | Me | Et | H |
| 3-11 | Me | Me | Me | H | H | $-C_3H_6-$ | |
| 3-12 | Me | Me | Me | H | H | $-C_4H_8-$ | |
| 3-13 | Me | Me | H | H | H | Me | H |
| 3-14 | H | Me | Me | H | H | Me | H |
| 3-15 | H | H | H | H | H | Me | H |
| 3-16 | H | Me | H | H | H | Me | H |
| 3-17 | H | Et | H | H | H | Me | H |
| 3-18 | Et | Me | H | H | H | Me | H |
| 3-19 | Et | Me | Me | H | H | Me | H |
| 3-20 | H | Me | Et | H | H | Me | H |
| 3-21 | Me | Me | Et | H | H | Me | H |
| 3-22 | Me | Me | Me | H | H | H | Et |
| 3-23 | Me | Me | Me | H | H | Me | Et |

Of the compounds listed above, the following compounds are preferred, that is to say Compounds No. 1-1, 1-2, 1-3, 1-4, 1-5, 1-7, 2-1, 2-2, 2-5, 2-7, 2-23, 2-24, 2-28, 2-30, 2-31, 2-32, 3-1, 3-2, 3-5, 3-7, 3-8 and 3-11, and the following are more preferred: Compounds No. 1-1, 1-2, 1-4, 1-5, 1-7, 2-1, 2-23, 2-30, 3-1, 3-5, 3-8 and 3-11.

The most preferred compounds are Compounds No.:

1-4. 3,4,8-Trimethyl-7-(2-morpholinyl)methoxycoumarin;

1-5. 3,4-Dimethyl-7-(2-morpholinyl)methoxycoumarin;

1-7. 3,4,8-Trimethyl-7-(4-methyl-2-morpholinyl)methoxycoumarin;

2-23. 7-(3-N,N-Dimethylaminopropoxy)-3,4,8-trimethylcoumarin;

2-30. 7-(3-Aminopropoxy)-3,4,8-trimethylcoumarin; and

3-1. 7-(3-Aminobutoxy)-3,4,8-trimethylcoumarin.

Also preferred are pharmaceutically acceptable salts of the above compounds, especially the hydrochlorides and fumarates.

The compounds of the present invention may be prepared by a variety of processes of the type well known in the art for the preparation of compounds of this kind. For example, in general terms, the compounds may be prepared by:

(a) reacting a compound of formula (IV):

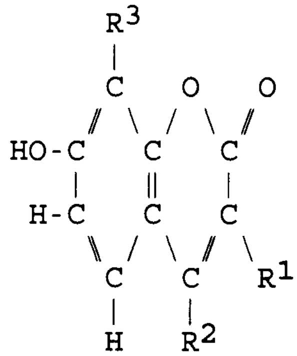

(IV)

(in which: $R^1$, $R^2$ and $R^3$ are as defined above) with a compound of formula (V):

$Y-(CH_2)_m-A'$ (V)

(in which: m is as defined above; Y represents a halogen atom, a sulphonyloxy group or a hydroxy group; and A' represents a group of formula (II') or (III'):

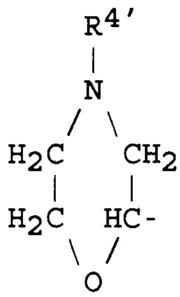

(II')

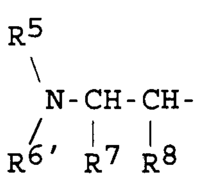

(III')

(in which: $R^5$, $R^7$ and $R^8$ are as defined above;

$R^{4'}$ represents an amino-protecting group or an alkyl group having from 1 to 4 carbon atoms, said alkyl group being unsubstituted or having at least one substituent selected from aryl groups having from 6 to 10 carbon atoms, where said aryl group is unsubstituted or is substituted by at least one of substituents

(a), defined above; and $R^{6'}$ represents an amino-protecting group or an alkyl group having from 1 to 4 carbon atoms); and

if required, one of the steps (b) or (c):

(b) where A' represents said group of formula (II') and $R^{4'}$ represents an amino-protecting group or represents said group of formula (III') and $R^{6'}$ represents an amino-protecting group, removing the amino-protecting group;

(c) where the product is a compound in which one or both of $R^5$ and $R^6$ represents a hydrogen atom, alkylating the hydrogen atom to convert it to an alkyl group having from 1 to 4 carbon atoms;

and optionally salifying the product.

In more detail, preferred processes of the present invention may be illustrated by the following reaction schemes:

Reaction Scheme A:

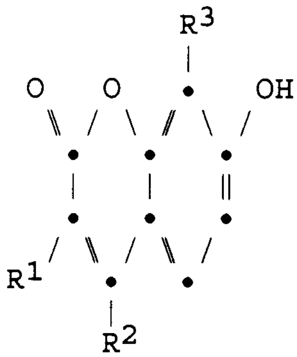

+

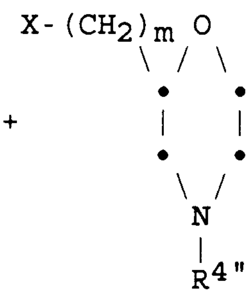

(IV) (VI)

Base

-HX

[Step A1]

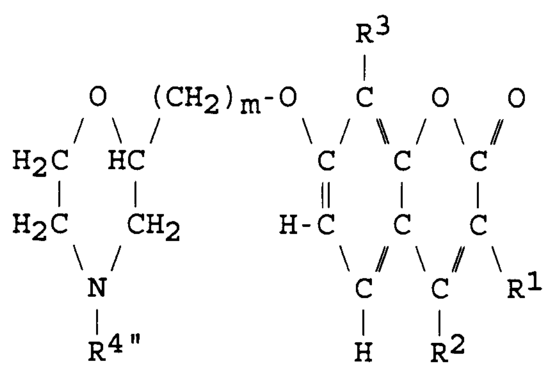

(Ia')

Reaction Scheme B:

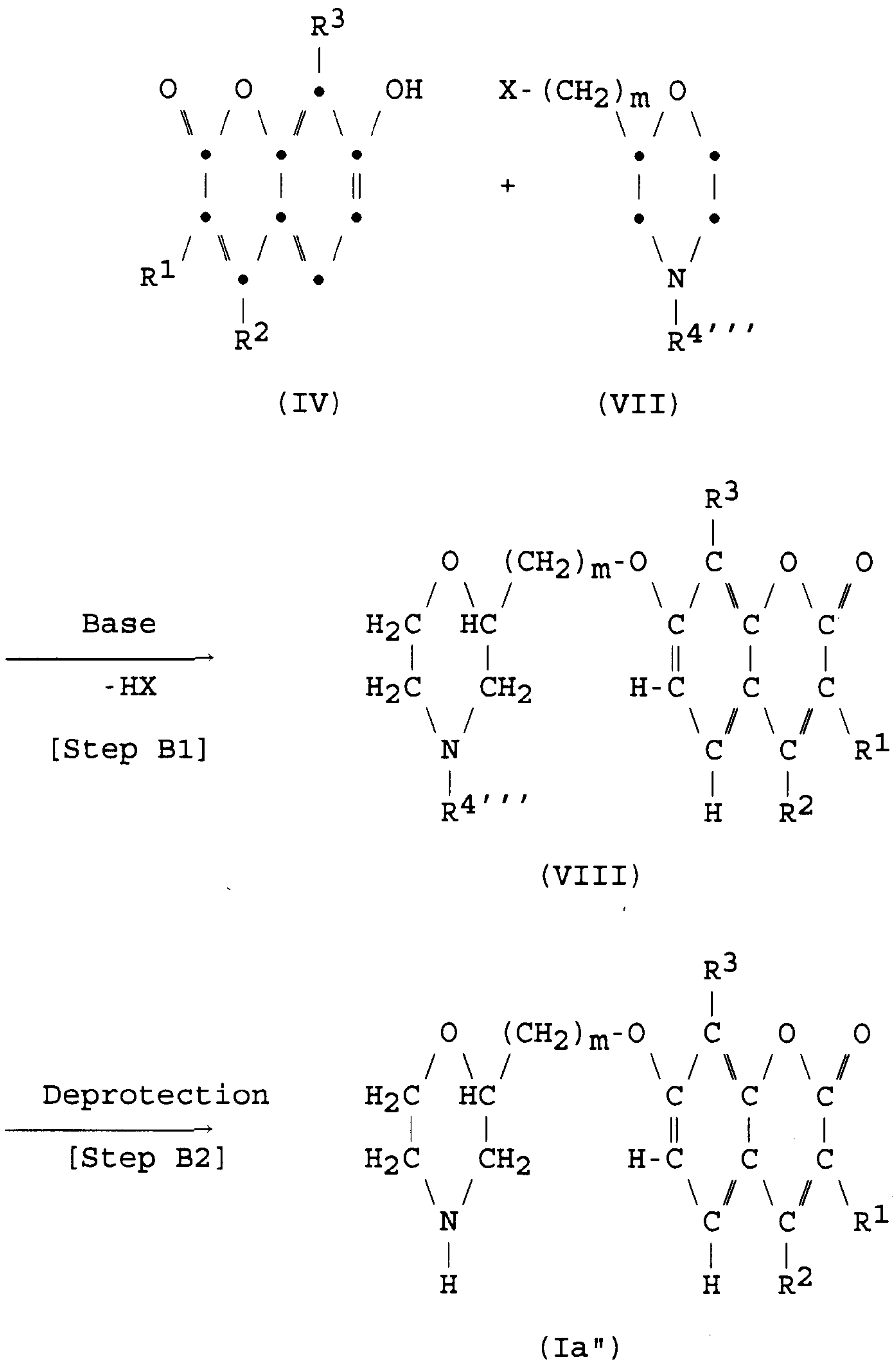


(IV) (VII)

(VIII)

(Ia")

Reaction Scheme C:

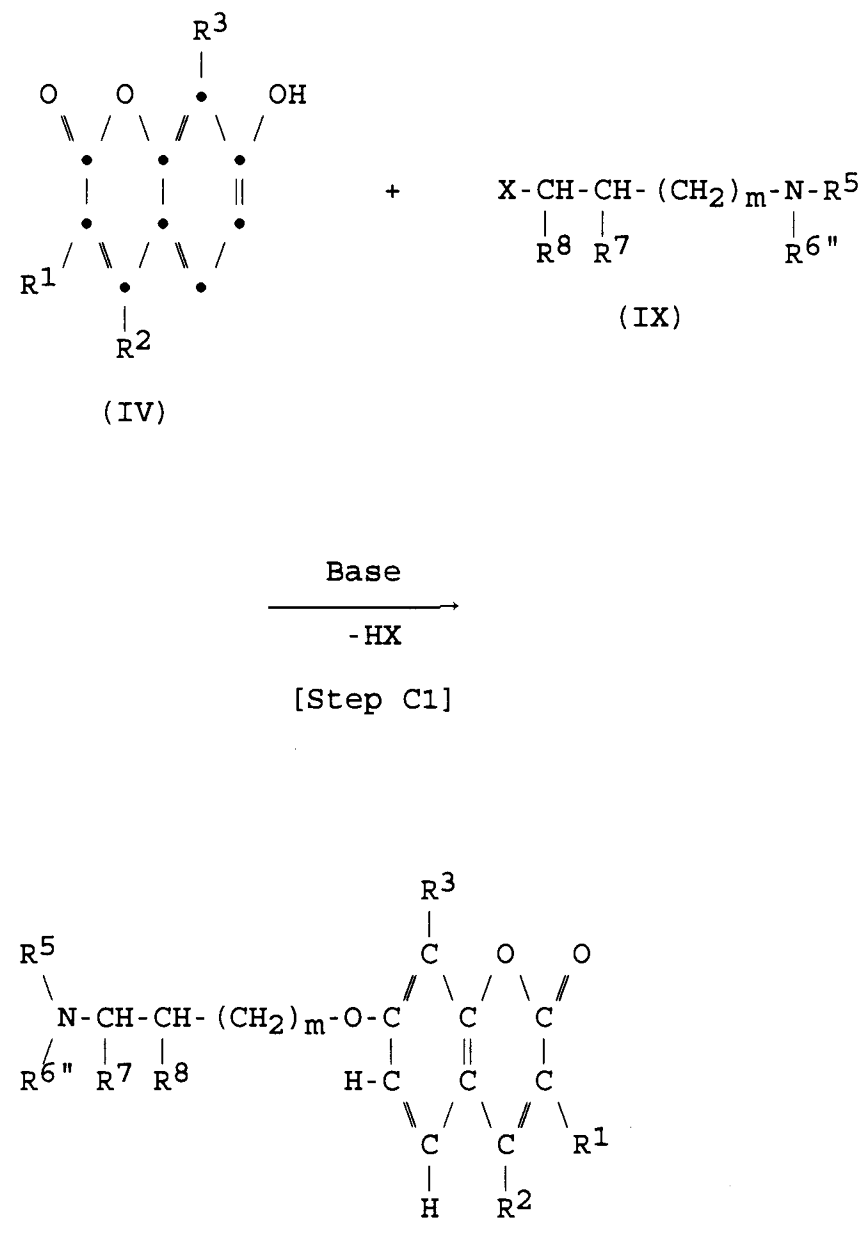


(IV)

(IX)

(Ib')

Reaction Scheme D:

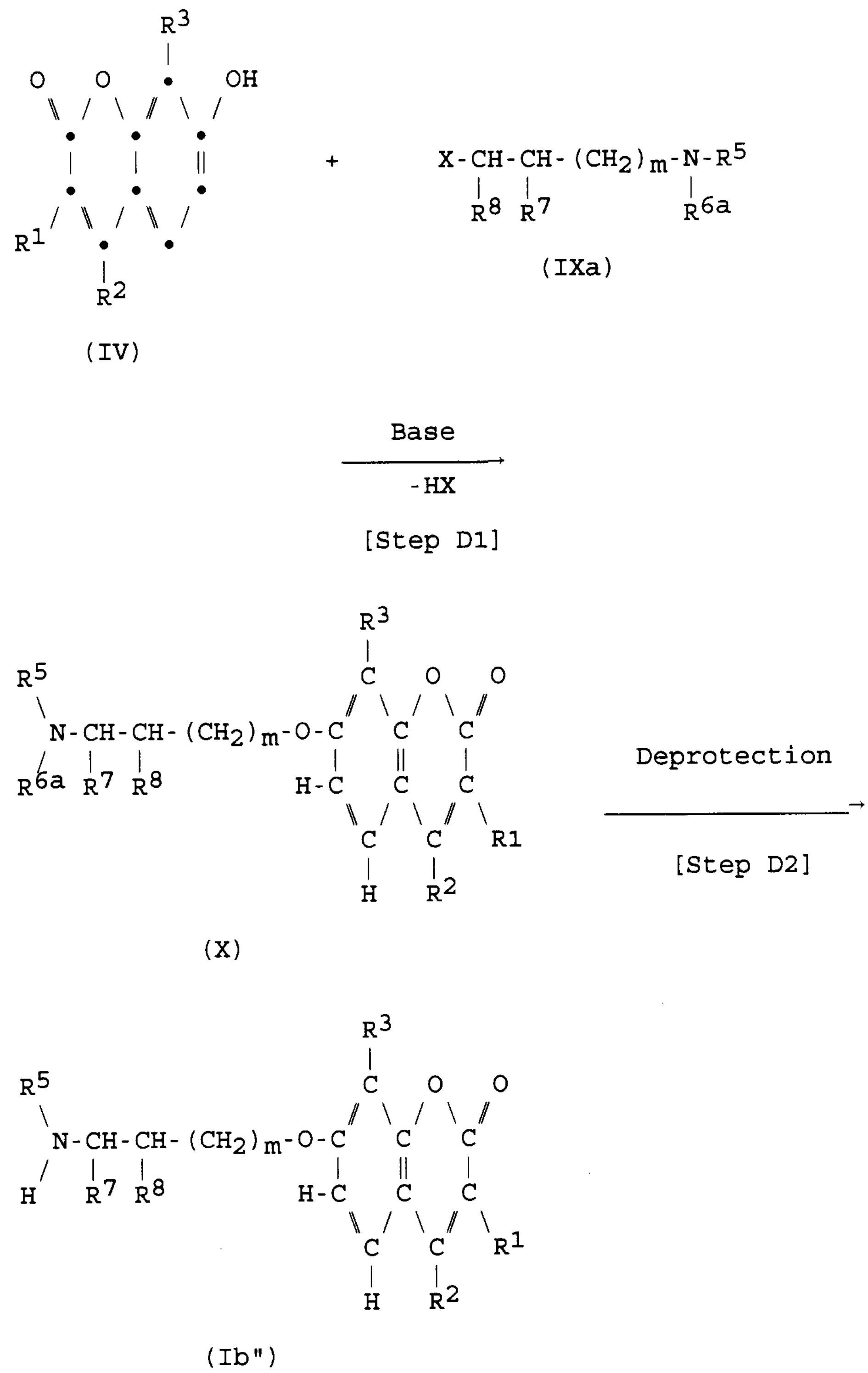


(IV)

(IXa)

(X)

(Ib")

Reaction Scheme E:

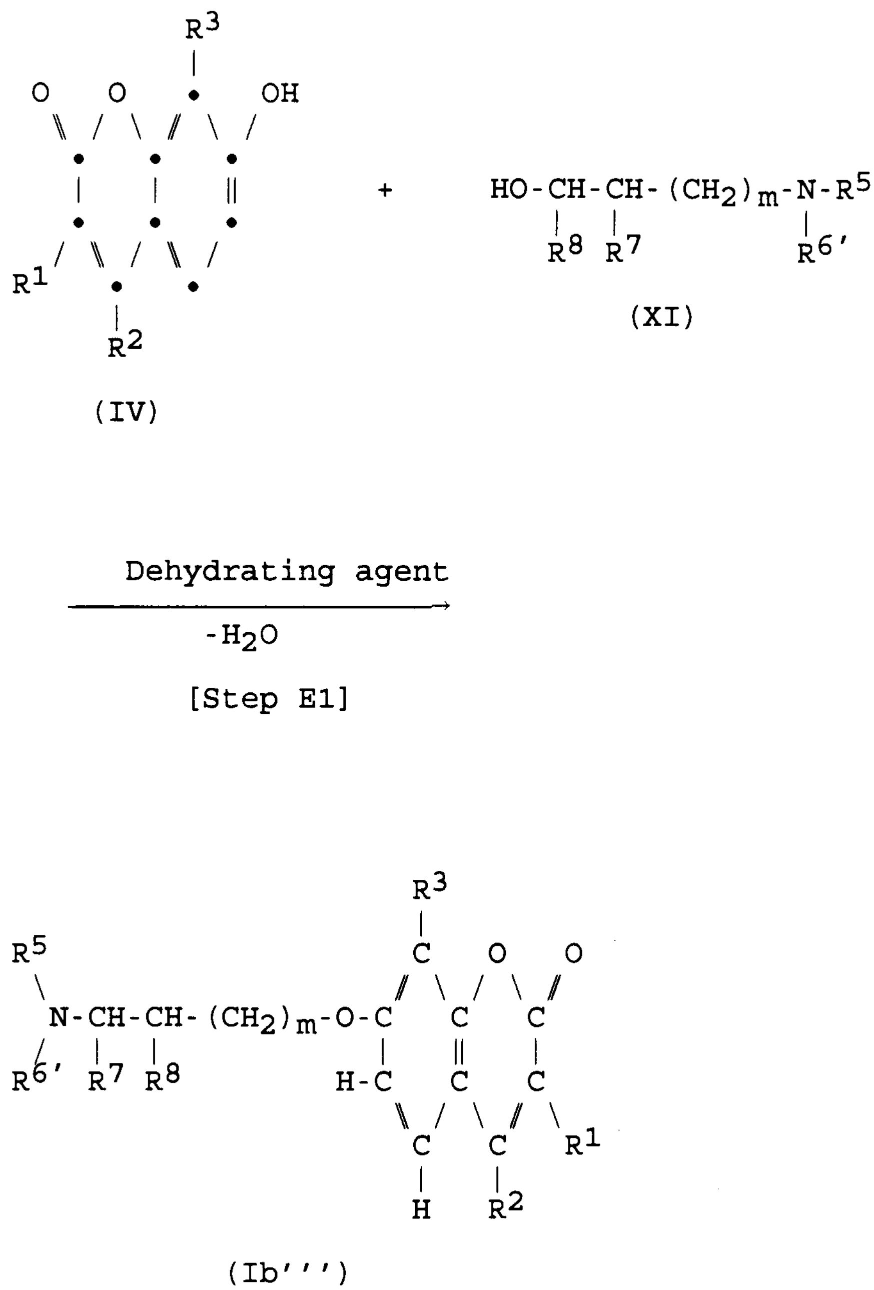


(IV) (XI) (Ib''')

In the above formulae, $R^1$, $R^2$, $R^3$, $R^4$, $R^5$, $R^6$, $R^7$, $R^8$, m and $R^{6'}$ are as defined above; $R^{4''}$ represents an alkyl group having from 1 to 4 carbon atoms, said alkyl group being unsubstituted or having at least one substituent selected from aryl groups having from 6 to 10 carbon atoms, where said aryl group is unsubstituted or is substituted by at least one of substituents (a), defined above; $R^{4'''}$ represents an amino-protecting group; $R^{6''}$ represents an alkyl group; $R^{6a}$ represents an amino-protecting group; and X represents a halogen atom or a sulphonyloxy group.

Examples of halogen atoms whioh may be represented by X or Y include the chlorine, bromine and iodine atoms. Examples of sulphonyloxy groups which may be represented by X or Y include: the alkylsulphonyloxy groups, such as the methylsulphonyloxy and ethylsulphonyloxy groups; and arylsulphonyloxy groups, such as the phenylsulphonyloxy, 4-methylphenylsulphonyloxy and 4-nitrophenylsulphonyloxy groups.

There is no particular restriction on the nature of the amino-protecting groups which may be employed in the above reactions, as the group is eliminated in the course of the reaction and thus has no effect on the final product. Hence any group which can protect the amino group from participating undesirably in the reaction may be employed. Examples of amino-protecting groups which may be employed in the reaction include: aralkyl groups, such as the benzyl, p-methoxybenzyl and triphenylmethyl groups; trialkylsilyl groups, such as the trimethylsilyl and t-butyldimethylsilyl groups; acyl groups, such as the formyl, acetyl and trifluoroacetyl groups; and alkoxycarbonyl and aralkyloxycarbonyl groups, such as the benzyloxycarbonyl, p-nitrobenzyloxycarbonyl, methoxycarbonyl, ethoxycarbonyl and t-butoxycarbonyl groups. of these, we particularly prefer the acyl, alkoxycarbonyl and aralkyloxycarbonyl groups.

Steps A1, B1, C1 and D1 of Reaction Schemes A, B, C and D, respectively, all involve essentially the same reaction, in which a hydroxy compound of formula (IV) is reacted with a halide or sulphonate of formula (VI), (VII), (IX) or (IXa), respectively, in the presence of a base and of an inert solvent.

There is no particular restriction on the nature of the solvent to be employed, provided that it has no adverse effect on the reaction or on the reagents involved. Examples of suitable solvents include: hydrocarbons, especially aromatic hydrocarbons, such as benzene or toluene; ethers, such as tetrahydrofuran and dioxane; ketones, such as acetone and methyl ethyl ketone; alcohols, such as methanol, ethanol and t-butanol; amides, such as N,N-dimethylformamide, N,N-dimethylacetamide and N-methyl-2-pyrrolidone; and sulphoxides, such as dimethyl sulphoxide. Of these, we prefer the ketones and the amides.

There is likewise no particular restriction on the nature of the base to be employed, and any base commonly used in reactions of this type may equally be employed here, provided that it has no adverse effect on any other part of the molecule. Examples of such bases include: alkali metal hydrides, such as lithium hydride and sodium hydride; alkali metal alkoxides, such as sodium methoxide, sodium ethoxide and potassium t-butoxide; alkali metal carbonates, such as sodium carbonate and potassium carbonate; and alkali metal bicarbonates, such as sodium bicarbonate and potassium bicarbonate. Of these, we prefer the alkali metal hydrides and the alkali metal carbonates.

The reaction can take place over a wide range of temperatures, and the precise reaction temperature is not critical to the invention. In general, we find it convenient to carry out the reaction at a temperature from 0° to 120°C (more preferably from 20° to 80°C). The time required for the reaction may also vary widely, depending on many factors, notably the reaction temperature and the nature of the reagents. However, provided that the reaction is effected under the preferred conditions outlined above, a period of from 1 to 24 hours will usually suffice.

The resulting compound of formula (Ia'), (VIII), (Ib') or (X) may, if required, be separated from the reaction mixture by any suitable method, as is well known in the art. For example, it may be separated by the following steps: extraction with an organic solvent, such as ethyl acetate; washing the extract with water; drying it, for example over anhydrous sodium or magnesium sulphate; and finally distilling off the solvent. If desired, the compound may be further purified by various conventional techniques, such as recrystallization or the various chromatography techniques, notably column chromatography or preparative thin layer chromatography. Alternatively, the compound may be used in any subsequent steps without any particular intermediate purification.

The starting compound of formula (IV) employed in this step is either a known compound or may easily be prepared by known processes, for example, by the process described in the Journal of Organic Chemistry 27, 3703 (1962).

In steps B2 and D2, the amino-protecting groups are, if necessary, removed. This removal reaction may be effected by any procedure known in the art, and, as is recognised in the art, the exact procedure used will depend on the nature of the protecting group to be removed. For example: aralkyl and aralkyloxycarbonyl groups, such as the benzyl and benzyloxycarbonyl groups, may be removed by catalytic reduction using a palladium catalyst; triphenylmethyl groups, trialkylsilyl groups (such as the trimethylsilyl) and t-butoxycarbonyl groups may be removed using an acid, such as aqueous acetic acid, hydrogen chloride - dioxane or hydrogen chloride - ethyl acetate; acyl groups (such as the formyl, acetyl and trifluoroacetyl groups) and alkoxycarbonyl and aralkyloxycarbonyl groups (such as the benzyloxycarbonyl group) may be removed by using an alkali, such as sodium methoxide, sodium ethoxide, sodium hydroxide or potassium hydroxide; and trialkylsilyl groups, such as the t-butyldimethylsilyl group, may be removed by using a compound capable of generating fluorine ions.

There is no particular restriction on the nature of the solvent to be employed in these deprotection reactions, provided that it has no adverse effect on the reactions or on the reagents involved. Examples of suitable solvents include: water; alcohols, such as methanol and ethanol; lower fatty acids, such as acetic acid; ethers, such as tetrahydrofuran and dioxane; esters, such as ethyl acetate; and halogenated

hydrocarbons, especially halogenated aliphatic hydrocarbons, such as methylene chloride. The reactions can take place over a wide range of temperatures, and the precise reaction temperature is not critical to the invention. In general, we find it convenient to carry out the reactions at a temperature from 0°C to 100°C (more preferably from room temperature to 80°C). The time required for the reactions may also vary widely, depending on many factors, notably the reaction temperature and the nature of the reagents. However, provided that the reactions are effected under the preferred conditions outlined above, a period of from 30 minutes to 24 hours (more preferably from 1 to 16 hours) will usually suffice.

After completion of the reaction, the reaction product in an organic solvent, such as ethyl acetate or diethyl ether, can be separated in the form of a salt of the compound of formula (I) by the addition of an acid, such as hydrogen chloride. Alternatively, the reaction product can be separated by making the reaction solution alkaline followed by extracting the free compound of formula (I) with an organic solvent, such as ethyl acetate. If desired, the compound may be further purified by various conventional techniques, such as recrystallization or the various chromatography techniques, notably column chromatography or preparative thin layer chromatography.

In Step E1 of Reaction Scheme E, a hydroxy compound of formula (IV) is reacted with an $\alpha$-amino-$\omega$-hydroxy compound of formula (XI) in the presence of a dehydrating agent. The reaction also normally and preferably takes place in the presence of a solvent. There is no particular restriction on the nature of the solvent to be employed, provided that it has no adverse effect on the reaction or on the reagents involved. Examples of suitable solvents include: hydrocarbons, especially aromatic hydrocarbons, such as benzene and toluene; halogenated hydrocarbons, especially halogenated aliphatic hydrocarbons, such as methylene chloride and 1,2-dichloroethane; ketones, such as acetone and methyl ethyl ketone; and amides, especially fatty acid amides, such as N,N-dimethylformamide, N,N-dimethylacetamide and N-methyl-2-pyrrolidone; of these, the halogenated hydrocarbons and amides are especially preferred.

The reaction can take place over a wide range of temperatures, and the precise reaction temperature is not critical to the invention. In general, we find it convenient to carry out the reaction at a temperature from 0° to 60°C, more preferably from 10° to 30°C. The time required for the reaction may also vary widely, depending on many factors, notably the reaction temperature and the nature of the reagents. However, provided that the reaction is effected under the preferred conditions outlined above, a period of from 1 to 24 hours will usually suffice.

Where $R^{6'}$ represents an amino-protecting group, this may be removed by conventional reactions, as outlined above, in relation to Steps B2 and D2.

The desired product may be recovered from the reaction mixture by conventional means, for example simply by concentrating the reaction mixture. The resulting compound may, if desired, be further purified by various conventional techniques, such as recrystallization or the various chromatography techniques, notably column chromatography or preparative thin layer chromatography.

As previously described, the compounds of the present invention may exist in the form of various optical isomers, because of the asymmetric carbon atom contained in the 2-morpholinylmethoxy group of the compound of formula (Ia) or because of the asymmetric carbon atom or atoms when $R^7$ and/or $R^8$ represents an alkyl group in the compounds of formula (Ib). Where the compound of the invention is obtained as a mixture of optical isomers, optical resolution may be effected by reacting an optically active compound of formula (VI), (VII) or (IX) with the compound of formula (IV) in step A1, B1 or C1, or by optical resolution after converting the compound of formula (I) to a salt by reaction with an optically active acid, especially a sulphonic acid or carboxylic acid such as L- or D-camphorsulphonic acid, L- or D-tartaric acid, L- or D-lactic acid or with an acylated L- or D-amino acid.

The compounds of formula (I) can be converted into pharmaceutically acceptable salts thereof by treatment with an acid by conventional means. For example, the compound of formula (I) may be dissolved in an organic solvent, such as ethyl acetate or methylene chloride, and an equimolar amount or an excess of an acid, such as hydrogen chloride - dioxane, may be added. The solvent may then be distilled off, and the resulting compound of formula (I) may be obtained in the form of a salt by crystallization or solidification in an organic solvent such as diethyl ether or diisopropyl ether.

A compound of formula (Ib) in which one of $R^5$ and $R^6$ represents a hydrogen atom and the other represents an alkyl group may be prepared from the corresponding compound in which both $R^5$ and $R^6$ represent hydrogen atoms, that is to say a compound of formula (Ic):

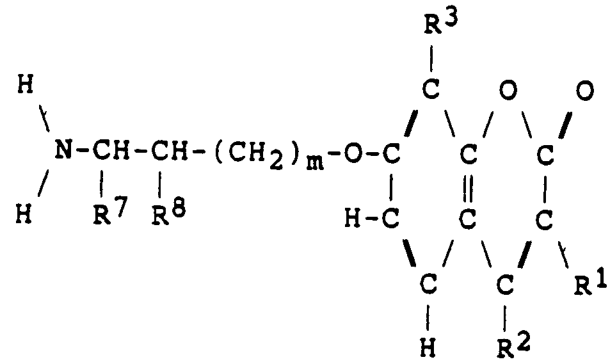

(Ic)

(in which $R^1$, $R^2$, $R^3$, $R^7$, $R^8$ and m are as defined above), by alkylating that compound. Suitable methods of alkylation include a reductive reaction with a carbonyl compound of formula $R^9R^{10}C=O$ (XII), where $R^9$ and $R^{10}$ are the same or different and each represents a hydrogen atom or an alkyl group having from 1 to 3 carbon atoms, and alkylation with a compound of formula $R^{6''}$-X (XIII), where $R^{6''}$ and X are as defined above.

In the reductive alkylation reaction, a Schiff base is prepared by reacting the aminoalkoxycoumarin derivative of formula (Ic) with the carbonyl compound of formula (XII), and this Schiff base is then reduced with a reducing agent (such as sodium cyanoborohydride) or by catalytic reduction in an atmosphere of hydrogen and in the presence of a reduction catalyst, such as Raney nickel or platinum,, preferably all in a single step. Thus, the reaction is preferably carried out in a suitable solvent (e.g. an alcohol, such as methanol or ethanol) and in the presence of hydrogen (which is preferably at atmospheric or superatmospheric pressure, e.g. from 1 to 5 atmospheres pressure) for catalytic reduction or in the presence of a reducing agent. The reaction will take place over a wide range of temperatures, and the precise reaction temperature chosen is not critical to the invention. In general, we find it convenient to carry out the reaction at a temperature in the range of from 0°C to 50°C (preferably from 0°C to room temperature). The time required for the reaction may likewise vary widely, depending on many factors, notably the reaction temperature and the nature of the reagents. However, in most cases, a period of from 30 minutes to 24 hours will normally suffice.

After completion of the reaction, the desired compound of formula (Ib' ) wherein $R^5$ represents a hydrogen atom can be separated from the reaction mixture by any of a number of conventional techniques well known in the art. For example, when the reaction is conducted by catalytic reduction, this may be achieved by filtering the catalyst off, followed by condensing the filtrate; alternatively, when a reducing agent is used, a suitable separation technique comprises condensing the reaction solution under reduced pressure and then extracting the residue with an organic solvent, such as methylene chloride or ethyl acetate.

Alternatively, the alkylation reaction comprises reacting the compound of formula (Ic) with a compound of formula $R^{6''}$-X (XIII), where $R^{6''}$ and X are as defined above, i.e. an alkyl halide or alkyl sulphonate. This reaction preferably takes place in the presence of an inert solvent and of a base; reaction conditions are similar to those employed when reacting the compound of formula (IV) with a compound of formula (XI) or (XIa). In order to produce a compound of formula (Ib) in which only one of $R^5$ and $R^6$ represents an alkyl group and the other represents a hydrogen atom, it is desirable to employ about 1 equivalent of the compound of formula (XIII) per equivalent of the compound of formula (Ic), or, in any event, less than 2 equivalents; if 2 or more equivalents of the compound of formula (XIII) are employed per equivalent of the compound of formula (Ic), the product will be predominantly a compound of formula (Ib) in which both $R^5$ and $R^6$ represent alkyl groups.

BIOLOGICAL ACTIVITY

The compounds of the present invention have the ability to suppress the increase in blood viscosity which otherwise occurs as a result of cerebral ischaemia; they also exhibit a potent antireserpine activity. Therefore, the compounds of the present invention are expected to improve cerebral metabolism and circulation as a result of their monoamine activating effect and microcirculation improving effect.

In the following experiments, the compounds of the present invention are identified by the number of the one of the subsequent Examples in which that compound was prepared.

(1) Antireserpine effect

The test animals employed were male ddY mice, 4 weeks old and each weighing 22 to 27 g. The animals were divided into 2 groups, each including 3 mice. The compound under test was dissolved or suspended in a physiological saline solution, in a 0.5% CMC (carboxymethyl cellulose) solution or in a 1% dimethyl sulphoxide solution, and 100 mg/kg of the sample solution were orally administered to each of the mice in one group (treated group). The mice in the other group were given only the solvent without any active compound (control group). Immediately after administration, 2 mg/kg of reserpine were administered subcutaneously to every mouse. After 90 minutes, it was determined how much the eyelids were closed (degree of ptosis). For assessment of the results, normal mice without ptosis were scored as 0, while a mouse which exhibited ptosis by 1/3 to 1/2 was scored as 1, a mouse which exhibited ptosis by 2/3 to slightly opened eyes was scored as 2, and a mouse with completely closed eyelids was scored as 3. The reserpine-inhibitory rate of the test sample was calculated from the following equation.

$$\text{Reserpine-inhibitory rate (\%)} = \frac{[\text{Score of control group}]-[\text{Score of treated group}]}{[\text{Score of control group}]} \times 100$$

The results are shown in Table 4.

Table 4

| Test compound | Reserpine-inhibitory rate (%) |
|---|---|
| Compound of Example 1 | 86 |
| Compound of Example 2 | 100 |
| Compound of Example 3 | 100 |
| Compound of Example 4 | 100 |
| Compound of Example 8 | 100 |
| Compound of Example 9 | 71 |
| Compound of Example 10 | 77 |
| Compound of Example 11 | 100 |
| Compound of Example 15 | 100 |
| Compound of Example 16 | 100 |
| Compound of Example 17 | 100 |
| Compound of Example 18 | 100 |
| Compound of Example 19 | 100 |
| Compound of Example 22 | 100 |
| Compound of Example 23 | 100 |
| Compound of Example 25 | 100 |
| Compound of Example 26 | 100 |
| Compound of Example 27 | 100 |
| Compound of Example 28 | 100 |
| Compound of Example 29 | 100 |
| Compound of Example 30 | 100 |
| Compound of Example 31 | 100 |
| Compound of Example 32 | 100 |

Table 4 (cont)

| Test compound | Reserpine-inhibitory rate (%) |
|---|---|
| Compound of Example 33 | 100 |
| Compound of Example 34 | 100 |
| Compound of Example 35 | 100 |
| Compound of Example 36 | 100 |
| Compound of Example 37 | 100 |
| Compound of Example 40 | 100 |
| Compound of Example 41 | 100 |
| Compound of Example 42 | 100 |
| Compound of Example 43 | 100 |

(2) Inhibitory effect on increase of blood viscosity following cerebral ischaemia

Adult male rats of the Wistar strain were divided into 2 groups, each including 6 rats. The test sample was dissolved or suspended in a physiological saline solution or in a 0.5% CMC solution, and 100 mg/kg of the sample were orally administered to each of the rats in one group (treated group). The rats in the other group were given only the solvent without any active compound (control group). Immediately after administration, 40 mg/kg of pentobarbital was intraperitoneally administered to each of the rats to anesthetize it. The rat was held in a supine position. 0.6 ml of a blood sample was collected from the jugular vein on one side. By using a blood viscosimeter (Biorheolyzer, Tokyo Keiki), the blood viscosities were measured at shear rates of 37.5/sec, 75/sec, 150/sec and 375/sec. The common carotid arteries of both sides were ligated for one hour to induce incomplete cerebral ischaemia, and 0.5 ml of blood was collected from the jugular vein on the other side to measure its viscoity in the same way as mentioned above. The inhibition of blood viscosity increase by the test drug was calculated according to the following equation at each shear rate.

The experiments were carried out using several of the compounds of the present invention as well as the known compound, Indeloxazine hydrochloride.

$$\text{Inhibition of blood viscosity increase (\%)} = \frac{\begin{bmatrix}\text{Increase in blood viscosity} \\ \text{induced by ligation in} \\ \text{the control group (\%)}\end{bmatrix} - \begin{bmatrix}\text{Increase in blood viscosity} \\ \text{induced by ligation in} \\ \text{the treated group (\%)}\end{bmatrix}}{\begin{bmatrix}\text{Increase in blood viscosity induced} \\ \text{by ligation in the control group (\%)}\end{bmatrix}} \times 10$$

The results are shown in Table 5.

Table 5

| Test compound | Blood viscosity increase inhibiting rate (%) shear rate/sec | | | |
|---|---|---|---|---|
| | 37.5 | 75 | 150 | 375 |
| Compound of Example 1 | 57 | 47 | 59 | 79 |
| Compound of Example 2 | 57 | 42 | 35 | 19 |
| Compound of Example 3 | 65 | 63 | 76 | 71 |
| Compound of Example 4 | 80 | 83 | 100 | 100 |
| Compound of Example 8 | 72 | 70 | 81 | 100 |
| Compound of Example 15 | 100 | 79 | 97 | 100 |
| Compound of Example 16 | 97 | 75 | 79 | 95 |
| Compound of Example 17 | 78 | 67 | 74 | 80 |
| Compound of Example 18 | 69 | 55 | 71 | 91 |
| Compound of Example 22 | 66 | 61 | 55 | 75 |
| Compound of Example 29 | 96 | 88 | 90 | 98 |
| Compound of Example 31 | 67 | 60 | 82 | 84 |
| Compound of Example 34 | 76 | 79 | 86 | 100 |
| Compound of Example 36 | 62 | 58 | 53 | 97 |
| Compound of Example 37 | 77 | 85 | 77 | 100 |
| Compound of Example 41 | 68 | 57 | 47 | 71 |
| Compound of Example 43 | 72 | 53 | 64 | 83 |
| Indeloxazine hydrochloride (control drug) | 55 | 47 | 52 | 79 |

The results given above demonstrate that the compounds of formula (I) and pharmaceutically acceptable salts thereof may be employed to improve cerebral circulation and metabolism. For this purpose, they can be administered orally or parenterally in forms suitable for their intended route of administration. For example, for oral administration, it is possible to employ such preparations as powders, granules, tablets or capsules, whilst for parenteral administration, such forms as injections, suppositories and plasters may be appropriate. The compound itself may be employed alone or in admixture with any other suitable pharmaceutically acceptable carriers, vehicles or diluents. The preferred dosage may vary, depending on the nature of the disorder to be treated, the age, condition and body weight of the patient and on the mode of administration; however, a daily dose of from 1 to 1,000 mg, more preferably from 1 to 100 mg is preferred, if the compound is administered orally; and a dose of from 0.1 to 100 mg, more preferably from 0.5 to 30 mg, is preferred, if the compound is administered intravenously. The compounds may be administered in single or divided doses one to 3 times a day, depending on the symptoms.

The invention is further illustrated by the following Examples which demonstrate the preparation of certain of the compounds of the present invention. The subsequent Preparations illustrate the preparation of certin of the starting materials used in these Examples, whilst the subsequent Formulations illustrate compositions of the invention.

EXAMPLE 1

7-(2-Morpholinyl)methoxycoumarin

1(a) 7-(4-t-Butoxycarbonyl-2-morpholinyl)methoxycoumarin

1.6 g of potassium carbonate was added to 25 ml of an acetone solution containing 0.32 g of 7-hydroxycoumarin and 0.80 g of 4-t-butoxycarbonyl-2-[(4-nitrophenylsulphonyloxy)methyl]morpholine (prepared as described in Preparation 2). The reaction mixture was then stirred for 16 hours, whilst heating it under reflux. At the end of this time, the acetone was removed by evaporation under reduced pressure. The residue was then extracted with ethyl acetate, and the extract was washed with water. The extract was then dried over anhydrous magnesium sulphate, after which the solvent was removed, to afford the title compound as crystals. These crystals were purified by silica gel column chromatography using a 1 : 4 by volume mixture of ethyl acetate and methylene chloride as the eluent, to give 0.62 g of the title compound as crystals, melting at 146°C.
Nuclear Magnetic Resonance Spectrum ($CDC\ell_3$), δ ppm:

1.46 (9H, singlet);
2.5 - 4.3 (9H, multiplet);
6.25 (1H, doublet, J = 9 Hz);
6.8 - 7.0 (2H, multiplet);
7.42 (1H, doublet, J = 9 Hz);
7.68 (1H, doublet, J = 9 Hz).

1(b) 7-(2-Morpholinyl)methoxycoumarin hydrochloride

A mixture of 0.62 g of 7-(4-t-butoxycarbonyl-2-morpholinyl)methoxycoumarin [prepared as described in step (a) above] and 1.5 ml of a 4N solution of hydrogen chloride in dioxane was stirred at room temperature for 2 hours. At the end of this time, the reaction solution was condensed by evaporation under reduced pressure, and ethyl acetate was added. The hydrochloride of the title compound separated as crystals, melting at 185 - 187 °C; these were collected by filtration, to give 0.53 g of the title compound.
Nuclear Magnetic Resonance Spectrum (hexadeuterated dimethyl sulphoxide), δ ppm:
2.6 - 4.4 (9H, multiplet);
6.27 (1H, doublet, J = 9 Hz);
6.88 - 7.02 (2H, multiplet);
7.66 (1H, doublet, J = 9 Hz);
8.02 (1H, doublet, J = 9 Hz);
9.95 (2H, broad singlet).

EXAMPLE 2

4-Methyl-7-(2-morpholinyl)methoxycoumarin

2(a) 7-(4-t-Butoxycarbonyl-2-morpholinyl)methoxy-4-methylcoumarin

0.35 g of 7-hydroxy-4-methylcoumarin, 0.80 g of 4-t-butoxycarbonyl-2-[(4-nitrophenylsulphonyloxy)-methyl]morpholine (prepared as described in Preparation 2) and 1.6 g of potassium carbonate were added to 19 ml of methyl ethyl ketone, and the reaction mixture was stirred for 23 hours, whilst being heated under reflux. It was then condensed by evaporation under reduced pressure. Ethyl acetate and water were added to the resulting residue. The ethyl acetate layer was separated, washed with water and dried over anhydrous magnesium sulphate, after which the solvent was removed by evaporation under reduced pressure. The residue was then purified by silica gel column chromatography using a 1 : 5 by volume mixture of ethyl acetate and methylene chloride as the eluent, to obtain 0.75 g of the title compound as crystals, melting at 124 - 126 °C.
Nuclear Magnetic Resonance Spectrum ($CDCl_3$), δ ppm:
1.48 (9H, singlet);
2.37 (3H, singlet);
2.5 - 7.3 (9H, multiplet);
6.12 (1H, broad singlet);
6.8 - 7.0 (2H, multiplet);
7.52 (1H, broad doublet, J = 9 Hz).

2(b) 4-Methyl-7-(2-morpholinyl)methoxycoumarin hydrochloride

0.70 g of 7-(4-t-butoxycarbonyl-2-morpholinyl)methoxy-4-methylcoumarin [prepared as described in step (a) above] was treated by a procedure similar to that described in Example 1(b), to give 0.61 g of the crystalline hydrochloride of the title compound in the form of hygroscopic crystals, melting at 169 - 171 °C.
Nuclear Magnetic Resonance Spectrum (hexadeuterated dimethyl sulphoxide), δ ppm:
2.39 (3H, singlet);
2.7 - 4.3 (9H, multiplet);
6.21 (1H, broad singlet);
6.9 - 7.1 (2H, multiplet);
7.72 (1H, doublet, J = 10 Hz);
9.92 (2H, broad singlet).

EXAMPLE 3

4,8-Dimethyl-7-(2-morpholinyl)methoxycoumarin

3(a) 7-(4-t-Butoxyoarbonyl-2-morpholinyl)methoxy-4,8-dimethylcoumarin

A procedure similar to that described in Example 2(a) was repeated, except that 0.57 g of 7-hydroxy-4,8-dimethylcoumarin and 0.74 g of 4-t-butoxycarbonyl-2-[(4-nitrophenylsulphonyloxy)methyl]morpholine (prepared as described in Preparation 2) were used, to give 0.84 g of the title compound as crystals, melting at 145 - 147°C.

Nuclear Magnetic Resonance Spectrum ($CDCl_3$), δ ppm:

1.46 (9H, singlet);
2.26 (3H, singlet);
2.34 (3H, broad singlet);
2.5 - 4.3 (9H, multiplet);
6.08 (1H, broad singlet);
6.81 (1H, doublet, J = 9 Hz);
7.40 (1H, doublet, J = 9 Hz).

3(b) 4,8-Dimethyl-7-(2-morpholinyl)methoxycoumarin hydrochloride

A procedure similar to that desoribed in Example 1(b) was repeated, except that 0.84 g of 7-(4-t-butoxycarbonyl-2-morpholinyl)methoxy-4,8-dimethylcoumarin were employed, to give 0.75 g of the title compound in the form of its crystalline hydrochloride, melting at 256 - 259°C.

Nuclear Magnetic Resonance Spectrum (hexadeuterated dimethyl sulphoxide), δ ppm:

2.17 (3H, singlet);
2.36 (3H, broad singlet);
2.7 - 4.3 (9H, multiplet);
6.17 (1H, broad singlet);
7.00 (1H, doublet, J = 9 Hz);
7.56 (1H, doublet, J = 9 Hz);
9.80 (2H, broad singlet).

EXAMPLE 4

3,4,8-Trimethyl-7-(2-morpholinyl)methoxycoumarin

4(a) 7-(4-t-Butoxycarbonyl-2-morpholinyl)methoxy-3,4,8-trimethylcoumarin

4.84 g of 7-hydroxy-3,4,8-trimethylcoumarin, 9.6 g of 4-t-butoxycarbonyl-2-[(4-nitrophenylsulphonyloxy)-methyl]morpholine(prepared as described in Preparation 2) and 12 g of potassium carbonate were mixed in 200 ml of dimethylformamide, and the mixture was stirred for 8 hours at 60°C. At the end of this time, ethyl acetate and water were added to the mixture. The ethyl acetate layer was then separated, washed with water and dried over anhydrous magnesium sulphate and then the solvent was removed by distillation under reduced pressure, to give the title compound as crystals. These crystals were washed with small amounts each of ethyl acetate and diethyl ether, and then collected by filtration, to give 7.35 g of the title compound as crystals, melting at 124 - 126°C.

Nuclear Magnetic Resonance Spectrum ($CDCl_3$), δ ppm:

1.51 (9H, singlet);
2.10 (3H, singlet);
2.25 (6H, singlet);
2.5 - 4.3 (9H, multiplet);
6.71 (1H, doublet, J = 9 Hz);
7.27 (1H, doublet, J = 9 Hz).

4(b) 3,4,8-Trimethyl-7-(2-morpholinyl)methoxycoumarin hydrochloride

40 ml of a 4N solution of hydrogen chloride in ethyl acetate were added to 6.87 g of 7-(4-t-butoxycarbonyl-2-morpholinyl)methoxy-3,4,8-trimethylcoumarin [prepared as described in step (a) above]. The reaction mixture was then stirred for 1 hour at room temperature, after which the ethyl acetate was removed by distillation under reduced pressure. Diethyl ether was added to the residue, and the title compound was obtained by filtration in the form of its crystalline hydrochloride, melting at 247 - 250 °C. The yield was 5.53 g, after recrystallization from ethanol.

Nuclear Magnetic Resonance Spectrum (hexadeuterated dimethyl sulphoxide), δ ppm:

2.05 (3H, singlet);
2.18 (3H, singlet);
2.32 (3H, singlet);
2.7 - 4.3 (9H, multiplet);
7.03 (1H, doublet, J = 9 Hz);
7.61 (1H, doublet, J = 9 Hz);
9.8 (2H, broad singlet).

EXAMPLE 5

3-Isopropyl-4,8-dimethyl-7-(2-morpholinyl)methoxycoumarin

5(a) 7-(4-t-Butoxycarbonyl-2-morpholinyl)methoxy-3-isopropyl-4,8-dimethylcoumarin

A procedure similar to that described in Example 4(a) was repeated, except that 0.93 g of 3-isopropyl-7-hydroxy-3,8-dimethylcoumarin and 1.60 g of 4-t-butoxycarbonyl-2-[(4-nitrophenylsulphonyloxy)methyl]-morpholine (prepared as described in Preparation 2) were used, to give 1.20 g of the title compound as crystals, melting at 106 - 108 °C.

Nuclear Magnetic Resonance Spectrum ($CDCl_3$), δ ppm:

1.33 (6H, doublet, J = 8 Hz);
1.46 (9H, singlet);
2.27 (3H, singlet);
2.36 (3H, singlet);
2.5 - 4.3 (10H, multiplet);
6.78 (1H, doublet, J = 9.5 Hz);
7.41 (1H, doublet, J = 9.5Hz).

5(b) 3-Isopropyl-4,8-dimethyl-7-(2-morpholinyl)methoxycoumarin fumarate

A procedure similar to that desoribed in Example 4(b) was repeated, except that 1.1 g of 7-(4-butoxycarbonyl-2-morpholinyl)methoxy-3-isopropyl-4,8-dimethylcoumarin [prepared as described in step (a) above] was employed and that, after the reaction, the reaction solution was condensed by evaporation under reduced pressure. The residue was dissolved in water and the resulting aqueous solution was washed with diethyl ether; sufficient potassium carbonate was then added to make the solution alkaline. The title compound separated and was extracted with ethyl acetate. Sodium chloride was then added to the residual aqueous layer for salting-out, and then the title compound remaining in the aqueous layer was extracted with ethyl acetate. The two ethyl acetate extracts were combined and dried over anhydrous magnesium sulphate. The solvent was then distilled off, giving 0.86 g of the title compound as a gum. This gum was dissolved in about 3 ml of ethyl acetate, and 5 ml of methanol containing 280 mg of fumaric acid were added to the resulting solution. The title compound separated in the form of its fumarate as crystals. After addition of diethyl ether, the separated compound was collected by filtration to afford 0.98 g of the title compound as crystals, melting at 188 °C.

Nuclear Magnetic Resonance Spectrum (hexadeuterated dimethyl sulphoxide), δ ppm:

1.25 (6H, doublet, J = 6.5 Hz);
2.17 (3H, singlet);
2.37 (3H, singlet);
2.7 - 4.2 (10H, multiplet);
5.40 (3H, broad singlet);
6.52 (2H, singlet);

7.00 (1H, doublet, J = 9.5 Hz);
7.62 (1H, doublet, J = 9.5 Hz).

EXAMPLE 6

6-Chloro-3,4-dimethyl-7-(2-morpholinyl)methoxycoumarin

6(a) 7-(4-t-Butoxycarbonyl-2-morpholinyl)methoxy-6-chloro-3,4-dimethylcoumarin

A procedure similar to that described in Example 4(a) was repeated, except that 0.85 g of 6-chloro-7-hydroxy-3,4-dimethylcoumarin and 1.60 g of 4-t-butoxycarbonyl-2-[(4-nitrophenylsulphonyloxy)methyl]-morpholine (prepared as described in Preparation 2) were employed, to give 1.16 g of the title compound as crystals, melting at 164°C.
Nuclear Magnetic Resonance Spectrum ($CDCℓ_3$), δ ppm:
1.45 (9H, singlet);
2.15 (3H, singlet);
2.30 (3H, singlet);
2.7 - 4.3 (9H, multiplet);
6.82 (1H, singlet);
7.54 (1H, singlet).

6(b) 6-Chloro-3,4-dimethyl-7-(2-morpholinyl)methoxycoumarin hydrochloride

A procedure similar to that described in Example 4(b) was repeated, except that 1.00 g of 7-(4-t-butoxycarbonyl-2-morpholinyl)methoxy-6-chloro-3,4-dimethylcoumarin [prepared as desoribed in step (a) above] was employed, to give 0.88 g of the title compound in the form of its hydrochloride as crystals. The compound gradually decomposes from 250°C, and melts at 278°C (with decomposition).
Nuclear Magnetic Resonance Spectrum (hexadeuterated dimethyl sulphoxide), δ ppm:
2.06 (3H, singlet);
2.32 (3H, singlet);
2.7 - 4.3 (9H, multiplet);
7.15 (1H, singlet);
7.76 (1H, singlet);
9.80 (2H, broad singlet).

EXAMPLE 7

7-(2-Morpholinyl)methoxy-4-propylcoumarin

7(a) 7-(4-t-Butoxycarbonyl-2-morpholinyl)methoxy-4-propylcoumarin

A procedure similar to that described in Example 4(a) was repeated, except that 0.50 g of 7-hydroxy-4-propylcoumarin and 0.91 g of 4-t-butoxycarbonyl-2-[(4-methylphenylsulphonyloxy)methyl]morpholine (prepared as described in Preparation 1) were employed, to give 0.89 g of the title compound as a gum.

7(b) 7-(2-Morpholinyl)methoxy-4-propylcoumarin maleate

A procedure similar to that described in Example 5(b) was repeated, except that 0.89 g of 7-(4-t-butoxycarbonyl-2-morpholinyl)methoxy-4-propylcoumarin [prepared as described in step (a) above] was employed, to give 0.64 g of the title compound in the form of its free amine as a gum. This free amine was converted into its crystalline maleate by reacting it with 0.24 g of maleic acid, to yield 0.86 g of the maleate, melting at 170 - 171°C.
Nuclear Magnetic Resonance Spectrum (hexadeuterated dimethyl sulphoxide), δ ppm:
1.00 (3H, triplet, J = 6.5 Hz);
1.66 (2H, multiplet);
2.7 - 4.3 (11H, multiplet);
6.10 (2H, singlet);
6.12 (1H, broad singlet);

6.9 - 7.1 (2H, multiplet);
7.74 (1H, doublet, J = 9 Hz).

EXAMPLE 8

7-(3-N-Methylaminopropoxy)-3,4,8-trimethylcoumarin hydrochloride

8(a) 7-[3-(N-t-Butoxycarbonyl-N-methylamino)propoxy]-3,4,8-trimethylcoumarin

9 g of potassium carbonate were added to 50 ml of a dimethylformamide solution containing 3.0 g of 7-hydroxy-3,4,8-trimethylcoumarin and 6.73 g of 3-(N-t-butoxycarbonyl-N-methylamino)propyl p-toluenesulphonate. The reaction mixture was then stirred for 5 hours at 65 ° C, after which ethyl acetate and water were added. The ethyl acetate layer was separated and dried over anhydrous magnesium sulphate, and then the solvent was removed by distillation under reduced pressure. The resulting crystalline residue was purified by silica gel column chromatography using a 1 : 9 by volume mixture of ethyl acetate and methylene chloride as the eluent, to afford 4.04 g of the title compound as crystals, melting at 110 ° C.
Nuclear Magnetic Resonance Spectrum ($CDCl_3$), δ ppm:
1.46 (9H, singlet);
1.85 - 2.25 (2H, multiplet);
2.17 (3H, singlet);
2.30 (3H, singlet);
2.34 (3H, singlet);
2.90 (3H, singlet);
3.45 (2H, triplet, J = 7 Hz);
4.06 (2H, triplet, J = 7 Hz);
6.77 (1H, doublet, J = 8.5 Hz);
7.37 (1H, doublet, J = 8.5 Hz).

8(b) 7-(3-N-Methylaminopropoxy)-3,4,8-trimethylcoumarin hydrochloride

30 ml of a 4N solution of hydrogen chloride in ethyl acetate were added to 3.9 g of 7-[3-(N-t-butoxycarbonyl-N-methylamino)propoxy]-3,4,8-trimethylcoumarin [prepared as described in step (a) above], and the mixture was stirred for 4 hours at room temperature. At the end of this time, the gelatinous reaction mixture was condensed by evaporation under reduced pressure. About 20 ml of ethanol were added to the residue, and the resulting mixture was heated under reflux and then cooled. 40 ml of ethyl acetate were then added, and the resulting precipitate was collected by filtration, to afford 2.99 g of the title compound, which softened above 215 ° C and melted at 253 ° C.
Nuclear Magnetic Resonance Spectrum (hexadeuterated dimethyl sulphoxide), δ ppm:
2.08 (3H, singlet);
2.21 (3H, singlet);
2.05 - 2.25 (2H, multiplet);
2.36 (3H, singlet);
2.59 (3H, singlet);
3.08 (2H, triplet, J = 7.5 Hz);
4.21 (2H, triplet, J = 7.5 Hz);
7.04 (1H, doublet, J = 8.5 Hz);
7.63 (1H, doublet, J = 8.5 Hz).

EXAMPLE 9

7-(4-N-Methylaminobutoxy)-3,4,8-trimethylcoumarin hydrochloride

9(a) 7-[4-(N-t-Butoxycarbonyl-N-methylamino)butoxy]-3,4,8-trimethylcoumarin

A procedure similar to that described in Example 8(a) was repeated, except that 0.96 g of 7-hydroxy-3,4,8-trimethylcoumarin, 2.19 g of 4-(N-t-butoxycarbonyl-N-methylamino)butyl p-nitrobenzenesulphonate and 2.6 g of potassium carbonate were employed, and then the product was purified by silica gel column chromatography using a 1 : 4 by volume mixture of ethyl acetate and hexane as the eluent, to afford 1.80 g

of the title compound as crystals, melting at 86 °C.
Nuclear Magnetic Resonance Spectrum ($CDCl_3$), δ ppm:

1.48 (9H, singlet);
1.65 - 1.95 (4H, multiplet);
2.16 (3H, singlet);
2.29 (3H, singlet);
2.33 (3H, singlet);
2.86 (3H, singlet);
3.15 - 3.45 (2H, multiplet);
3.95 - 4.15 (2H, multiplet);
6.76 (1H, doublet, J = 8.5 Hz);
7.37 (1H, doublet, J = 8.5 Hz).

9(b) 7-(4-N-Methylaminobutoxy)-3,4,8-trimethylcoumarin hydrochloride

A procedure similar to that described in Example 8(b) was repeated, except that 1.70 g of 7-[4-(N-t-butoxycarbonyl-N-methylamino)butoxy]-3,4,8-trimethylcoumarin [prepared as desoribed in step (a) above] was employed, to give 1.30 g of the title compound as crystals, melting at 197 °C.
Nuclear Magnetic Resonance Spectrum (hexadeuterated dimethyl sulphoxide), δ ppm:

1.75 - 1.95 (4H, multiplet);
2.09 (3H, singlet);
2.22 (3H, singlet);
2.36 (3H, singlet);
2.55 (3H, singlet);
2.85 - 3.10 (2H, multiplet);
4.00 - 4.20 (2H, multiplet);
7.03 (1H, doublet, J = 8.5 Hz);
7.59 (1H, doublet, J = 8.5 Hz).

EXAMPLE 10

3-Chloro-4,8-dimethyl-7-(3-N-methylaminopropoxy)-coumarin hydrochloride

10(a) 7-[3-(N-t-Butoxycarbonyl-N-methylamino)propoxy]-3-chloro-4,8-dimethylcoumarin

A procedure similar to that described in Example 8(a) was repeated, except that 0.65 g of 3-chloro-7-hydroxy-4,8-dimethylcoumarin, 1.44 g of 3-(N-t-butoxycarbonyl-N-methylamino)propyl p-toluenesulphonate and 2.0 g of potassium carbonate were employed, to give, after purification by silica gel column chromatography using a 1 : 9 by volume mixture of ethyl acetate and methylene chloride as the eluent, 0.93 g of the title compound as crystals, melting at 161 °C.
Nuclear Magnetic Resonance Spectrum ($CDCl_3$), δ ppm:

1.41 (9H, singlet);
2.06 (2H, quintet, J = 7 Hz);
2.27 (3H, singlet);
2.50 (3H, singlet);
2.87 (3H, singlet);
3.44 (2H, triplet, J = 7 Hz);
4.08 (2H, triplet, J = 8.5 Hz);
6.86 (1H, doublet, J = 8.5 Hz);
7.44 (1H, doublet, J = 8.5 Hz).

10(b) 3-Chloro-4,8-dimethyl-7-(3-N-methylaminopropoxy)coumarin hydrochloride

A procedure similar to that described in Example 8(b) was repeated, except that 0.90 g of 7-[3-(N-t-butoxycarbonyl-N-methylamino)propoxy]-3-chloro-4,8-dimethylcoumarin [prepared as described in step (a) above] was employed, to give 0.73 g of the title compound as crystals, whioh softened above 245 °C and melted at 273 °C (with colouration).
Nuclear Magnetic Resonance Spectrum (hexadeuterated dimethyl sulphoxide), δ ppm:

2.0 - 2.35 (2H, multiplet);
2.21 (3H, singlet);
2.54 (6H, singlet);
2.95 - 3.2 (2H, multiplet);
4.24 (2H, triplet, J = 7.5 Hz);
7.10 (1H, doublet, J = 8.5 Hz);
7.68 (1H, doublet, J = 8.5 Hz).

EXAMPLE 11

3,4-Dimethyl-7-(4-N-methylaminobutoxy)coumarin hydrochloride

11(a) 7-[4-(N-t-Butoxycarbonyl-N-methylamino)butoxy]-3,4-dimethylcoumarin

A procedure similar to that described in Example 8(a) was repeated, except that 1.00 g of 3,4-dimethyl-7-hydroxycoumarin, 2.4 g of 4-(N-t-butoxycarbonyl-N-methylamino)butyl p-nitrobenzenesulphonate and 2.83 g of potassium carbonate were employed, to give 2.06 g of the title compound as a syrup after purification by silica gel column chromatography using a 1 : 2 by volume mixture of ethyl acetate and hexane as the eluent.

Nuclear Magnetic Resonance Spectrum ($CDCl_3$), δ ppm:

1.44 (9H, singlet);
1.65 - 1.85 (4H, multiplet);
2.17 (3H, singlet);
2.36 (3H, singlet);
2.86 (3H, singlet);
3.18 - 3.41 (2H, multiplet);
3.92 - 4.13 (2H, multiplet);
6.78 - 6.96 (2H, multiplet);
7.42 - 7.60 (1H, multiplet).

11(b) 3,4-Dimethyl-7-(4-N-methylaminobutoxy)coumarin hydrochloride

A procedure similar to that described in Example 8(b) was repeated, except that 2.00 g of 7-[4-N-t-butoxycarbonyl-N-methylamino)butoxy]-3,4-dimethylcoumarin [prepared as described in step (a) above] were employed, to give 1.24 g of the title compound as crystals, melting at 196 - 198 °C.

Nuclear Magnetic Resonance Spectrum (hexadeuterated dimethyl sulphoxide), δ ppm:

1.8 - 1.9 (4H, multiplet);
2.08 (3H, singlet);
2.37 (3H, singlet);
2.54 (3H, singlet);
2.8 - 3.05 (2H, multiplet);
4.0 - 4.2 (2H, multiplet);
6.85 - 7.05 (2H, multiplet);
7.65 - 7.76 (1H, multiplet).

EXAMPLE 12

7-(3-N-Ethylaminopropoxy)-3,4,8-trimethylcoumarin hydrochloride

12(a) 7-[3-(N-t-Butoxycarbonyl-N-ethylamino)propoxy]-3,4,8-trimethylcoumarin

A procedure similar to that described in Example 8(a) was repeated, except that 1.0 g of 7-hydroxy-3,4,8-trimethylcoumarin, 2.3 g of 3-(N-t-butoxycarbonyl-N-ethylamino)propyl p-toluenesulphonate and 3.0 g of potassium carbonate were employed, to give 1.20 g of the title compound as crystals, melting at 107 °C, after purification by silica gel column chromatography using a 1 : 2 by volume mixture of ethyl acetate and hexane as the eluent.

Nuclear Magnetic Resonance Spectrum ($CDCl_3$), δ ppm:

1.11 (3H, triplet, J = 7 Hz);

1.43 (9H, singlet);
1.85 - 2.25 (2H, multiplet);
2.17 (3H, singlet);
2.29 (3H, singlet);
2.34 (3H, singlet);
3.25 (2H, quartet, J = 7 Hz);
3.41 (2H, triplet, J = 7 Hz);
4.06 (2H, triplet, J = 7 Hz);
6.81 (1H, doublet, J = 10 Hz);
7.42 (1H, doublet, J = 10 Hz).

12(b) 7-(3-N-Ethylaminopropoxy)-3,4,8-trimethylcoumarin hydrochloride

A procedure similar to that described in Example 8(b) was repeated, except that 0.90 g of [3-(N-t-butoxycarbonyl-N-ethylamino)propoxy]-3,4,8-trimethylcoumarin [prepared as described in step (a) above] was employed, to give 0.74 g of the title compound as crystals, melting at 245°C.
Nuclear Magnetic Resonance Spectrum (hexadeuterated dimethyl sulphoxide), δ ppm:
1.27 (3H, triplet, J = 7 Hz);
1.9 - 2.4 (2H, multiplet);
2.08 (3H, singlet);
2.20 (3H, singlet);
2.33 (3H, multiplet);
2.7 - 3.3 (4H, multiplet);
4.20 (2H, triplet, J = 6 Hz);
6.98 (1H, doublet, J = 9.5 Hz);
7.55 (1H, doublet, J = 9.5 Hz);
9.30 (2H, broad singlet).

EXAMPLE 13

7-(3-N-Isopropylaminopropoxy)-3,4,8-trimethylcoumarin hydrochloride

13(a) 7-[3-(N-t-Butoxycarbonyl-N-isopropylamino)propoxy]-3,4,8-trimethylcoumarin

0.23 ml of acetone and 0.30 ml of acetic acid were added to 15 ml of a methanolic solution containing 642 mg of 7-(3-aminopropoxy)-3,4,8-trimethylcoumarin (prepared as described in Example 17, hereafter). Whilst keeping the mixture on an ice bath, 157 mg of sodium cyanoborohydride were then added. The mixture was then stirred for 2 hours at room temperature, after which it was condensed by evaporation under reduced pressure. A saturated aqueous solution of sodium bicarbonate was then added, to give the desired free amino compound as a precipitate. This precipitate was collected by filtration and suspended in ethyl acetate. 0.42 ml of triethylamine and 643 mg of di-t-butyl pyrocarbonate were then added to the suspension, and the reaction mixture was stirred for 3 hours. At the end of this time, it was condensed by evaporation under reduced pressure. The residue was purified by silica gel column chromatography using a 1 : 9 by volume mixture of ethyl acetate and methylene chloride as the eluent, to obtain 0.57 g of 7-[3-(N-t-butoxycarbonyl-N-isopropylamino)propoxy]-3,4,8-trimethylcoumarin as crystals, melting at 132°C.
Nuclear Magnetic Resonance Spectrum ($CDC\ell_3$), δ ppm:
1.17 (6H, doublet, J = 7 Hz);
1.50 (9H, singlet);
1.95 - 2.25 (2H, multiplet);
2.20 (3H, singlet);
2.33 (3H, singlet);
2.36 (3H, singlet);
3.31 (2H, doublet of doublets, J = 6 & 7.5 Hz);
4.08 (2H, triplet, J = 7 Hz);
4.0 - 4.8 (1H, multiplet);
6.81 (1H, doublet, J = 8.5 Hz);
7.42 (1H, doublet, J = 8.5 Hz).

13(b) 7-(3-N-Isopropylaminopropoxy)-3,4,8-trimethylcoumarin hydrochloride

A procedure similar to that described in Example 8(b) was repeated, except that 0.52 g of 7-[3-(N-t-butoxycarbonyl-N-isopropylamino)propoxy]-3,4,7-trimethylcoumarin [prepared as described in step (a) above] were employed, to give 0.44 g of the title compound as crystals, which softened above 270 °C and melted at 286 °C.

Nuclear Magnetic Resonance Spectrum ($D_2O$), δ ppm:

1.89 (6H, doublet, J = 6.5 Hz);
2.19 (3H, singlet);
2.05 - 2.4 (2H, multiplet);
2.33 (6H, singlet);
3.7 - 3.9 (2H, multiplet);
3.85 - 4.2 (1H, multiplet);
4.45 - 4.6 (2H, multiplet);
7.07 (1H, doublet, J = 8.5 Hz);
7.43 (1H, doublet, J = 8.5 Hz).

EXAMPLE 14

7-(3-N-Methylaminopropoxy)coumarin fumarate

Procedures similar to those described in Examples 8(a) and 8(b) were repeated, except that 1.3 g of 7-hydroxycoumarin was employed, to give 2.1 g of the hydrochloride of the title compound as very hygroscopic crystals. This hydrochloride was dissolved in 10 ml of water. The resulting solution was neutralized by the addition of a saturated aqueous solution of sodium bicarbonate and was then extracted with methylene chloride. The methylene chloride extract was dried over anhydrous magnesium sulphate, and the solvent was removed by distillation under reduced pressure, to give 1.66 g of the free coumarin derivative corresponding to the title compound as a gum. This gum was treated with 0.83 g of fumaric acid, and the product was washed first with diethyl ether and then with ethyl acetate, to obtain 2.30 g of the title compound as crystals, melting at 108 °C (with softening).

Nuclear Magnetic Resonance Spectrum (hexadeuterated dimethyl sulphoxide), δ ppm:

1.9 - 2.35 (2H, multiplet);
2.56 (3H, singlet);
3.05 (2H, triplet, J = 8 Hz);
4.18 (2H, triplet, J = 6 Hz);
6.28 (1H, doublet, J = 9.5 Hz);
6.50 (2H, singlet);
6.8 - 7.05 (2H, multiplet);
7.64 (1H, doublet, J = 9.5 Hz);
7.98 (1H, doublet, J = 9.5 Hz).

EXAMPLE 15

3,4-Dimethyl-7-(3-N-methylaminopropoxy)coumarin hydrochloride

Procedures similar to those described in Examples 8(a) and 8(b) were repeated, except that 2.88 g of 3-(N-t-butoxycarbonyl-N-methylamino)propyl p-toluenesulphonate and 1.2 g of 7-hydroxy-3,4-dimethylcoumarin were employed, to give 1.69 g of the title compound as crystals, melting at 180 - 182 °C.

Nuclear Magnetic Resonance Spectrum (hexadeuterated dimethyl sulphoxide), δ ppm:

1.95 - 2.3 (2H, multiplet);
2.09 (3H, singlet);
2.37 (3H, singlet);
2.57 (3H, singlet);
3.06 (2H, triplet, J = 7.5 Hz);
4.20 (2H, triplet, J = 6 Hz);
6.9 - 7.05 (2H, multiplet);
7.71 (1H, doublet, J = 9.5 Hz);
9.23 (2H, broad singlet).

EXAMPLE 16

7-(3-N,N-Dimethylaminopropoxy)-3,4,8-trimethylcoumarin and its hydrochloride

1.2 g of 7-hydroxy-3,4,8-trimethylcoumarin, 1.0 g of 3-(dimethylamino)propyl chloride hydrochloride and 1.5 g of potassium carbonate were added to 30 ml of methyl ethyl ketone. The mixture was then stirred for 9 hours whilst being heated under reflux. At the end of this time, the reaction solution was condensed by evaporation under reduced pressure. The residue was dissolved in ethyl acetate and water. The ethyl acetate layer was separated and dried over anhydrous magnesium sulphate. The solvent was then removed by distillation under reduced pressure, and the crystals which separated were washed with diethyl ether, to afford 0.98 g of the title compound as crystals, melting at 86 °C.

Nuclear Magnetic Resonance Spectrum ($CDCℓ_3$), δ ppm:

1.8 - 2.6 (4H, multiplet);
2.16 (3H, singlet);
2.27 (3H, singlet);
2.30 (3H, singlet);
2.33 (3H, singlet);
4.09 (2H, triplet, J = 6 Hz);
6.79 (1H, doublet, J = 9 Hz);
7.36 (1H, doublet, J = 9 Hz).

0.90 g of these crystals were reacted with a 4N solution of hydrogen chloride in ethyl acetate, to yield 1.05 g of the hydrochloride of the title compound, softening above 220 °C and melting at 252 °C.

Nuclear Magnetic Resonance Spectrum (hexadeuterated dimethyl sulphoxide), δ ppm:

2.09 (3H, singlet);
2.05 - 2.35 (2H, multiplet);
2.23 (3H, singlet);
2.37 (3H, singlet);
2.77 (3H, singlet);
2.83 (3H, singlet);
3.1 - 3.4 (2H, multiplet);
4.21 (2H, triplet, J = 6 Hz);
7.05 (1H, doublet, J = 9 Hz);
7.62 (1H, doublet, J = 9 Hz).

EXAMPLE 17

7-(3-Aminopropoxy)-3,4,8-trimethylcoumarin hydrochloride

17(a) 7-[3-(N-t-Butoxycarbonylamino)propoxy]-3,4,8-trimethylcoumarin

4 g of potassium carbonate were added to a solution of 2.7 g of 7-hydroxy-3,4,8-trimethylcoumarin and 3.8 g of 3-(N-t-butoxycarbonylamino)propyl bromide in 50 ml of dimethylformamide. The mixture was stirred at 65 °C for 5 hours, after which ethyl acetate and water were added. The ethyl acetate layer was separated, dried over anhydrous magnesium sulphate and then concentrated by evaporation *in vacuo*. The resulting residue was recrystallized from ethanol, to give 3.80 g of the title compound as crystals, melting at 137 °C.

Nuclear Magnetic Resonance Spectrum ($CDCℓ_3$), δ ppm:

1.45 (9H, singlet);
1.8 - 2.3 (2H, multiplet);
2.15 (3H, singlet);
2.29 (3H, singlet);
2.32 (3H, singlet);
3.35 (2H, quartet, J = 6 Hz);
4.10 (2H, triplet, J = 6 Hz);
4.95 (1H, broad singlet);
6.78 (1H, doublet, J = 9 Hz);
7.35 (1H, doublet, J = 9 Hz).

17(b) 7-(3-Aminopropoxy)-3,4,8-trimethylcoumarin hydrochloride

30 ml of a 4N solution of hydrogen chloride in ethyl acetate were added to a hot solution of 7-[3-(N-t-butoxycarbonylamino)propoxy]-3,4,8-trimethylcoumarin [prepared as described in step (a) above] in 40 ml of ethyl acetate, and the mixture was stirred for 4 hours at room temperature. At the end of this time, the reaction mixture was concentrated by evaporation in vacuo, to give the title compound as crystals. This product was recrystallized from 80% by volume aqueous ethanol, to give 2.88 g of the title compound as crystals, melting at 260 °C.
Nuclear Magnetic Resonance Spectrum (hexadeuterated dimethyl sulphoxide), δ ppm:

1.95 - 2.35 (2H, multiplet);
2.08 (3H, singlet);
2.21 (3H, singlet);
2.37 (3H, singlet);
2.8 - 3.15 (2H, multiplet);
4.22 (2H, triplet, J = 6 Hz);
7.03 (1H, doublet, J = 9 Hz);
7.62 (1H, doublet, J = 9 Hz);
8.27 (3H, broad singlet).

EXAMPLE 18

7-(4-Aminobutoxy)-3,4-dimethylcoumarin hydrochloride

Procedures similar to those described in Examples 8(a) and 8(b) were repeated, except that 3.12 g of 4-(N-t-butoxycarbonylamino)butyl p-toluenesulphonate and 1.3 g of 7-hydroxy-3,4-dimethylcoumarin were employed, to give 1.87 g of the title compound, whioh softened at 190 °C and melted at 215 °C.
Nuclear Magnetic Resonance Spectrum (hexadeuterated dimethyl sulphoxide), δ ppm:

1.65 - 1.95 (4H, multiplet);
2.08 (3H, singlet);
2.36 (3H, singlet);
2.7 - 3.0 (2H, multiplet);
4.0 - 4.25 (2H, multiplet);
6.85 - 7.05 (2H, multiplet);
7.68 (1H, doublet, J = 9 Hz);
8.22 (3H, broad singlet).

EXAMPLE 19

7-(4-Aminobutoxy)-3,4,8-trimethylcoumarinhydrochloride

Procedures similar to those described in Examples 8(a) and 8(b) were repeated, except that 2.91 g of 4-(N-t-butoxycarbonylamino)butyl p-toluenesulphonate and 1.3 g of 7-hydroxy-3,4,8-trimethylcoumarin were employed, to give 1.71 g of the title compound, melting at 245 - 247 °C.
Nuclear Magnetic Resonance Spectrum (hexadeuterated dimethyl sulphoxide), δ ppm:

1.65 - 2.05 (4H, multiplet);
2.06 (3H, singlet);
2.20 (3H, singlet);
2.34 (3H, singlet);
2.7 - 3.1 (2H, multiplet);
4.0 - 4.3 (2H, multiplet);
7.02 (1H, doublet, J = 9 Hz);
7.59 (1H, doublet, J = 9 Hz);
8.20 (3H, broad singlet).

EXAMPLES 20 TO 24

Following a procedure similar to that described in Example 4(a) and 4(b), the following compounds were obtained.

EXAMPLE 20

4-Ethyl-7-(2-morpholinyl)methoxycoumarin hydrochloride

Melting at 115 °C (with decomposition).
Nuclear Magnetic Resonance Spectrum (hexadeuterated dimethyl sulphoxide), δ ppm:
1.23 (3H, triplet, J = 7.5 Hz);
2.80 (2H, quartet, J = 7.5 Hz);
2.90 - 4.37 (9H, multiplet);
6.17 (1H, singlet);
6.97 - 7.02 (2H, multiplet);
7.74 (1H, doublet, J = 9 Hz);
9.74 (2H, broad singlet).

EXAMPLE 21

7-(2-Morpholinyl)methoxy-3,4,5-trimethylcoumarin hydrochloride

Melting at 250 °C (colouration), 265 °C (with decomposition).
Nuclear Magnetic Resonance Spectrum ($D_2O$), δ ppm:
2.23 (3H, singlet);
2.57 (3H, singlet);
2.66 (3H, singlet);
3.5 - 4.8 (9H, multiplet);
6.73 (2H, broad singlet).

EXAMPLE 22

3-Chloro-4,8-dimethyl-7-(2-morpholinyl)methoxycoumarin hydrochloride

Melting at 265 °C (colouration), 275 °C (with decomposition).
Nuclear Magnetic Resonance Spectrum ($D_2O$), δ ppm:
1.88 (3H, singlet);
2.08 (3H, singlet);
2.8 - 4.6 (9H, multiplet);
6.76 (1H, doublet, J = 8 Hz);
7.12 (1H, doublet, J = 8 Hz).

EXAMPLE 23

3,4-Dimethyl-7-(2-morpholinyl)methoxycoumarin hydrochloride

Melting at 225 - 226 °C (with decomposition).
Nuclear Magnetic Resonance Spectrum (hexadeuterated dimethyl sulphoxide), δ ppm:
2.04 (3H, singlet);
2.32 (3H, singlet);
2.7 - 4.4 (9H, multiplet);
6.8 - 7.1 (2H, multiplet);
7.67 (1H, doublet, J = 9 Hz);
9.90 (2H, broad singlet).

EXAMPLE 24

8-Methyl-7-(2-morpholinyl)methoxycoumarin hydrochloride

Melting at 233 °C (colouration), 244 - 246 °C (with decomposition).
Nuclear Magnetic Resonance Spectrum ($D_2O$), δ ppm:
2.43 (3H, singlet);

3.6 - 4.9 (9H, multiplet);
6.52 (1H, doublet, J = 9 Hz);
7.22 (1H, doublet, J = 9 Hz);
8.11 (1H, doublet, J = 9.5 Hz).

EXAMPLE 25

3,4,8-Trimethyl-7-(4-methyl-2-morpholinyl)methoxycoumarin hydrochloride

2 ml of 35% v/v aqueous formaldehyde were added to 20 ml of a methanolic solution containing 1.1 g of 3,4,8-trimethyl-7-(2-morpholinyl)methoxycoumarin hydrochloride (prepared as described in Example 4). 0.21 g of sodium cyanoborohydride was then added to the mixture on an ice bath, and the mixture was stirred for 4 hours. At the end of this time, the reaction mixture was condensed by evaporation under reduced pressure. The resulting residue was dissolved in methylene chloride and a saturated aqueous solution of sodium bicarbonate. The organic layer was separated and dried over anhydrous magnesium sulphate. The solvent was then removed by distillation under reduced pressure. The resulting residue was purified by silica gel column chromatography using a 1 : 9 by volume mixture of methanol and methylene chloride as the eluent, after which it was treated with a 4N solution of hydrogen chloride in ethyl acetate, to obtain 0.85 g of the title compound, melting at 248 - 250 °C.
Nuclear Magnetic Resonance Spectrum (hexadeuterated dimethyl sulphoxide), δ ppm:
2.06 (3H, singlet);
2.20 (3H, singlet);
2.33 (3H, singlet);
2.85 (3H, singlet);
2.7 - 4.4 (9H, multiplet);
6.98 (1H, doublet, J = 9 Hz);
7.54 (1H, doublet, J = 9 Hz).

EXAMPLE 26

3,4,8-Trimethyl-7-[(S)-(2-morpholinyl)methoxy]coumarin hydrochloride

26(a) 7-[(S)-4-t-Butoxycarbonyl-(2-morpholinyl)methoxy]-3,4,8-trimethylcoumarin

A procedure similar to that described in Example 4(a) was repeated, except that 0.63 g of 7-hydroxy-3,4,8-trimethylcoumarin and 1.25 g of (S)-4-t-butoxycarbonyl-2-[(4-nitrophenylsulphonyloxy)methyl]-morpholine (prepared as described in Preparation 5), were employed, to give 1.11 g of the title compound as crystals, melting at 137 - 139 °C.
Optical rotation $[\alpha]_D^{25}$ + 23.7°, (c = 1, dimethylformamide).
The Nuclear Magnetic Resonance Spectrum of this compound was the same as that of the product of Example 4(a).

26(b) 3,4,8-Trimethyl-7-[(S)-(2-morpholinyl)methoxy]coumarin hydrochloride

A procedure similar to that described in Example 4(b) was repeated, except that 1.00 g of 7-[(S)-4-t-butoxycarbonyl-(2-morpholinyl)methoxy]-3,4,8-trimethylcoumarin [prepared as described in step (a) above] was employed, to give 0.70 g of the title compound as crystals, melting at 226 - 228 °C.
Optical rotation $[\alpha]_D^{25}$ + 2.0°, (c = 1, $H_2O$).
The Nuclear Magnetic Resonance Spectrum of this compound was the same as that of the product of Example 4(b).

EXAMPLE 27

7-[(S)-(2-Morpholinyl)methoxy]coumarin hydrochloride

A procedure similar to that described in Example 26 was repeated, except that 0.65 g of 7-hydroxycoumarin and 1.60 g of (S)-4-t-butoxycarbonyl-2-[(4-nitrophenylsulphonyloxy)methyl]morpholine (prepared as described in Preparation 5) were employed, to give 1.05 g of the title compound as crystals,

melting at 186 - 188 °C.
Optical rotation $[\alpha]_D^{25}$ + 23.6 °, (c = 1, dimethylformamide).

The Nuclear Magnetic Resonance Spectrum of this compound was the same as that of the product of Example 1(b).

EXAMPLE 28

3,4-Dimethyl-7[(S)-(2-morpholinyl)methoxy]coumarin hydrochloride

A procedure similar to that described in Example 26, was repeated, except that 0.90 g of 7-hydroxy-3,4-dimethylcoumarin and 1.90 g of (S)-4-t-butoxycarbonyl-2-[(4-nitrophenylsulphonyloxy)methyl]morpholine (prepared as described in Preparation 5) were employed, to give 1.37 g of the title compound as crystals, melting at 226 - 228 °C.
Optical rotation $[\alpha]_D^{25}$ + 9.8 °, (c = 1, dimethylformamide).

The Nuclear Magnetic Resonance Spectrum of this compound was the same as that of the product of Example 23.

EXAMPLE 29

3,4,8-Trimethyl-7[(R)-(2-morpholinyl)methoxy]coumarin hydrochloride

A procedure similar to that desoribed in Example 26 was repeated, except that 2.54 g of 7-hydroxy-3,4,8-trimethylcoumarin and 5.0 g of (R)-4-t-butoxycarbonyl-2-[(4-nitrophenylsulphonyloxy)methyl]-morpholine (prepared as described in Preparation 3) were employed, to give 3.40 g of the title compound as crystals, melting at 225 - 226 °C.
Optical rotation $[\alpha]_D^{25}$ - 1.8 °, (c = 1, $H_2O$).

The Nuclear Magnetic Resonance Spectrum of this compound was the same as that of the product of Example 4(b).

EXAMPLE 30

7[(R)-(2-Morpholinyl)methoxy]coumarin hydrochloride

A procedure similar to that described in Example 26 waa repeated, except that 0.81 g of 7-hydroxycoumarin and 1.86 g of (R)-4-t-butoxycarbonyl-2-[(4-methylphenylsulphonyloxy)methyl]morpholine (prepared as described in Preparation 4) were employed, to give 1.05 g of the title compound as crystals, melting at 187 - 189 °C.
Optical rotation $[\alpha]_D^{25}$ - 22.4 °, (c = 1, dimethylformamide).

The Nuclear Magnetic Resonance Spectrum of this compound was the same as that of the product of Example 1(b).

EXAMPLE 31

3,4-Dimethyl-7[(R)-(2-morpholinyl)methoxy]coumarin hydrochloride

A procedure similar to that described in Example 26 was repeated, except that 0.90 g of 3,4-dimethyl-7-hydroxycoumarin and 1.80 g of (R)-4-t-butoxycarbonyl-2-[(4-methylsulphonyloxy)methyl]morpholine (prepared as described in Preparation 4) were employed, to give 1.40 g of the title compound as crystals, melting at 225 - 228 °C.
Optical rotation $[\alpha]_D^{25}$ - 8.8 °, (c = 1, $H_2O$).

The Nuclear Magnetic Resonance Spectrum of this compound was the same as that of the product of Example 23.

EXAMPLE 32

7-[(RS)-3-Aminobutoxy]-3,4,8-trimethylcoumarin hydrochloride

32(a) 7-[(RS)-3-(t-Butoxycarbonylamino)butoxy]-3,4,8-trimethylcoumarin

4.02 g of potassium carbonate were added to 20 ml of a dimethylformamide solution containing 1.32 g of 7-hydroxy-3,4,8-trimethylcoumarin and 2.67 g of 3-(t-butoxycarbonylamino)butyl p-toluenesulphonate. The reaction mixture was then stirred for 5 hours at 80 °C, after which ethyl acetate and water were added. The ethyl acetate layer was separated and dried over anhydrous magnesium sulphate, and the solvent was then removed by distillation under reduced pressure. The crystalline residue was purified by silica gel column chromatography using a 2 : 5 by volume mixture of ethyl acetate and hexane as the eluent, to obtain 1.87 g of the title compound as crystals, melting at 132 - 134 °C.

Nuclear Magnetic Resonance Spectrum ($CDCl_3$) δ ppm:

1.23 (3H, doublet, J = 7 Hz);
1.43 (9H, singlet);
1.8 - 2.3 (2H, multiplet);
2.16 (3H, singlet);
2.30 (3H, singlet);
2.34 (3H, singlet);
3.55 - 4.3 (3H, multiplet);
4.65 (1H, broad doublet, J = 7 Hz);
6.77 (1H, doublet, J = 8.5 Hz);
7.36 (1H, doublet, J = 8.5 Hz).

32(b) 7-[(RS)-3-Aminobutoxy]-3,4,8-trimethylcoumarin hydrochloride

20 ml of a 4N solution of hydrogen chloride in ethyl acetate were added to 1.87 g of 7-[(RS)-3-(t-butoxycarbonylamino)butoxy]-3,4,8-trimethylcoumarin [prepared as described in step (a) above]. The reaction mixture was then stirred for 4 hours at room temperature and then the title compound which had separated in the reaction mixture was obtained by filtration and washed with a mixture of ethyl acetate and diethyl ether. Recrystallization from 90% v/v aqueous ethanol afforded 1.26 g of the title compound, melting at 226 - 227 °C.

Nuclear Magnetic Resonance Spectrum (hexadeuterated dimethyl sulphoxide) δ ppm:

1.31 (3H, doublet, J = 6 Hz);
1.9 - 2.3 (2H, multiplet);
2.06 (3H, singlet);
2.18 (3H, singlet);
2.34 (3H, singlet);
3.3 - 3.65 (1H, multiplet);
4.21 (2H, triplet, J = 6 Hz);
7.03 (1H, doublet, J = 9 Hz);
7.61 (1H, doublet, J = 9 Hz);
8.30 (3H, broad singlet).

EXAMPLE 33

7-[(RS)-3-Aminopentoxy]-3,4,8-trimethylcoumarin hydrochloride

33(a) 7-[(RS)-3-(t-Butoxycarbonylamino)pentoxy]-3,4,8-trimethylcoumarin

2 ml of a methylene chloride solution containing 2.26 g of diethyl azodicarboxylate were added dropwise to 30 ml of a methylene chloride solution containing 2.04 g of 7-hydroxy-3,4,8-trimethylcoumarin, 2.58 g of 3-(t-butoxycarbonylamino)pentanol and 3.40 g of triphenylphosphine on an ice bath. The reaction mixture was then stirred for 2 hours at room temperature. At the end of this time, the solvent was removed by distillation under reduced pressure. The residue was purified by silica gel column chromatography using a 2 : 3 by volume mixture of ethyl acetate and hexane as the eluent, to obtain 3.65 g of the title compound as crystals, melting at 143 °C.

Nuclear Magnetic Resonance Spectrum ($CDCl_3$) δ ppm:
0.95 (3H, triplet, J = 7 Hz);
1.39 (9H, singlet);
1.4 - 2.1 (4H, multiplet);
2.14 (3H, singlet);
2.28 (3H, singlet);
2.32 (3H, singlet);
3.5 - 3.9 (1H, multiplet);
4.12 (2H, triplet, J = 6 Hz);
6.80 (1H, doublet, J = 9 Hz);
7.38 (1H, doublet, J = 9 Hz).

33(b) 7-[(RS)-3-Aminopentoxy]-3,4,8-trimethylcoumarin hydrochloride

A procedure similar to that described in Example 32(a) was repeated, except that 3.30 g of 7-[(RS)-3-(t-butoxycarbonylamino)pentoxy]-3,4,8-trimethylcoumarin [prepared as described in step (a) above] were employed, to give 2.04 g of the title compound as crystals, melting at 242 - 245 °C.
Nuclear Magnetic Resonance Spectrum ($D_2O$), δ ppm:
1.54 (3H, triplet, J = 7 Hz);
2.13 (3H, singlet);
2.25 (3H, singlet);
2.30 (3H, singlet);
2.25 - 2.45 (2H, multiplet);
2.5 - 2.8 (2H, multiplet);
3.85 - 4.1 (1H, multiplet);
4.4 - 4.7 (2H, multiplet);
7.08 (1H, doublet, J = 9 Hz);
7.41 (1H, doublet, J = 9 Hz).

EXAMPLES 34 TO 43

Following a procedure similar to that described in Example 33, the following compounds were obtained.

EXAMPLE 34

7-[(S)-3-Aminobutoxy]-3,4,8-trimethylcoumarin hydrochloride

Melting at 232 - 234 °C.
Optical rotation $[\alpha]_D^{25}$ -5.8 ° (c = 1, $H_2O$).
The Nuclear Magnetic Resonance Spectrum of this compound was observed to accord with that of the compound prepared as described in Example 32(b).

EXAMPLE 35

7-[(R)-3-Aminobutoxy]-3,4,8-trimethylcoumarin hydrochloride

Melting at 232 - 234 °C.
Optical rotation $[\alpha]_D^{25}$ -5.5 ° (c = 1, $H_2O$).
The Nuclear Magnetic Resonance Spectrum of this compound was observed to accord with that of the compound prepared in Example 32(b).

EXAMPLE 36

7-[(RS)-3-Amino-2-methylpropoxy]-3,4,8-trimethylcoumarin hydrochloride

Softened at 230 °C. Decomposed at 238 - 241 °C.
Nuclear Magnetic Resonance Spectrum (hexadeuterated dimethyl sulphoxide), δ ppm:
1.12 (3H, doublet, J = 7 Hz);

2.0 - 2.4 (1H, multiplet);
2.08 (3H, singlet);
2.21 (3H, singlet);
2.36 (3H, singlet);
2.7 - 3.15 (2H, multiplet);
4.07 (2H, doublet, J = 6 Hz);
7.02 (1H, doublet, J = 9 Hz);
7.61 (1H, doublet, J = 9 Hz);
8.30 (3H, broad singlet).

EXAMPLE 37

7-[(RS)-3-Amino-4-methylpentoxy]-3,4,8-trimethylcoumarin hydrochloride

Softened at 240 °C. Decomposed at 243-245 °C.
Nuclear Magnetic Resonance Spectrum (hexadeuterated dimethyl sulphoxide) δ ppm:
1.00 (6H, doublet, J = 6.5 Hz);
1.85 - 2.20 (3H, multiplet);
2.09 (3H, singlet);
2.21 (3H, singlet);
2.36 (3H, singlet);
3.05 - 3.40 (1H, multiplet);
4.28 (2H, triplet, J = 6 Hz);
7.05 (1H, doublet, J = 9 Hz);
7.62 (1H, doublet, J = 9 Hz);
8.23 (3H, broad singlet).

EXAMPLE 38

7-[(RS)-trans-(2-Aminocyclohexyl)methoxy]-3,4,8-trimethylcoumarin hydrochloride

Melting at 293 - 296 °C (with decomposition).
Nuclear Magnetic Resonance Spectrum (hexadeuterated dimethyl sulphoxide) δ ppm:
1.0 - 2.3 (9H, multiplet);
2.06 (3H, singlet);
2.21 (3H, singlet);
2.35 (3H, singlet);
2.7 - 3.3 (1H, multiplet);
4.0 - 4.3 (2H, multiplet);
7.05 (1H, doublet, J = 9 Hz);
7.61 (1H, doublet, J = 9 Hz);
8.38 (3H, broad singlet).

EXAMPLE 39

7-[(RS)-cis-(2-Aminocyclohexyl)methoxy]-3,4,8-trimethylcoumarin hydrochloride

Melting at 261 - 265 °C (with decomposition).
Nuclear Magnetic Resonance Spectrum (hexadeuterated dimethyl sulphoxide) δ ppm:
1.1 - 2.1 (9H, multiplet);
2.09 (3H, singlet);
2.22 (3H, singlet);
2.36 (3H, singlet);
3.2 - 3.6 (1H, multiplet);
4.0-4.2 (2H, multiplet);
7.05 (1H, doublet, J = 9 Hz);
7.58 (1H, doublet, J = 9 Hz);
8.30 (3H, broad singlet).

EXAMPLE 40

7-[(RS)-trans or cis-(2-Aminocyclopentyl)methoxy]-3,4,8-trimethylcoumarin hydrochloride

Melting at 264°C (colouration), 265 - 269°C (with decomposition).
Nuclear Magnetic Resonance Spectrum ($D_2O$), δ ppm:
2.16 (3H, singlet);
2.28 (3H, singlet);
2.33 (3H, singlet);
2.1 - 3.2 (7H, multiplet);
4.0 - 4.25 (1H, multiplet);
4.46 (2H, doublet, J = 6 Hz);
7.08 (1H, doublet, J = 9 Hz);
7.44 (1H, doublet, J = 9 Hz).

EXAMPLE 41

7-[(RS)-trans or cis-(2-Aminocyclopentyl)methoxy]-3,4,8-trimethylcoumarin hydrochloride

This is the diastereoisomer of the compound prepared as described in Example 40.
Melting at 260°C (with decomposition).
Nuclear Magnetic Resonance Spectrum ($D_2O$), δ ppm:
2.1 - 2.8 (6H, multiplet);
2.20 (3H, singlet);
2.37 (3H, singlet);
2.40 (3H, singlet);
3.0 - 3.3 (1H, multiplet);
4.3 - 4.45 (1H, multiplet);
4.60 (2H, doublet, J = 6 Hz);
7.22 (1H, doublet, J = 9 Hz);
7.58 (1H, doublet, J = 9 Hz).

EXAMPLE 42

7-[(RS)-3-(Methylamino)pentoxy]-3,4,8-trimethylcoumarin hydrochloride

Melting at 218 - 220°C.
Nuclear Magnetic Resonance Spectrum ($D_2O$), δ ppm:
1.52 (3H, triplet, J = 7 Hz);
2.15 (31, singlet);
2.2 - 2.45 (2H, multiplet);
2.26 (3H, singlet);
2.32 (3H, singlet);
2.55 - 2.8 (2H, multiplet);
3.7 - 3.95 (1H, multiplet);
4.4 - 4.65 (2H, multiplet);
7.09 (1H, doublet, J = 9 Hz);
7.42 (1H, doublet, J = 9 Hz).

EXAMPLE 43

7-[(RS)-3-(Dimethylamino)butoxy]-3,4,8-trimethylcoumarin hydrochloride

Melting at 220 - 222°C.
Nuclear Magnetic Resonance Spectrum (hexadeuterated dimethyl sulphoxide) δ ppm:
1.34 (3H, doublet, J = 7 Hz);
1.8 - 2.5 (3H, multiplet);
2.06 (3H, singlet);

2.20 (3H, singlet);
2.33 (3H, singlet);
2.66 (3H, singlet);
2.73 (3H, singlet);
3.1 - 3.7 (1H, multiplet);
4.20 (2H, triplet, J = 6 Hz);
7.04 (1H, doublet, J = 9 Hz);
7.60 (1H, doublet, J = 9 Hz);
11.10 (1H, broad singlet).

PREPARATION 1

4-t-Butoxycarbonyl-2-(4-methylphenylsulphonyloxy)methylmorpholine

68 ml of triethylamine, followed by 100 ml of methylene chloride containing 102 g of di-t-butyl pyrocarbonate, were added to a mixture of 100 ml of methanol and 200 ml of methylene chloride containing 52 g of 2-hydroxymethylmorpholine [described in Chem. Pharm. Bull. 33 (9), 3766 (1985)], whilst stirring on an ice bath. The reaction mixture was then stirred for an additional 1 hour at room temperature. At the end of this time, the solvent was removed by distillation under reduced pressure. The residue was dissolved in ethyl acetate and water, and the ethyl acetate layer was separated, washed with water and dried over anhydrous magnesium sulphate. The solvent was then removed by distillation under reduced pressure, to give 82.9 g of oily 4-t-butoxycarbonyl-2-hydroxymethylmorpholine.

The whole of this oil was dissolved in a mixture of 150 ml of methylene chloride and 70 ml of triethylamine, and, whilst keeping this solution on an ice bath, 150 ml of methylene chloride containing 73 g of p-toluenesulphonyl chloride were added dropwise. The reaction solution was then stirred for 16 hours at room temperature, after which it was condensed by evaporation under reduced pressure. The residue was dissolved in ethyl acetate, and the resulting solution was washed with water and condensed by evaporation under reduced pressure. Hexane was added to the syrupy residue, in order to assist the title compound to crystallize, and the resulting crystals were then collected by filtration, yielding 56.8 g of the title compound, melting at 85 - 87 °C.

Nuclear Magnetic Resonance Spectrum ($CDC\ell_3$), δ ppm:
1.43 (9H, singlet);
2.42 (3H, singlet);
2.6 - 4.2 (7H, multiplet);
4.00 (2H, doublet, J = 5 Hz);
7.36 (2H, doublet, J = 8 Hz);
7.82 (2H, doublet, J = 8 Hz).

PREPARATION 2

4-t-Butoxycarbonyl-2-[(4-nitrophenylsulphonyloxy)methyl]morpholine

Following a procedure similar to that described in Preparation 1, 40.7 g of 4-t-butoxycarbonyl-2-hydroxymethylmorpholine (which was obtained as the intermediate compound in Preparation 1) were sulphonylated with 47 g of p-nitrobenzenesulphonyl chloride, to yield 61 g of the title compound as crystals, melting at 112 - 113 °C.

Nuclear Magnetic Resonance Spectrum ($CDC\ell_3$), δ ppm:
1.44 (9H, singlet);
2.5 - 4.1 (7H, multiplet);
4.17 (2H doublet, J = 5 Hz);
8.16 (2H, doublet, J = 8 Hz);
8.45 (2H, doublet, J = 8 Hz).

PREPARATION 3

(R)-4-t-Butoxycarbonyl-2-[(4-nitrophenylsulphonyloxy)methyl]morpholine

3(a) (R)-2-(Benzyloxymethyl)morpholine

48 g of 2-aminoethyl hydrogensulphate were added to 18 ml of water containing 13.6 g of sodium hydroxide, and then 10 ml of isopropanol containing 13.9 g of (S)-benzyl-2,3-epoxypropyl ether [described in J. Chem. Soc. (C) 1021 (1967) and Heterocycles 16, 381 (1981)] were added dropwise at 50 °C to this solution. The resulting mixture was then stirred for 1 hour at 50 °C. At the end of this time, 60 ml of water containing 27 g of sodium hydroxide were added to the reaction solution, and the mixture was stirred for 20 hours at 55 °C. Toluene and a small amount of water were then added to the reaction mixture. The toluene layer was separated and dried over anhydrous magnesium sulphate, and the solvent was removed by distillation under reduced pressure. The residue was purified by silica gel column chromatography using a 9 : 1 by volume mixture of methylene chloride and methanol as the eluent, to obtain 10.9 g of the title compound as a syrup.

Optical rotation $[\alpha]_D^{25}$ + 4.5° (neat).

Nuclear Magnetic Resonance Spectrum ($CDC\ell_3$), δ ppm:

2.11 (1H, singlet);
2.55 - 3.1 (4H, multiplet);
3.55 - 4.05 (5H, multiplet);
4.51 (2H, singlet);
7.30 (5H, singlet).

3(b) (R)-2-(Benzyloxymethyl)-4-t-butoxycarbonylmorpholine

7.3 ml of triethylamine were added to 100 ml of methylene chloride containing 10.9 g of (R)-2-(benzyloxymethyl)morpholine [prepared as described in step (a) above], and then 20 ml of methylene chloride containing 12.1 g of di-t-butyl pyrocarbonate was added dropwise to the mixture on an ice bath. The mixture was then stirred for 2 hours at room temperature, after which the reaction mixture was condensed by evaporation under reduced pressure. The residue was purified by silica gel column chromatography using a 1 : 4 by volume mixture of ethyl acetate and hexane as the eluent, to obtain 14.2 g of the title compound as a syrup.

Optical rotation $[\alpha]_D^{25}$ - 17.2° (c = 1, chloroform).

Nuclear Magnetic Resonance Spectrum ($CDC\ell_3$), δ ppm:

1.48 (9H, singlet);
2.5 - 4.05 (9H, multiplet);
4.52 (2H, singlet);
7.32 (5H, singlet).

3(c) (R)-4-t-Butoxycarbonyl-2-(hydroxymethyl)morpholine

4.1 g of 5% w/w palladium-on-carbon were added to 140 ml of an ethanolic solution containing 14.2 g of (R)-2-(benzyloxymethyl)-4-t-butoxycarbonylmorpholine [prepared as described in step (b) above]. The mixture was then stirred for 9 hours at 70 °C in an atmosphere of hydrogen gas under normal atmospheric pressure. At the end of this time, the catalyst was removed by filtration. The solvent was then removed by distillation under reduced pressure, to obtain 10.0 g of the title compound as crystals, melting at 61 - 62 °C.

Optical rotation $[\alpha]_D^{25}$ - 8.1° (c = 1, dimethylformamide).

Nuclear Magnetic Resonance Spectrum ($CDC\ell_3$), δ ppm:

1.43 (9H, singlet);
2.6 (1H, broad singlet);
2.5 - 4.1 (9H, multiplet).

3(d) (R)-4-t-Butoxycarbonyl-2-[(4-nitrophenylsulphonyloxy)methyl]morpholine

A procedure similar to that described in Preparation 1 was repeated, except that 5.6 g of (R)-4-t-butoxycarbonyl-2-(hydroxymethyl)morpholine [prepared as described in step (d) above] and 6.0 g of p-nitrobenzenesulphonyl chloride were employed, to give 10.1 g of the title compound as crystals, melting at

106 - 107 ° C.
Optical rotation $[\alpha]_D^{25}$ - 19.7 ° (c = 1, dimethylformamide).

The Nuclear Magnetic Resonance Spectrum of this compound was the same as that of the product of Preparation 2.

PREPARATION 4

(R)-4-t-Butoxycarbonyl-2-[(4-methylphenylsulphonyloxy)methyl]morpholine

A procedure similar to that described in Preparation 1 was repeated, except that 9.2 g of (R)-4-t-butoxycarbonyl-2-(hydroxymethyl)morpholine [prepared as desoribed in Preparation 3(c) above] and 8.5 g of p-toluenesulphonyl chloride were employed, to give 15.1 g of the title compound as crystals, melting at 105.5 - 107 ° C (after recrystallization from ethanol).
Optical rotation $[\alpha]_D^{25}$ - 19.3 ° (c = 1, dimethylformamide). The Nuclear Magnetic Resonance Spectrum of this compound was the same as that of the product of Preparation 1.

PREPARATION 5

(S)-4-t-Butoxycarbonyl-2-[(4-nitrophenylsulphonyloxy)methyl]morpholine

5(a) (S)-N-Benzyl-3-chloroacetamidopropane-1,2-diol

39.5 g of 1,2,5,6-di-O-isopropylidene-D-mannitol were added to 240 ml of water containing 35 g of sodium periodate on an ice bath, and the reaction mixture was stirred for 1 hour at 5 to 10 ° C. At the end of this time, 240 ml of ethanol were added to the reaction mixture, and the mixture was stirred for a further 1 hour at 5 to 10 ° C. A further 240 ml of ethanol were then added. The resulting precipitate was filtered off, and 35 g of benzylamine and about 15 ml of Raney nickel were added to the filtrate, which was then stirred for 1.5 hours at 5 to 10 ° C in an atmosphere of hydrogen gas under normal atmospheric pressure. It was then stirred for 4.5 hours at room temperature. At the end of this time, the catalyst was filtered off. 180 ml of triethylamine were added to the filtrate, and then 70 ml of chloroacetyl chloride were added on an ice bath at 10 to 15 ° C. The reaction mixture was stirred for 1 hour at room temperature, after which it was condensed by evaporation under reduced pressure. The resulting residue was dissolved in ethyl acetate and water. The ethyl acetate layer was separated, washed with water and condensed again by evaporation under reduced pressure. The residue was dissolved in 300 ml of acetic acid and 100 ml of water. The resulting solution was stirred for 2 hours at 100 ° C. At the end of this time, the reaction solution was condensed by evaporation under reduced pressure. The residue was dissolved in ethyl acetate. The resulting solution was washed with a saturated aqueous solution of sodium bicarbonate and dried over anhydrous magnesium sulphate. The solvent was then removed by distillation under reduced pressure. The residue was purified by silica gel column chromatography using a 9 : 1 by volume mixture of methylene chloride and methanol as the eluent, to obtain 33.1 g of the title compound as a syrup.
Optical rotation $[\alpha]_D^{25}$ - 4.9 ° (c = 1, dimethylformamide).
Nuclear Magnetic Resonance Spectrum ($CDC\ell_3$), δ ppm:

3.1 - 3.6 (4H, multiplet);
4.06 (2H, singlet);
3.6 - 5.1 (5H, multiplet);
7.05 - 7.40 (5H, multiplet).

5(b) (S)-N-Benzyl-3-chloroacetamido-1-triphenylmethoxy-2-propanol

39.4 g of triphenylmethyl chloride were added at room temperature to 200 ml of methylene chloride containing 33.1 g of (S)-N-benzyl-3-chloroacetamidopropane-1,2-diol [prepared as described in step (a) above] and 25 ml of triethylamine. The mixture was then stirred for 2 hours at room temperature, after which the mixture was condensed by evaporation under reduced pressure. The resulting residue was dissolved in ethyl acetate and water. The organic layer was separated and dried over anhydrous magnesium sulphate, and then the solvent was removed by distillation under reduced pressure. The resulting residue was purified by silica gel column chromatography using a 1 : 1 by volume mixture of ethyl acetate and hexane as the eluent, to obtain 50.0 g of the title compound as a gum.
Optical rotation $[\alpha]_D^{25}$ - 4.4 ° (c = 1, dimethylformamide).

Nuclear Magnetic Resonance Spectrum ($CDC\ell_3$), δ ppm:

3.11 (2H, broad doublet, J = 5 Hz);
3.1 - 5.1 (8H, multiplet);
7.1 - 7.5 (20H, multiplet).

5(c) (S)-4-Benzyl-3-oxo-6-(triphenylmethoxymethyl)morpholine

4.77 g of sodium hydride (as a 55% by weight suspension in mineral oil) were added to a mixture of 200 ml of dimethylformamide and 66 ml of toluene containing 50.0 g of (S)-N-benzyl-3-chloroacetamido-1-triphenylmethoxy-2-propanol [prepared as described in step (b) above]. The reaction mixture was then stirred for 2.5 hours at 100 °C under a stream of nitrogen. At the end of this time, ethyl acetate and water were added to the reaction mixture. The organic layer was then separated, washed with water and dried over anhydrous magnesium sulphate, after which the solvent was removed by distillation under reduced pressure. The resulting residue was purified by silica gel column chromatography using a 1 : 2 by volume mixture of ethyl acetate and hexane as the eluent, to obtain 38.6 g of the title compound as a gum.

Optical rotation $[\alpha]_D^{25}$ - 27.1 ° (c = 1, dimethylformamide).

Nuclear Magnetic Resonance Spectrum ($CDC\ell_3$), δ ppm:

2.9 - 3.45 (4H, multiplet);
3.6 - 3.95 (1H, multiplet);
4.22 (2H, AB quartet, Δδ = 0.27 ppm, J = 16 Hz);
4.53 (2H, AB quartet, Δδ = 0.29 ppm, J = 15 Hz);
7.1 - 7.5 (20H, multiplet).

5(d) (S)-4-Benzyl-2-(triphenylmethoxymethyl)morpholine

3.15 g of lithium aluminium hydride were added to 350 ml of tetrahydrofuran containing 38.6 g of (S)-4-benzyl-3-oxo-6-(triphenylmethoxymethyl)morpholine [prepared as described in step (c) above]. The reaction mixture was then stirred for 3.5 hours on a water bath at 75 °C, after which sufficient crystalline sodium sulphate decahydrate was added to decompose the excess lithium aluminium hydride. The resulting precipitate was then removed by filtration. The filtrate was condensed by evaporation under reduced pressure, and the resulting residue was purified by silica gel column chromatography using a 1 : 2 by volume mixture of ethyl acetate and hexane as the eluent, to obtain 33.6 g of the title compound as a syrup.

Optical rotation $[\alpha]_D^{25}$ + 6.3 ° (c = 1, dimethylformamide).

Nuclear Magnetic Resonance Spectrum ($CDC\ell_3$), δ ppm:

1.8 - 3.9 (9H, multiplet);
3.48 (2H, singlet);
7.1 - 7.5 (20H, multiplet).

5(e) (S)-4-Benzyl-2-(hydroxymethyl)morpholine

A mixture of 140 ml of acetic acid and 70 ml of water containing 33.6 g of (S)-4-benzyl-2-(triphenylmethoxymethyl)morpholine [prepared as described in step (d) above] was stirred at 100 °C for 2 hours. At the end of this time, the reaction solution was cooled. The triphenylmethanol which separated was filtered off, and then the solvent was removed by distillation under reduced pressure. The remaining acetic acid was then removed by azeotropic distillation with toluene. The resulting residue was purified by silica gel column chromatography using a 1 : 9 by volume mixture of methanol and methylene chloride as the eluent, to obtain 14.8 g of the title compound as an oil.

Optical rotation $[\alpha]_D^{25}$ + 9.9 ° (c = 1, dimethylformamide).

Nuclear Magnetic Resonance Spectrum ($CDC\ell_3$), δ ppm:

1.8 - 2.8 (5H, multiplet);
3.48 (2H, singlet);
3.45 - 4.0 (5H, multiplet);
7.31 (5H, singlet).

5(f) (S)-4-Butoxycarbonyl-2-(hydroxymethyl)morpholine

2.3 g of 10% w/w palladium-on-carbon were added to 120 ml of methanol containing 14.8 g of (S)-4-benzyl-2-hydroxymethylmorpholine [prepared as described in step (e) above]. The mixture was then stirred

at 60°C for 7 hours in an atmosphere of hydrogen gas under normal atmospheric pressure. At the end of this time, the catalyst was filtered off. 12 ml of triethylamine were added to the filtrate, after which 20 ml of a methylene chloride solution containing 15.6 g of di-t-butyl pyrocarbonate were added dropwise to the mixture on an ice bath. The mixture was then stirred for 1 hour at room temperature, after which it was condensed by evaporation under reduced pressure. The resulting residue was purified by silica gel column chromatography using a 1 : 1 by volume mixture of ethyl acetate and hexane as the eluent, to obtain 12.2 g of the title compound as a syrup.
Optical rotation $[\alpha]_D^{25}$ + 8.3° (c = 1, dimethylformamide).

5(g) (S)-4-t-Butoxycarbonyl-2-[(4-nitrophenylsulphonyloxy)methyl]morpholine

A procedure similar to that described in Preparation 1 was repeated, except that 12.2 g of (S)-4-t-butoxycarbonyl-2-(hydroxymethyl)morpholine [prepared as described in step (f) above] and 13.0 g of p-nitrobenzenesulphonyl chloride were employed, to give 19.7 g of the title compound as crystals, melting at 107°C.
Optical rotation $[\alpha]_D^{25}$ + 23.1° (c = 1, dimethylformamide).

The Nuclear Magnetic Resonance Spectrum of this compound was the same as that of the product of Preparation 2.

FORMULATION 1

| Capsules | |
|---|---|
| 7-[3-(Methylamino)propoxy]-3,4,8-trimethylcoumarin hydrochloride (Compound of Example 8) | 20.0 mg |
| Lactose | 158.7 mg |
| Corn starch | 70.0 mg |
| Magnesium stearate | 1.3 mg |
| | 250 mg |

Powders of the components shown above were well mixed and filtered through a 60-mesh sieve. (The mesh standard used herein is the Tyler standard). 250 mg of the resulting powder were weighed out and charged into a gelatin capsule No. 3 to prepare capsules.

FORMULATION 2

| Tablets | |
|---|---|
| 7-[3-(Methylamino)propoxy]-3,4,8-trimethylcoumarin hydrochloride (Compound of Example 8) | 20.0 mg |
| Lactose | 154.0 mg |
| Corn starch | 25.0 mg |
| Magnesium stearate | 1.0 mg |
| | 200 mg |

Powders of the components shown above were well mixed, and pressed into tablets each weighing 200 mg.

If necessary, these tablets may be coated with sugar.

FORMULATION 3

| Capsules | |
|---|---|
| 7-[(RS)-3-Amino-2-methylbutoxy]-3,4,8-trimethylcoumarin hydrochloride (Compound of Example 36) | 20.0 mg |
| Lactose | 158.7 mg |
| Corn starch | 70.0 mg |
| Magnesium stearate | 1.3 mg |
| | 250 mg |

Powders of the components shown above were well mixed and filtered through a 60-mesh sieve. 250 mg of the resulting powder were weighed out and charged into a gelatin capsule No. 3 to prepare capsules.

FORMULATION 4

| Tablets | |
|---|---|
| 7-[(RS)-3-Amino-2-methylbutoxy]-3,4,8-trimethylcoumarin hydrochloride (Compound of Example 36) | 20.0 mg |
| Lactose | 154.0 mg |
| Corn starch | 25.0 mg |
| Magnesium stearate | 1.0 mg |
| | 200 mg |

Powders of the components shown above were well mixed, and pressed into tablets each weighing 200 mg.

If necessary, these tablets may be coated with sugar.

**Claims**

**1.** The use of at least one compound of formula (I):

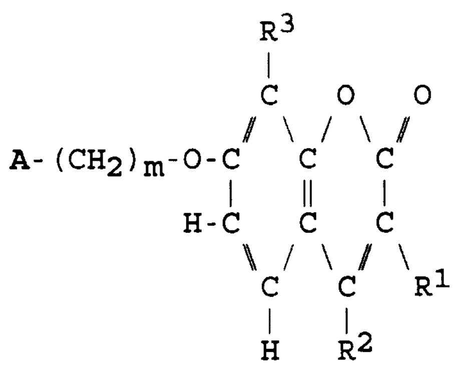

(I)

in which:

A represents a group of formula (II) or (III):

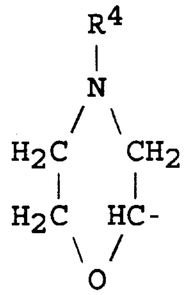

(II)

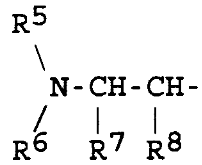

(III);

$R^1$, $R^2$ and $R^3$ are the same or different and each represents a hydrogen atom, an alkyl group having from 1 to 4 carbon atoms or a halogen atom;

$R^4$ represents a hydrogen atom or an alkyl group having from 1 to 4 carbon atoms, said alkyl group being unsubstituted or having at least one substituent selected from aryl groups having from 6 to 10 carbon atoms, where said aryl group is unsubstituted or is substituted by at least one of substituents (a), defined below;

$R^5$ and $R^6$ are the same or different and each represents a hydrogen atom or an alkyl group having from 1 to 4 carbon atoms;

$R^7$ and $R^8$ are the same or different and each represents a hydrogen atom or an alkyl group having from 1 to 4 carbon atoms; or $R^7$ and $R^8$ together represent an alkylene group having 3 or 4 carbon atoms; and

m is 1 or 2;

substituents (a):

alkyl groups having from 1 to 4 carbon atoms, alkoxy groups having from 1 to 4 carbon atoms, trifluoromethyl groups, nitro groups, cyano groups and halogen atoms;

or a pharmaceutically acceptable salt thereof

for the manufacture of a medicament for the treatment of a cerebrovascular disorder.

2. A use according to Claim 1, in which said compound has the formula (Ia):

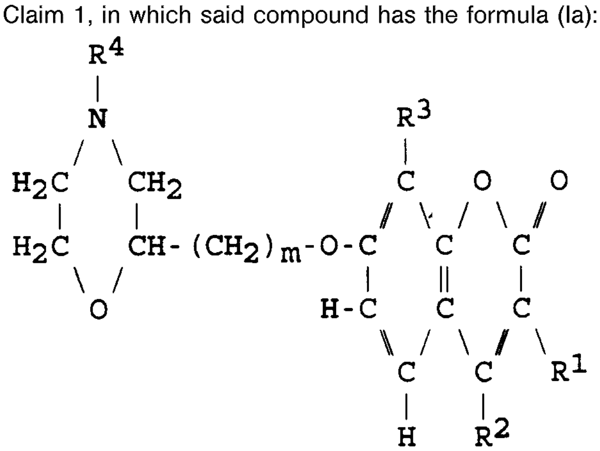

(Ia)

in which $R^1$, $R^2$, $R^3$, $R^4$ and m are as defined in Claim 1.

3. A use according to Claim 2, in which m is 1.

4. A use according to Claim 1, in which said compound has the formula (Ib):

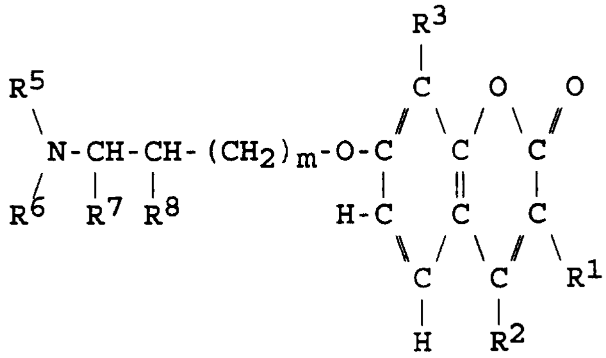

(Ib)

in which $R^1$, $R^2$, $R^3$, $R^5$, $R^6$, $R^7$, $R^8$ and m are as defined in Claim 1.

5. A use according to Claim 4, in which $R^7$ and $R^8$ both represent hydrogen atoms.

6. A use according to Claim 2, in which $R^1$ represents a hydrogen atom, a methyl group, an ethyl group, a propyl group, an isopropyl group, a fluorine atom or a chlorine atom.

7. A use according to Claim 2 or Claim 6, in which $R^2$ represents a hydrogen atom, a methyl group, an ethyl group, a propyl group or an isopropyl group.

8. A use according to any one of Claims 2, 6 and 7, in which $R^3$ represents a hydrogen atom, a methyl group, an ethyl group or an isopropyl group.

9. A use according to any one of Claims 2 and 6 to 8, in which $R^4$ represents a hydrogen atom.

10. A use according to Claim 2, in which:
$R^1$ represents a hydrogen atom, a methyl group, a fluorine atom or a chlorine atom;
$R^2$ and $R^3$ are the same or different and each represents a hydrogen atom or a methyl group; and
$R^4$ represents a hydrogen atom.

11. A use according to Claim 1, in which m is 1.

12. A use according to Claim 4, in which both $R^5$ and $R^6$ represent hydrogen atoms.

13. A use according to Claim 4, in which $R^5$ represents a hydrogen atom and $R^6$ represents an alkyl group containing from 1 to 4 carbon atoms.

14. A use according to Claim 4, in which $R^5$ represents a hydrogen atom and $R^6$ represents a methyl group.

15. A use according to Claim 4, in which $R^5$ and $R^6$ are the same or different and each represents a methyl or ethyl group.

16. A use according to any one of Claims 4 and 12 to 15, in which $R^1$ represents a hydrogen atom, a chlorine atom or a methyl group.

17. A use according to any one of Claims 4 and 12 to 16, in which $R^2$ represents a hydrogen atom or a methyl group.

18. A use according to any one of Claims 4 and 12 to 17, in which $R^3$ represents a hydrogen atom or a methyl group.

**19.** A use according to Claim 4, in which:
$R^1$ represents a hydrogen atom, a chlorine atom or a methyl group;
$R^2$ represents a hydrogen atom or a methyl group;
$R^3$ represents a hydrogen atom or a methyl group; and
both $R^5$ and $R^6$ represent hydrogen atoms.

**20.** A use according to Claim 4, in which:
$R^1$ represents a hydrogen atom, a chlorine atom or a methyl group;
$R^2$ represents a hydrogen atom or a methyl group;
$R^3$ represents a hydrogen atom or a methyl group;
$R^5$ represents a hydrogen atom; and
$R^6$ represents an alkyl group containing from 1 to 4 carbon atoms.

**21.** A use according to Claim 4, in which:
$R^1$ represents a hydrogen atom, a chlorine atom or a methyl group;
$R^2$ represents a hydrogen atom or a methyl group;
$R^3$ represents a hydrogen atom or a methyl group;
$R^5$ represents a hydrogen atom; and
$R^6$ represents a methyl group.

**22.** A use according to Claim 4, in which:
$R^1$ represents a hydrogen atom, a chlorine atom or a methyl group;
$R^2$ represents a hydrogen atom or a methyl group;
$R^3$ represents a hydrogen atom or a methyl group; and
$R^5$ and $R^6$ are the same or different and each represents a methyl or ethyl group.

**23.** A use according to Claim 4, in which:
$R^1$, $R^2$ and $R^3$ all represent methyl groups;
$R^5$ and $R^6$ both represent hydrogen atoms; and
$R^7$ and $R^8$ are the same or different and each represents a hydrogen atom, a methyl group, an ethyl group or an isopropyl group.

**24.** A use according to Claim 4, in which:
$R^1$, $R^2$ and $R^3$ all represent methyl groups;
$R^5$ represents a hydrogen atom;
$R^6$ represents a methyl group; and
$R^7$ and $R^8$ are the same or different and each represents a hydrogen atom, a methyl group, an ethyl group or an isopropyl group.

**25.** A use according to Claim 4, in which:
$R^1$, $R^2$ and $R^3$ all represent methyl groups;
$R^5$ and $R^6$ both represent methyl groups; and
$R^7$ and $R^8$ are the same or different and each represents a hydrogen atom, a methyl group, an ethyl group or an isopropyl group.

**26.** A use according to Claim 1, in which said compound is 3,4,8-trimethyl-7-(2-morpholinyl)-methoxycoumarin or a pharmaceutically acceptable salt thereof.

**27.** A use according to Claim 1, in which said compound is 3,4-dimethyl-7-(2-morpholinyl)methoxycoumarin or a pharmaceutically acceptable salt thereof.

**28.** A use according to Claim 1, in which said compound is 3,4,8-trimethyl-7-(4-methyl-2-morpholinyl)-methoxycoumarin or a pharmaceutically acceptable salt thereof.

**29.** A use according to Claim 1, in which said compound is 7-(3-N,N-dimethylaminopropoxy)-3,4,8-trimethylcoumarin or a pharmaceutically acceptable salt thereof.

**30.** A use according to Claim 1, in which said compound is 7-(3-aminopropoxy)-3,4,8-trimethylcoumarin or a pharmaceutically acceptable salt thereof.

**31.** A use according to Claim 1, in which said compound is 7-(3-aminobutoxy)-3,4,8-trimethylcoumarin or a pharmaceutically acceptable salt thereof.

**32.** A compound of formula (I):

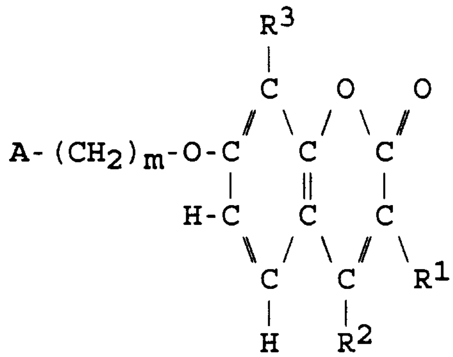

(I)

in which:
A represents a group of formula (II) or (III):

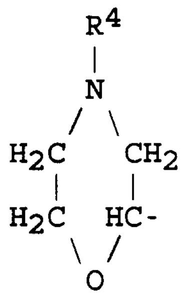

(II)

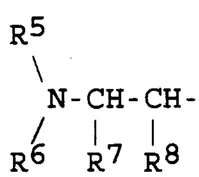

(III);

$R^1$, $R^2$ and $R^3$ are the same or different and each represents a hydrogen atom, an alkyl group having from 1 to 4 carbon atoms or a halogen atom;
$R^4$ represents a hydrogen atom or an alkyl group having from 1 to 4 carbon atoms, said alkyl group being unsubstituted or having at least one substituent selected from aryl groups having from 6 to 10 carbon atoms, where said aryl group is unsubstituted or is substituted by at least one of substituents (a), defined below;
$R^5$ and $R^6$ are the same or different and each represents a hydrogen atom or an alkyl group having from 1 to 4 carbon atoms;
$R^7$ and $R^8$ are the same or different and each represents a hydrogen atom or an alkyl group having from 1 to 4 carbon atoms; or $R^7$ and $R^8$ together represent an alkylene group having 3 or 4 carbon atoms; and
m is 1 or 2;
substituents (a):
alkyl groups having from 1 to 4 carbon atoms, alkoxy groups having from 1 to 4 carbon atoms,

trifluoromethyl groups, nitro groups, cyano groups and halogen atoms;
and pharmaceutically acceptable salts thereof,
PROVIDED THAT $R^5$ and $R^6$ are not identical and either a methyl or an ethyl group when: A represents a group of formula (III), $R^1$, $R^3$, $R^7$ and $R^8$ all represent hydrogen atoms, $R^2$ represents a methyl group and m is 1.

**33.** A compound according to Claim 32, which has the formula (Ia):

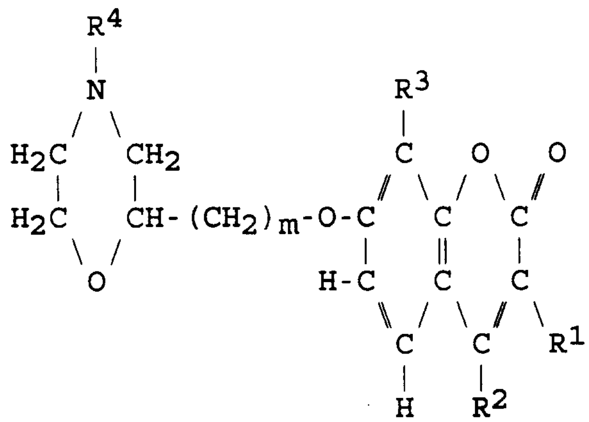

(Ia)

in which $R^1$, $R^2$, $R^3$, $R^4$ and m are as defined in Claim 32.

**34.** A compound according to Claim 33, in which m is 1.

**35.** A compound according to Claim 32, which has the formula (Ib):

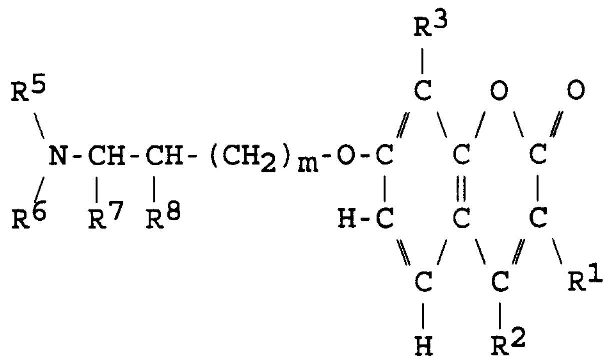

(Ib)

in which $R^1$, $R^2$, $R^3$, $R^5$, $R^6$, $R^7$, $R^8$ and m are as defined in Claim 32.

**36.** A compound according to Claim 35, in which $R^7$ and $R^8$ both represent hydrogen atoms.

**37.** A compound according to Claim 33, in which $R^1$ represents a hydrogen atom, a methyl group, an ethyl group, a propyl group, an isopropyl group, a fluorine atom or a chlorine atom.

**38.** A compound according to Claim 33 or Claim 37, in which $R^2$ represents a hydrogen atom, a methyl group, an ethyl group, a propyl group or an isopropyl group.

**39.** A compound according to any one of Claims 33, 37 and 38, in which $R^3$ represents a hydrogen atom, a methyl group, an ethyl group or an isopropyl group.

**40.** A compound according to any one of Claims 33, and 37 to 39, in which $R^4$ represents a hydrogen atom.

**41.** A compound according to Claim 33, in which:
$R^1$ represents a hydrogen atom, a methyl group, a fluorine atom or a chlorine atom;
$R^2$ and $R^3$ are the same or different and each represents a hydrogen atom or a methyl group; and
$R^4$ represents a hydrogen atom.

**42.** A compound according to Claim 32, in which $\underline{m}$ is 1.

**43.** A compound according to Claim 35, in which both $R^5$ and $R^6$ represent hydrogen atoms.

**44.** A compound according to Claim 35 or Claim 43, in which $R^5$ represents a hydrogen atom and $R^6$ represents an alkyl group containing from 1 to 4 carbon atoms.

**45.** A compound according to any one of Claims 35, 43 and 44, in which $R^5$ represents a hydrogen atom and $R^6$ represents a methyl group.

**46.** A compound according to any one of Claims 35 and 43 to 45, in which $R^5$ and $R^6$ are the same or different and each represents a methyl or ethyl group.

**47.** A compound according to any one of Claims 35 and 43 to 46, in which $R^3$ represents a hydrogen atom or a methyl group.

**48.** A compound according to any one of Claims 35 and 43 to 47, in which $R^2$ represents a hydrogen atom or a methyl group.

**49.** A compound according to any one of Claims 35 and 43 to 48, in which $R^1$ represents a hydrogen atom, a chlorine atom or a methyl group.

**50.** A compound according to Claim 35, in which:
$R^1$ represents a hydrogen atom, a chlorine atom or a methyl group;
$R^2$ represents a hydrogen atom or a methyl group;
$R^3$ represents a hydrogen atom or a methyl group; and
both $R^5$ and $R^6$ represent hydrogen atoms.

**51.** A compound according to Claim 35, in which:
$R^1$ represents a hydrogen atom, a chlorine atom or a methyl group;
$R^2$ represents a hydrogen atom or a methyl group;
$R^3$ represents a hydrogen atom or a methyl group;
$R^5$ represents a hydrogen atom; and
$R^6$ represents an alkyl group containing from 1 to 4 carbon atoms.

**52.** A compound according to Claim 35, in which:
$R^1$ represents a hydrogen atom, a chlorine atom or a methyl group;
$R^2$ represents a hydrogen atom or a methyl group;
$R^3$ represents a hydrogen atom or a methyl group;
$R^5$ represents a hydrogen atom; and
$R^6$ represents a methyl group.

**53.** A compound according to Claim 35, in which:
$R^1$ represents a hydrogen atom, a chlorine atom or a methyl group;
$R^2$ represents a hydrogen atom or a methyl group;
$R^3$ represents a hydrogen atom or a methyl group; and
$R^5$ and $R^6$ are the same or different and each represents a methyl or ethyl group.

**54.** A compound according to Claim 35, in which:
$R^1$, $R^2$ and $R^3$ all represent methyl groups;
$R^5$ and $R^6$ both represent hydrogen atoms; and
$R^7$ and $R^8$ are the same or different and each represents a hydrogen atom, a methyl group, an ethyl group or an isopropyl group.

**55.** A compound according to Claim 35, in which:
$R^1$, $R^2$ and $R^3$ all represent methyl groups;
$R^5$ represents a hydrogen atom;
$R^6$ represents a methyl group; and
$R^7$ and $R^8$ are the same or different and each represents a hydrogen atom, a methyl group, an ethyl group or an isopropyl group.

**56.** A compound according to Claim 35, in which:
$R^1$, $R^2$ and $R^3$ all represent methyl groups;
$R^5$ and $R^6$ both represent methyl groups; and
$R^7$ and $R^8$ are the same or different and each represents a hydrogen atom, a methyl group, an ethyl group or an isopropyl group.

**57.** The compounds of Claim 32, selected from:
3,4,8-trimethyl-7-(2-morpholinyl)methoxycoumarin;
3,4-dimethyl-7-(2-morpholinyl)methoxycoumarin;
3,4,8-trimethyl-7-(4-methyl-2-morpholinyl)methoxycoumarin;
7-(3-N,N-dimethylaminopropoxy)-3,4,8-trimethylcoumarin;
7-(3-aminopropoxy)-3,4,8-trimethylcoumarin; and
7-(3-aminobutoxy)-3,4,8-trimethylcoumarin;
and pharmaceutically acceptable salts thereof.

**58.** A pharmaceutical composition for the treatment of cerebrovascular disorders comprising a therapeutically effective amount of at least one compound of formula (I) or a pharmaceutically acceptable salt thereof as defined in any one of Claims 32 to 57 in admixture with a pharmaceutically acceptable carrier, diluent or excipient.

**59.** A process for preparing a compound of formula (I) according to any of claims 32 to 57, which process comprises the steps:
(a) reacting a compound of formula (IV):

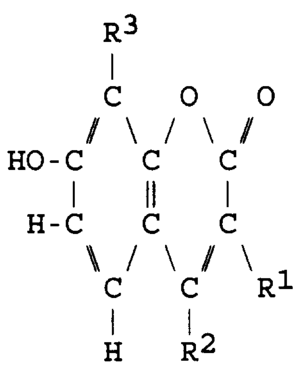

(IV)

(in which: $R^1$, $R^2$ and $R^3$ are as defined in Claim 1) with a compound of formula (V):

$Y-(CH_2)_m-A'$ (V)

[in which: m is as defined in Claim 1; Y represents a halogen atom, a sulphonyloxy group or a hydroxy group; and A' represents a group of formula (II') or (III'):

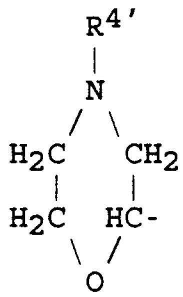

(II')

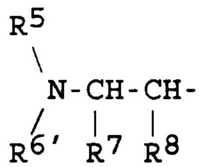

(III')

(in which: $R^5$, $R^7$ and $R^8$ are as defined in Claim 1; $R^{4'}$ represents an amino-protecting group or an alkyl group having from 1 to 4 carbon atoms, said alkyl group being unsubstituted or having at least one substituent selected from aryl groups having from 6 to 10 carbon atoms, where said aryl group is unsubstituted or is substituted by at least one of substituents (a), defined in Claim 1; and $R^{6'}$ represents an amino-protecting group or an alkyl group having from 1 to 4 carbon atoms)]; and
if required, one of the steps (b) or (c):
(b) where A' represents said group of formula (II') and $R^{4'}$ represents an amino-protecting group or A' represents said group of formula (III') and $R^{6'}$ represents an amino-protecting group, removing the amino-protecting group;
(c) where the product is a compound in which one or both of $R^5$ and $R^6$ represents a hydrogen atom, alkylating the hydrogen atom to convert it to an alkyl group having from 1 to 4 carbon atoms;
and optionally salifying the product.

**60.** A process for preparing a pharmaceutical composition for the treatment of cerebrovascular disorders by mixing a compound of formula (I), as defined in any of Claims 1 to 31, or a pharmaceutically acceptable salt thereof with a pharmaceutically acceptable carrier or diluent.

**Patentansprüche**

**1.** Verwendung mindestens einer Verbindung der Formel (I):

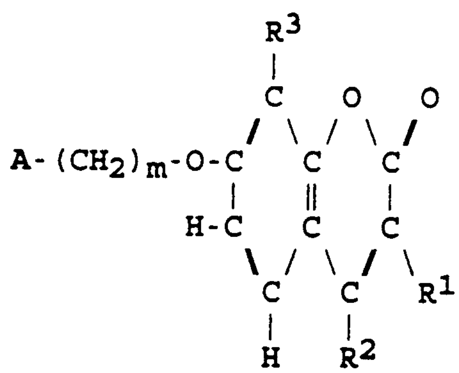

(I)

,

in der:
A eine Gruppe der Formel (II) oder (III) darstellt:

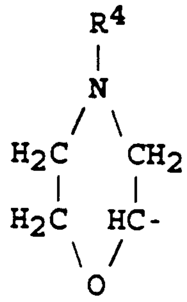

(II)

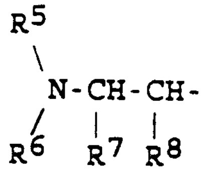

(III);

$R^1$, $R^2$ und $R^3$ gleich oder verschieden sind, und jeweils ein Wasserstoffatom, eine Alkylgruppe mit 1 bis 4 Kohlenstoffatomen oder ein Halogenatom darstellen,
$R^4$ ein Wasserstoffatom oder eine Alkylgruppe mit 1 bis 4 Kohlenstoffatomen darstellt, wobei die Alkylgruppe unsubstituiert ist oder mindestens einen Substituenten aufweist, der ausgewählt ist unter Arylgruppen mit 6 bis 10 Kohlenstoffatomen, wobei die Arylgruppe unsubstituiert ist oder durch mindestens einen der untenstehend definierten Substituenten (a) substituiert ist,
$R^5$ und $R^6$ gleich oder verschieden sind und jeweils ein Wasserstoffatom oder eine Alkylgruppe mit 1 bis 4 Kohlenstoffatomen darstellen,
$R^7$ und $R^8$ gleich oder verschieden sind und jeweils ein Wasserstoffatom oder eine Alkylgruppe mit 1 bis 4 Kohlenstoffatomen darstelle oder $R^7$ und $R^8$ zusammen eine Alkylengruppe mit 3 oder 4 Kohlenstoffatomen darstellen, und
m 1 oder 2 ist,
Substituenten (a):
Alkylgruppen mit 1 bis 4 Kohlenstoffatomen, Alkoxygruppen mit 1 bis 4 Kohlenstoffatomen, Trifluormethylgruppen, Nitrogruppen, Cyangruppen und Halogenatome,
oder eines pharmazeutisch geeigneten Salzes derselben
zur Herstellung eines Arzneimittels zur Behandlung von cerebrovaskulären Störungen.

**2.** Verwendung gemäß Anspruch 1, wobei die Verbindung die Formel (Ia) besitzt:

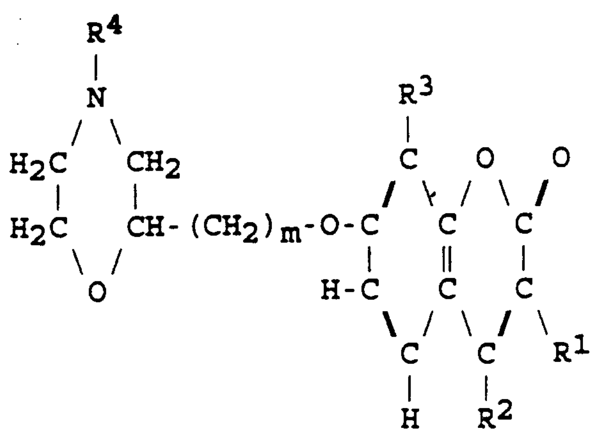

(Ia)

,

in der $R^1$, $R^2$, $R^3$, $R^4$ und m wie in Anspruch 1 definiert sind.

**3.** Verwendung gemäß Anspruch 2, wobei m 1 ist.

4. Verwendung gemäß Anspruch 1, wobei die Verbindung die Formel (Ib) besitzt:

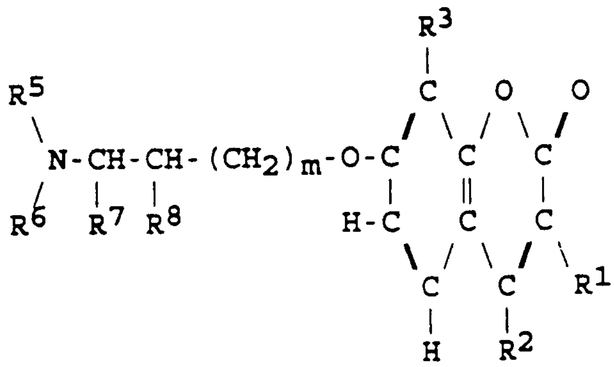

(Ib)

,

in der $R^1$, $R^2$, $R^3$, $R^5$, $R^6$, $R^7$, $R^8$ und m wie in Anspruch 1 definiert sind.

5. Verwendung gemäß Anspruch 4, wobei $R^7$ und $R^8$ jeweils Wasserstoffatome darstellen.

6. Verwendung gemäß Anspruch 2, wobei $R^1$ ein Wasserstoffatom, eine Methylgruppe, eine Ethylgruppe, eine Propylgruppe, eine Isopropylgruppe, ein Fluoratom oder ein Chloratom darstellt.

7. Verwendung gemäß Anspruch 2 oder Anspruch 6, wobei $R^2$ ein Wasserstoffatom, eine Methylgruppe, eine Ethylgruppe, eine Propylgruppe oder eine Isopropylgruppe darstellt.

8. Verwendung gemäß einem der Ansprüche 2, 6 und 7, wobei $R^3$ ein Wasserstoffatom, eine Methylgruppe, eine Ethylgruppe oder eine Isopropylgruppe darstellt.

9. Verwendung gemäß einem der Ansprüche 2 und 6 bis 8, wobei $R^4$ ein Wasserstoffatom darstellt.

10. Verwendung gemäß Anspruch 2, wobei:
$R^1$ ein Wasserstoffatom, eine Methylgruppe, ein Fluoratom oder ein Chloratom darstellt,
$R^2$ und $R^3$ gleich oder verschieden sind und jeweils ein Wasserstoffatom oder eine Methylgruppe darstellen, und
$R^4$ ein Wasserstoffatom darstellt.

11. Verwendung gemäß Anspruch 1, wobei m 1 ist.

12. Verwendung gemäß Anspruch 4, wobei $R^5$ und $R^6$ jeweils Wasserstoffatome darstellen.

13. Verwendung gemäß Anspruch 4, wobei $R^5$ ein Wasserstoffatom darstellt und $R^6$ eine Alkylgruppe mit 1 bis 4 Kohlenstoffatomen darstellt.

14. Verwendung gemäß Anspruch 4, wobei $R^5$ ein Wasserstoffatom darstellt und $R^6$ eine Methylgruppe darstellt.

15. Verwendung gemäß Anspruch 4, wobei $R^5$ und $R^6$ gleich oder verschieden sind und jeweils eine Methyl- oder Ethylgruppe darstellen.

16. Verwendung gemäß einem der Ansprüche 4 und 12 bis 15, wobei $R^1$ ein Wasserstoffatom, ein Chloratom oder eine Methylgruppe darstellt.

17. Verwendung gemäß einem der Ansprüche 4 und 12 bis 16, wobei $R^2$ ein Wasserstoffatom oder eine Methylgruppe darstellt.

18. Verwendung gemäß einem der Ansprüche 4 und 12 bis 17, wobei $R^3$ ein Wasserstoffatom oder eine Methylgruppe darstellt.

19. Verwendung gemäß Anspruch 4, wobei:
$R^1$ ein Wasserstoffatom, ein Chloratom oder eine Methylgruppe darstellt,
$R^2$ ein Wasserstoffatom oder eine Methylgruppe darstellt,
$R^3$ ein Wasserstoffatom oder eine Methylgruppe darstellt, und
$R^5$ und $R^6$ jeweils Wasserstoffatome darstellen.

20. Verwendung gemäß Anspruch 4, wobei:
$R^1$ ein Wasserstoffatom, ein Chloratom oder eine Methylgruppe darstellt,
$R^2$ ein Wasserstoffatom oder eine Methylgruppe darstellt,
$R^3$ ein Wasserstoffatom oder eine Methylgruppe darstellt,
$R^5$ ein Wasserstoffatom darstellt, und
$R^6$ eine Alkylgruppe mit 1 bis 4 Kohlenstoffatomen darstellt.

21. Verwendung gemäß Anspruch 4, wobei:
$R^1$ ein Wasserstoffatom, ein Chloratom oder eine Methylgruppe darstellt,
$R^2$ ein Wasserstoffatom oder eine Methylgruppe darstellt,
$R^3$ ein Wasserstoffatom oder eine Methylgruppe darstellt,
$R^5$ ein Wasserstoffatom darstellt und
$R^6$ eine Methylgruppe darstellt.

22. Verwendung gemäß Anspruch 4, wobei:
$R^1$ ein Wasserstoffatom, ein Chloratom oder eine Methylgruppe darstellt,
$R^2$ ein Wasserstoffatom oder eine Methylgruppe darstellt,
$R^3$ ein Wasserstoffatom oder eine Methylgruppe darstellt,
$R^5$ und $R^6$ gleich oder verschieden sind und jeweils eine Methyl- oder Ethylgruppe darstellen.

23. Verwendung gemäß Anspruch 4, wobei:
$R^1$, $R^2$ und $R^3$ jeweils Methylgruppen darstellen,
$R^5$ und $R^6$ jeweils Wasserstoffatome darstellen, und
$R^7$ und $R^8$ gleich oder verschieden sind und jeweils ein Wasserstoffatom, eine Methylgruppe, eine Ethylgruppe oder eine Isopropylgruppe darstellen.

24. Verwendung gemäß Anspruch 4, wobei:
$R^1$, $R^2$ und $R^3$ jeweils Methylgruppen darstellen,
$R^5$ ein Wasserstoffatom darstellt,
$R^6$ eine Methylgruppe darstellt, und
$R^7$ und $R^8$ gleich oder verschieden sind und jeweils ein Wasserstoffatom, eine Methylgruppe, eine Ethylgruppe oder eine Isopropylgruppe darstellen.

25. Verwendung gemäß Anspruch 4, wobei:
$R^1$, $R^2$ und $R^3$ jeweils Methylgruppen darstellen,
$R^5$ und $R^6$ jeweils Methylgruppen darstellen, und
$R^7$ und $R^8$ gleich oder verschieden sind und jeweils ein Wasserstoffatom, eine Methylgruppe, eine Ethylgruppe oder eine Isopropylgruppe darstellen.

26. Verwendung gemäß Anspruch 1, wobei die Verbindung 3,4,8-Trimethyl-7-(2-morpholinyl)-methoxycumarin oder ein pharmazeutisch geeignetes Salz derselben ist.

27. Verwendung gemäß Anspruch 1, wobei die Verbindung 3,4-Dimethyl-7-(2-morpholinyl)methoxycumarin oder ein pharmazeutisch geeignetes Salz derselben ist.

28. Verwendung gemäß Anspruch 1, wobei die Verbindung 3,4,8-Trimethyl-7-(4-methyl-2-morpholinyl)-methoxycumarin oder ein pharmazeutisch geeignetes Salz derselben ist.

29. Verwendung gemäß Anspruch 1, wobei die Verbindung 7-(3-N,N-dimethylaminopropoxy)-3,4,8-trimethylcumarin oder ein pharmazeutisch geeignetes Salz derselben ist.

**30.** Verwendung gemäß Anspruch 1, wobei die Verbindung 7-(3-Aminopropoxy)-3,4,8-trimethylcumarin oder ein pharmazeutisch geeignetes Salz derselben ist.

**31.** Verwendung gemäß Anspruch 1, wobei die Verbindung 7-(3-Aminobutoxy)-3,4,8-trimethylcumarin oder ein pharmazeutisch geeignetes Salz derselben ist.

**32.** Verbindung der Formel (I):

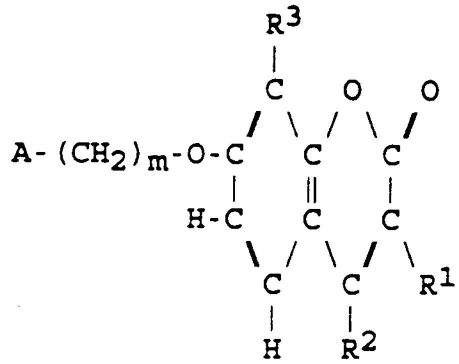

(I)

,

worin:
A eine Gruppe der Formel (II) oder (III) darstellt:

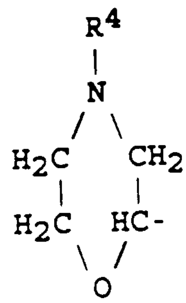

(II)

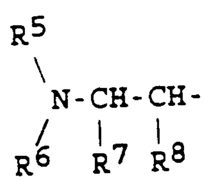

(III);

$R^1$, $R^2$ und $R^3$ gleich oder verschieden sind und jeweils ein Wasserstoffatom, eine Alkylgruppe mit 1 bis 4 Kohlenstoffatomen oder ein Halogenatom darstellen,
$R^4$ ein Wasserstoffatom oder eine Alkylgruppe mit 1 bis 4 Kohlenstoffatomen darstellt, wobei die Alkylgruppe unsubstituiert ist, oder mindestens einen Substituenten aufweist, der ausgewählt ist unter Arylgruppen mit 6 bis 10 Kohlenstoffatomen, wobei die Arylgruppe unsubstituiert ist oder durch mindestens einen der untenstehend definierten Substituenten (a) substituiert ist,
$R^5$ und $R^6$ gleich oder verschieden sind und jeweils ein Wasserstoffatom oder eine Alkylgruppe mit 1 bis 4 Kohlenstoffatomen darstellen,
$R^7$ und $R^8$ gleich oder verschieden sind und jeweils ein Wasserstoffatom oder eine Alkylgruppe mit 1 bis 4 Kohlenstoffatomen darstellen, oder $R^7$ und $R^8$ zusammen eine Alkylengruppe mit 3 oder 4 Kohlenstoffatomen darstellen, und
m 1 oder 2 ist,
<u>Subsituenten (a):</u>
Alkylgruppen mit 1 bis 4 Kohlenstoffatomen, Alkoxygruppen mit 1 bis 4 Kohlenstoffatomen, Trifluormethylgruppen, Nitrogruppen, Cyangruppen und Halogenatome,

und pharmazeutisch geeignete Salze derselben,
mit der Maßgabe, daß $R^5$ und $R^6$ nicht gleich sind, und entweder eine Methyl- oder eine Ethylgruppe sind, falls A eine Gruppe der Formel (III) darstellt, $R^1$, $R^3$, $R^7$ und $R^8$ jeweils Wasserstoffatome darstellen, $R^2$ eine Methylgruppe darstellt, und m 1 ist.

**33.** Verbindung gemäß Anspruch 32 mit der Formel (Ia):

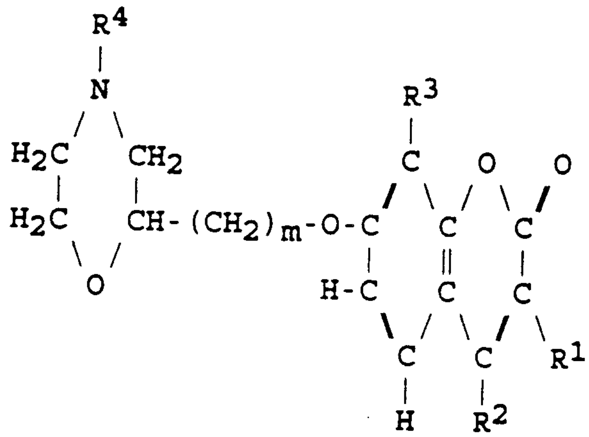

(Ia)

,

worin $R^1$, $R^2$, $R^3$, $R^4$ und m wie in Anspruch 32 definiert sind.

**34.** Verbindung gemäß Anspruch 33, worin m 1 ist.

**35.** Verbindung gemäß Anspruch 32 mit der Formel (Ib):

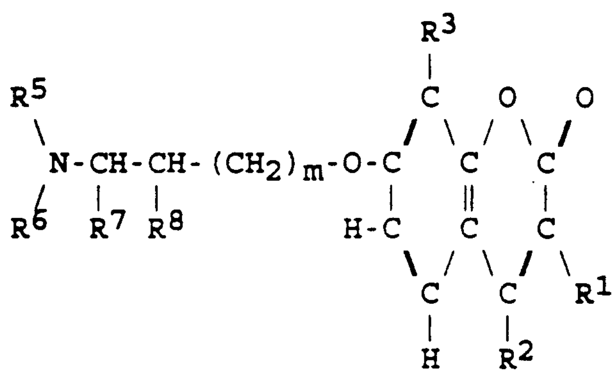

(Ib)

,

worin $R^1$, $R^2$, $R^3$, $R^5$, $R^6$, $R^7$, $R^8$ und m wie in Anspruch 32 definiert sind.

**36.** Verbindung gemäß Anspruch 35, worin $R^7$ und $R^8$ jeweils Wasserstoffatome darstellen.

**37.** Verbindung gemäß Anspruch 33, worin $R^1$ ein Wasserstoffatom, eine Methylgruppe, eine Ethylgruppe, eine Propylgruppe, eine Isopropylgruppe, ein Fluoratom oder ein Chloratom darstellt.

**38.** Verbindung gemäß Anspruch 33 oder 37, worin $R^2$ ein Wasserstoffatom, eine Methylgruppe, eine Ethylgruppe, eine Propylgruppe oder eine Isopropylgruppe darstellt.

**39.** Verbindung gemaß einem der Ansprüche 33, 37 und 38, worin $R^3$ ein Wasserstoffatom, eine Methylgruppe, eine Ethylgruppe oder eine Isopropylgruppe darstellt.

**40.** Verbindung gemäß einem der Ansprüche 33 und 37 bis 39, worin $R^4$ ein Wasserstoffatom darstellt.

**41.** Verbindung gemäß Anspruch 33, worin:
$R^1$ ein Wasserstoffatom, eine Methylgruppe, ein Fluoratom oder ein Chloratom darstellt,

$R^2$ und $R^3$ gleich oder verschieden sind und jeweils ein Wasserstoffatom oder eine Methylgruppe darstellen, und
$R^4$ ein Wasserstoffatom darstellt.

**42.** Verbindung gemäß Anspruch 32, worin m 1 ist.

**43.** Verbindung gemäß Anspruch 35, worin $R^5$ und $R^6$ jeweils Wasserstoffatome darstellen.

**44.** Verbindung gemäß Anspruch 35 oder 43, worin $R^5$ ein Wasserstoffatom und $R^6$ eine Alkylgruppe mit 1 bis 4 Kohlenstoffatomen darstellt.

**45.** Verbindung gemäß einem der Ansprüche 35, 43 und 44, worin $R^5$ ein Wasserstoffatom und $R^6$ eine Methylgruppe darstellt.

**46.** Verbindung gemäß einem der Ansprüche 35 und 43 bis 45, worin $R^5$ und $R^6$ gleich oder verschieden sind und jeweils eine Methyl- oder Ethylgruppe darstellen.

**47.** Verbindung gemäß einem der Ansprüche 35 und 43 bis 46, worin $R^3$ ein Wasserstoffatom oder Methylgruppe darstellt.

**48.** Verbindung gemäß einem der Ansprüche 35 und 43 bis 47, worin $R^2$ ein Wasserstoffatom oder eine Methylgruppe darstellt.

**49.** Verbindung gemäß einem der Ansprüche 35 und 43 bis 48, worin $R^1$ ein Wasserstoffatom, ein Chloratom oder eine Methylgruppe darstellt.

**50.** Verbindung gemäß Anspruch 35, worin:
$R^1$ ein Wasserstoffatom, ein Chloratom oder eine Methylgruppe darstellt,
$R^2$ ein Wasserstoffatom oder eine Methylgruppe darstellt,
$R^3$ ein Wasserstoffatom oder eine Methylgruppe darstellt, und
$R^5$ und $R^6$ jeweils Wasserstoffatome darstellen.

**51.** Verbindung gemäß Anspruch 35, worin:
$R^1$ ein Wasserstoffatom, ein Chloratom oder eine Methylgruppe darstellt,
$R^2$ ein Wasserstoffatom oder eine Methylgruppe darstellt,
$R^3$ ein Wasserstoffatom oder eine Methylgruppe darstellt,
$R^5$ ein Wasserstoffatom darstellt, und
$R^6$ eine Alkylgruppe mit 1 bis 4 Kohlenstoffatomen darstellt.

**52.** Verbindung gemäß Anspruch 35, worin:
$R^1$ ein Wasserstoffatom, ein Chloratom oder eine Methylgruppe darstellt,
$R^2$ ein Wasserstoffatom oder eine Methylgruppe darstellt,
$R^3$ ein Wasserstoffatom oder eine Methylgruppe darstellt,
$R^5$ ein Wasserstoffatom darstellt, und
$R^6$ eine Methylgruppe darstellt.

**53.** Verbindung gemäß Anspruch 35, worin:
$R^1$ ein Wasserstoffatom, ein Chloratom oder eine Methylgruppe darstellt,
$R^2$ ein Wasserstoffatom oder eine Methylgruppe darstellt,
$R^3$ ein Wasserstoffatom oder eine Methylgruppe darstellt, und
$R^5$ und $R^6$ gleich oder verschieden sind und jeweils eine Methyl- oder Ethylgruppe darstellen.

**54.** Verbindung gemäß Anspruch 35, worin:
$R^1$, $R^2$ und $R^3$ jeweils Methylgruppen darstellen,
$R^5$ und $R^6$ jeweils Wasserstoffatome darstellen, und
$R^7$ und $R^8$ gleich oder verschieden sind und jeweils ein Wasserstoffatom, eine Methylgruppe, eine Ethylgruppe oder eine Isopropylgruppe darstellen.

**55.** Verbindung gemäß Anspruch 35, worin:
$R^1$, $R^2$ und $R^3$ jeweils Methylgruppen darstellen,
$R^5$ ein Wasserstoffatome darstellt,
$R^6$ eine Methylgruppe darstellt, und
$R^7$ und $R^8$ gleich oder verschieden sind und jeweils ein Wasserstoffatom, eine Methylgruppe, eine Ethylgruppe oder eine Isopropylgruppe darstellen.

**56.** Verbindung gemäß Anspruch 35, worin:
$R^1$, $R^2$ und $R^3$ jeweils Methylgruppen darstellen,
$R^5$ und $R^6$ jeweils Methylgruppen darstellen, und
$R^7$ und $R^8$ gleich oder verschieden sind und jeweils ein Wasserstoffatom, eine Methylgruppe, eine Ethylgruppe oder eine Isopropylgruppe darstellen.

**57.** Verbindungen gemäß Anspruch 32, die ausgewählt sind unter:
3,4,8,-Trimethyl-7-(2-morpholinyl)methoxycumarin;
3,4-Dimethyl-7-(2-morpholinyl)methoxycumarin;
3,4,8-Trimethyl-7-(4-methyl-2-morpholinyl)methoxycumarin;
7-(3-N,N-Dimethylaminopropoxy)-3,4,8-trimethylcumarin;
7-(3-Aminopropoxy)-3,4,8-trimethylcumarin; und
7-(3-Aminobutoxy)-3,4,8-trimethylcumarin
und pharmazeutisch geeigneten Salzen derselben.

**58.** Arzneimittelzusammensetzung zur Behandlung von cerebrovaskulären Störungen, die eine therapeutisch wirksame Menge mindestens einer Verbindung der Formel (I) oder eines pharmazeutisch geeigneten Salzes derselben, wie in einem der Ansprüche 32 bis 57 definiert, in Beimischung mit einem pharmazeutisch geeigneten Träger, Verdünnungsmittel oder Vehikel enthält.

**59.** Verfahren zur Herstellung einer Verbindung der Formel (I) gemäß einem der Ansprüche 32 bis 57, das die folgenden Schritte umfaßt:
(a) Umsetzung einer Verbindung der Formel (IV):

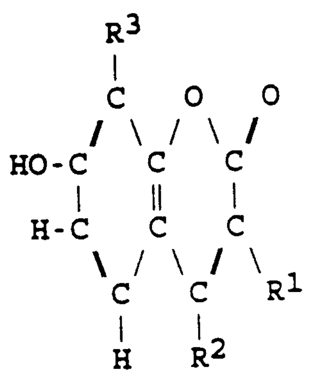

(IV)

,

(worin: $R^1$, $R^2$ und $R^3$ wie in Anspruch 1 definiert sind)
mit einer Verbindung der Formel (V):

$Y-(CH_2)_m-A'$ (V)

[worin: m wie in Anspruch 1 definiert ist, Y ein Halogenatom, eine Sulfonyloxygruppe oder eine Hydroxylgruppe darstellt, und A' eine Gruppe mit der Formel (II') oder (III') darstellt:

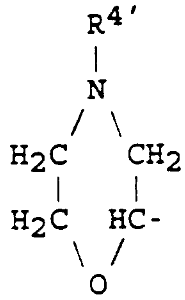

(II')

,

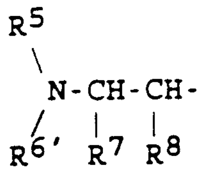

(III')

,

(worin: $R^5$, $R^7$ und $R^8$ wie in Anspruch 1 definiert sind, $R^{4'}$ eine Schutzgruppe für Aminogruppen oder eine Alkylgruppe mit 1 bis 4 Kohlenstoffatomen darstellt, wobei die Alkylgruppe unsubstituiert ist oder mindestens einen Substituenten aufweist, der ausgewählt ist unter Arylgruppen mit 6 bis 10 Kohlenstoffatomen, wobei die Arylgruppe unsubstituiert ist oder durch mindestens einen der in Anspruch 1 definierten Substituenten (a) substituiert ist, und $R^{6'}$ eine Schutzgruppe für Aminogruppen oder eine Alkylgruppe mit 1 bis 4 Kohlenstoffatomen darstellt)], und,
falls erforderlich, einen der Schritte (b) oder (c):
(b) wenn A' die Gruppe der Formel (II') darstellt und $R^{4'}$ eine Schutzgruppe für Aminogruppen darstellt oder A' die Gruppe der Formel (III') darstellt und $R^{6'}$ eine Schutzgruppe für Aminogruppen darstellt, Entfernen der Schutzgruppe für Aminogruppen,
(c) wenn das Produkt eine Verbindung ist, in der eines oder beide der $R^5$ und $R^6$ ein Wasserstoffatom ist, Alkylieren des Wasserstoffatoms, wobei es in eine Alkylgruppe mit 1 bis 4 Kohlenstoffatomen umgewandelt wird,
und gegebenenfalls Bilden eines Salzes des Produkts.

**60.** Verfahren zur Herstellung einer Arzneimittelzusammensetzung zur Behandlung von cerebrovaskulären Störungen durch Mischen einer in einem der Ansprüche 1 bis 31 definierten Verbindung der Formel (I) oder eines pharmazeutisch geeigneten Salzes derselben mit einem pharmazeutisch geeigneten Träger oder Verdünnungsmittel.

## Revendications

**1.** Utilisation d'au moins un composé de formule (I) :

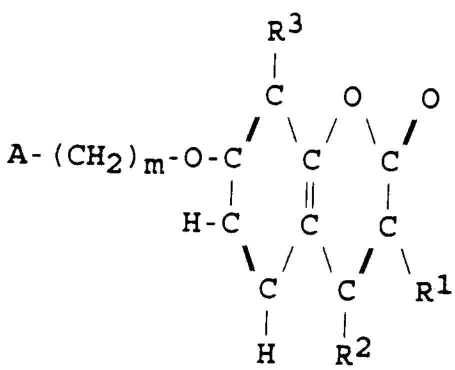

(I)

dans laquelle :
A représente un groupe de formule (II) ou (III) :

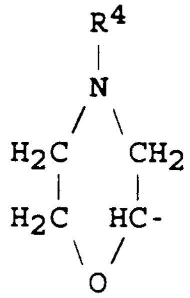

(II)

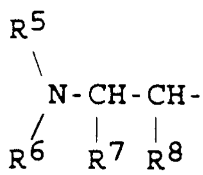

(III);

$R^1$, $R^2$ et $R^3$ sont identiques ou différents et représentent chacun un atome d'hydrogène, un groupe alkyle ayant 1 à 4 atomes de carbone ou un atome d'halogène ;
$R^4$ représente un atome d'hydrogène ou un groupe alkyle ayant 1 à 4 atomes de carbone, ledit groupe alkyle étant non substitué ou ayant au moins un substituant choisi parmi les groupes aryle ayant 6 à 10 atomes de carbone, ledit groupe aryle étant non substitué ou substitué par au moins un des substituants (a) définis ci-après ;
$R^5$ et $R^6$ sont identiques ou différents et représentent chacun un atome d'hydrogène ou un groupe alkyle ayant 1 à 4 atomes de carbone ;
$R^7$ et $R^8$ sont identiques ou différents et représentent chacun un atome d'hydrogène ou un groupe alkyle ayant 1 à 4 atomes de carbone ;
ou $R^7$ et $R^8$ ensemble représentent un groupe alkylène ayant 3 ou 4 atomes de carbone ; et
m est 1 ou 2 ;
substituants (a) :
groupes alkyle ayant 1 à 4 atomes de carbone, groupes alcoxy ayant 1 à 4 atomes de carbone, groupes trifluorométhyle, groupes nitro, groupes cyano et atomes d'halogène ;
ou d'un de ses sels pharmaceutiquement acceptables pour la préparation d'un médicament pour le traitement d'un trouble cérébro-vasculaire.

**2.** Utilisation selon la revendication 1, où ledit composé répond à la formule (Ia) :

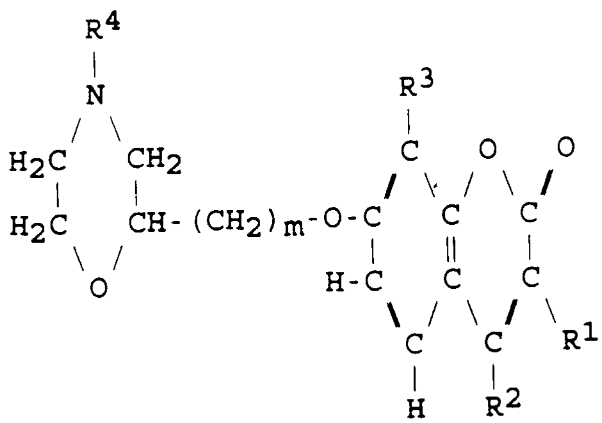

(Ia)

dans laquelle $R^1$, $R^2$, $R^3$, $R^4$ et m sont tels que définis dans la revendication 1.

**3.** Utilisation selon la revendication 2, où m est 1.

**4.** Utilisation selon la revendication 1, où ledit composé répond à la formule (Ib) :

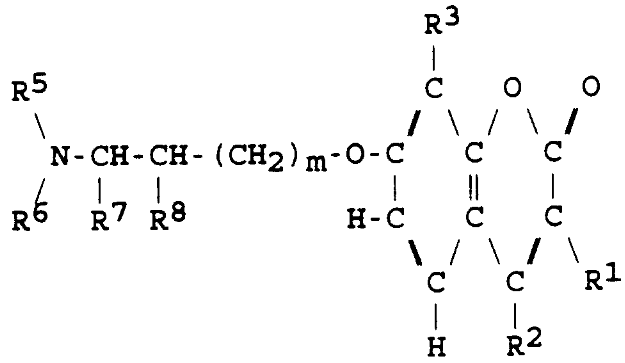

(Ib)

dans laquelle $R^1$, $R^2$, $R^3$, $R^5$, $R^6$, $R^7$, $R^8$ et m sont tels que définis dans la revendication 1.

**5.** Utilisation selon la revendication 4, où $R^7$ et $R^8$ représentent tous deux des atomes d'hydrogène.

**6.** Utilisation selon la revendication 2, où $R^1$ représente un atome d'hydrogène, un groupe méthyle, un groupe éthyle, un groupe propyle, un groupe isopropyle, un atome de fluor ou un atome de chlore.

**7.** Utilisation selon la revendication 2 ou la revendication 6, où $R^2$ représente un atome d'hydrogène, un groupe méthyle, un groupe éthyle, un groupe propyle ou un groupe isopropyle.

**8.** Utilisation selon l'une quelconque des revendications 2, 6 et 7, où $R^3$ représente un atome d'hydrogène, un groupe méthyle, un groupe éthyle ou un groupe isopropyle.

**9.** Utilisation selon l'une quelconque des revendications 2 et 6 à 8, où $R^4$ représente un atome d'hydrogène.

**10.** Utilisation selon la revendication 2, où : $R^1$ représente un atome d'hydrogène, un groupe méthyle, un atome de fluor ou un atome de chlore ; $R^2$ et $R^3$ sont identiques ou différents et représentent chacun un atome d'hydrogène ou un groupe méthyle ; et $R^4$ représente un atome d'hydrogène.

**11.** Utilisation selon la revendication 1, où m est 1.

**12.** Utilisation selon la revendication 4, où $R^5$ et $R^6$ représentent tous deux des atomes d'hydrogène.

**13.** Utilisation selon la revendication 4, où $R^5$ représente un atome d'hydrogène et $R^6$ représente un groupe alkyle contenant 1 à 4 atomes de carbone.

**14.** Utilisation selon la revendication 4, où $R^5$ représente un atome d'hydrogène et $R^6$ représente un groupe méthyle.

**15.** Utilisation selon la revendication 4, où $R^5$ et $R^6$ sont identiques ou différents et représentent chacun un groupe méthyle ou éthyle.

**16.** Utilisation selon l'une quelconque des revendications 4 et 12 à 15, où $R^1$ représente un atome d'hydrogène, un atome de chlore ou un groupe méthyle.

**17.** Utilisation selon l'une quelconque des revendications 4 et 12 à 16, où $R^2$ représente un atome d'hydrogène ou un groupe méthyle.

**18.** Utilisation selon l'une quelconque des revendications 4 et 12 à 17, où $R^3$ représente un atome d'hydrogène ou un groupe méthyle.

**19.** Utilisation selon la revendication 4, où :
$R^1$ représente un atome d'hydrogène, un atome de chlore ou un groupe méthyle ;
$R^2$ représente un atome d'hydrogène ou un groupe méthyle ;
$R^3$ représente un atome d'hydrogène ou un groupe méthyle ; et
$R^5$ et $R^6$ représentent tous deux des atomes d'hydrogène.

**20.** Utilisation selon la revendication 4, où :
$R^1$ représente un atome d'hydrogène, un atome de chlore ou un groupe méthyle ;
$R^2$ représente un atome d'hydrogène ou un groupe méthyle ;
$R^3$ représente un atome d'hydrogène ou un groupe méthyle ;
$R^5$ représente un atome d'hydrogène ; et
$R^6$ représente un groupe alkyle contenant 1 à 4 atomes de carbone.

**21.** Utilisation selon la revendication 4, où :
$R^1$ représente un atome d'hydrogène, un atome de chlore ou un groupe méthyle ;
$R^2$ représente un atome d'hydrogène ou un groupe méthyle ;
$R^3$ représente un atome d'hydrogène ou un groupe méthyle ;
$R^5$ représente un atome d'hydrogène ; et
$R^6$ représente un groupe méthyle.

**22.** Utilisation selon la revendication 4, où :
$R^1$ représente un atome d'hydrogène, un atome de chlore ou un groupe méthyle ;
$R^2$ représente un atome d'hydrogène ou un groupe méthyle ;
$R^3$ représente un atome d'hydrogène ou un groupe méthyle ; et
$R^5$ et $R^6$ sont identiques ou différents et représentent chacun un groupe méthyle ou éthyle.

**23.** Utilisation selon la revendication 4, où :
$R^1$, $R^2$ et $R^3$ représentent tous des groupes méthyle ;
$R^5$ et $R^6$ représentent tous deux des atomes d'hydrogène ; et
$R^7$ et $R^8$ sont identiques ou différents et représentent chacun un atome d'hydrogène, un groupe méthyle, un groupe éthyle ou un groupe isopropyle.

**24.** Utilisation selon la revendication 4, où :
$R^1$, $R^2$ et $R^3$ représentent tous des groupes méthyle ;
$R^5$ représente un atome d'hydrogène ;
$R^6$ représente un groupe méthyle ; et
$R^7$ et $R^8$ sont identiques ou différents et représentent chacun un atome d'hydrogène, un groupe méthyle, un groupe éthyle ou un groupe isopropyle.

**25.** Utilisation selon la revendication 4, où :
$R^1$, $R^2$ et $R^3$ représentent tous des groupes méthyle ;
$R^5$ et $R^6$ représentent tous deux des groupes méthyle ; et
$R^7$ et $R^8$ sont identiques ou différents et représentent chacun un atome d'hydrogène, un groupe méthyle, un groupe éthyle ou un groupe isopropyle.

**26.** Utilisation selon la revendication 1, où ledit composé est la 3,4,8-triméthyl-7-(2-morpholinyl)-méthoxycoumarine ou un sel pharmaceutiquement acceptable de celle-ci.

**27.** Utilisation selon la revendication 1, où ledit composé est la 3,4-diméthyl-7-(2-morpholinyl)-méthoxycoumarine ou un sel pharmaceutiquement acceptable de celle-ci.

**28.** Utilisation selon la revendication 1, où ledit composé est la 3,4,8-triméthyl-7-(4-méthyl-2-morpholinyl)-méthoxycoumarine ou un sel pharmaceutiquement acceptable de celle-ci.

**29.** Utilisation selon la revendication 1, où ledit composé est la 7-(3-N,N-diméthylaminopropoxy)-3,4,8-triméthylcoumarine ou un sel pharmaceutiquement acceptable de celle-ci.

**30.** Utilisation selon la revendication 1, où ledit composé est la 7-(3-aminopropoxy)-3,4,8-triméthylcoumarine ou un sel pharmaceutiquement acceptable de celle-ci.

**31.** Utilisation selon la revendication 1, où ledit composé est la 7-(3-aminobutoxy)-3,4,8-triméthylcoumarine ou un sel pharmaceutiquement acceptable de celle-ci.

**32.** Composé de formule (I) :

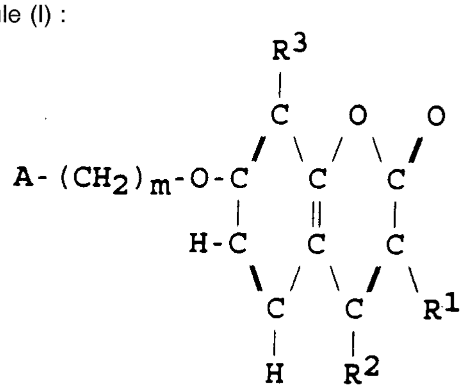

(I)

dans laquelle :
A représente un groupe de formule (II) ou (III) :

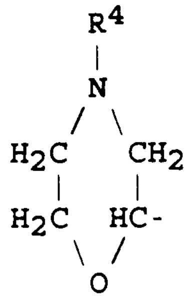

(II)

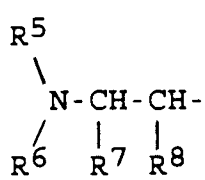

(III) ;

$R^1$, $R^2$ et $R^3$ sont identiques ou différents et représentent chacun un atome d'hydrogène, un groupe alkyle ayant 1 à 4 atomes de carbone ou un atome d'halogène ;
$R^4$ représente un atome d'hydrogène ou un groupe alkyle ayant 1 à 4 atomes de carbone, ledit groupe alkyle étant non substitué ou ayant au moins un substituant choisi parmi les groupes aryle ayant 6 à 10 atomes de carbone, ledit groupe aryle étant non substitué ou substitué par au moins un des substituants (a) définis ci-après ;
$R^5$ et $R^6$ sont identiques ou différents et représentent chacun un atome d'hydrogène ou un groupe alkyle ayant 1 à 4 atomes de carbone ;
$R^7$ et $R^8$ sont identiques ou différents et représentent chacun un atome d'hydrogène ou un groupe alkyle ayant 1 à 4 atomes de carbone ;
ou $R^7$ et $R^8$ ensemble représentent un groupe alkylène ayant 3 ou 4 atomes de carbone ; et
m est 1 ou 2 ;
substituants (a) :
groupes alkyle ayant 1 à 4 atomes de carbone, groupes alcoxy ayant 1 à 4 atomes de carbone, groupes trifluorométhyle, groupes nitro, groupes cyano et atomes d'halogène ;
et ses sels pharmaceutiquement acceptables,
sous réserve que $R^5$ et $R^6$ ne soient pas identiques et soient un groupe méthyle ou un groupe éthyle

lorsque : A représente un groupe de formule III, $R^1$, $R^3$, $R^7$ et $R^8$ représentent tous des atomes d'hydrogène, $R^2$ représente un groupe méthyle et m est 1.

**33.** Composé selon la revendication 32, qui répond à la formule (Ia) :

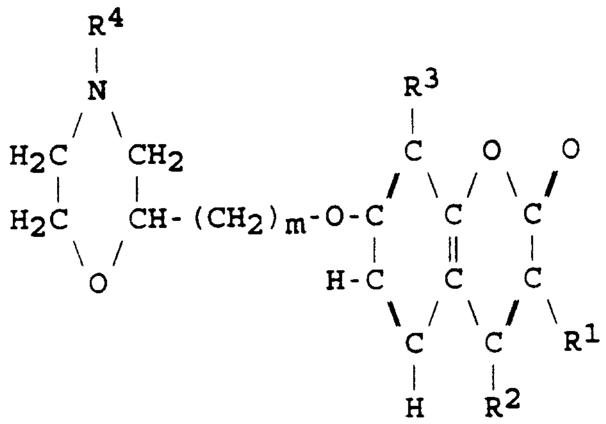

(Ia)

dans laquelle $R^1$, $R^2$, $R^3$, $R^4$ et m sont tels que définis dans la revendication 32.

**34.** Composé selon la revendication 33, où m est 1.

**35.** Composé selon la revendication 32 qui répond à la formule (Ib) :

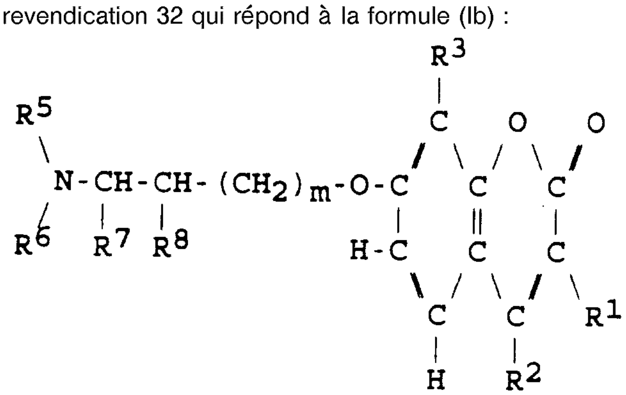

(Ib)

dans laquelle $R^1$, $R^2$, $R^3$, $R^5$, $R^6$, $R^7$, $R^8$ et m sont tels que définis dans la revendication 32.

**36.** Composé selon la revendication 35, où $R^7$ et $R^8$ représentent tous deux des atomes d'hydrogène.

**37.** Composé selon la revendication 33, où $R^1$ représente un atome d'hydrogène, un groupe méthyle, un groupe éthyle, un groupe propyle, un groupe isopropyle, un atome de fluor ou un atome de chlore.

**38.** Composé selon la revendication 33 ou la revendication 37, où $R^2$ représente un atome d'hydrogène, un groupe méthyle, un groupe éthyle, un groupe propyle ou un groupe isopropyle.

**39.** Composé selon l'une quelconque des revendications 33, 37 et 38, où $R^3$ représente un atome d'hydrogène, un groupe méthyle, un groupe éthyle ou un groupe isopropyle.

**40.** Composé selon l'une quelconque des revendications 33 et 37 à 39, où $R^4$ représente un atome d'hydrogène.

**41.** Composé selon la revendication 33, où :
$R^1$ représente un atome d'hydrogène, un groupe méthyle, un atome de fluor ou un atome de chlore ;
$R^2$ et $R^3$ sont identiques ou différents et représentent chacun un atome d'hydrogène ou un groupe méthyle ; et

$R^4$ représente un atome d'hydrogène.

**42.** Composé selon la revendication 32, où m est 1.

**43.** Composé selon la revendication 35, où $R^5$ et $R^6$ représentent tous deux des atomes d'hydrogène.

**44.** Composé selon la revendication 35 ou la revendication 43, où $R^5$ représente un atome d'hydrogène et $R^6$ représente un groupe alkyle contenant 1 à 4 atomes de carbone.

**45.** Composé selon l'une quelconque des revendications 35, 43 et 44, où $R^5$ représente un atome d'hydrogène et $R^6$ représente un groupe méthyle.

**46.** Composé selon l'une quelconque des revendications 35 et 43 à 45, où $R^5$ et $R^6$ sont identiques ou différents et représentent chacun un groupe méthyle ou éthyle.

**47.** Composé selon l'une quelconque des revendications 35 et 43 à 46, où $R^3$ représente un atome d'hydrogène ou un groupe méthyle.

**48.** Composé selon l'une quelconque des revendications 35 et 43 à 47, où $R^2$ représente un atome d'hydrogène ou un groupe méthyle.

**49.** Composé selon l'une quelconque des revendications 35 et 43 à 48, où $R^1$ représente un atome d'hydrogène, un atome de chlore ou un groupe méthyle.

**50.** Composé selon la revendication 35, où :
$R^1$ représente un atome d'hydrogène, un atome de chlore ou un groupe méthyle ;
$R^2$ représente un atome d'hydrogène ou un groupe méthyle ;
$R^3$ représente un atome d'hydrogène ou un groupe méthyle ; et
$R^5$ et $R^6$ représentent tous deux des atomes d'hydrogène.

**51.** Composé selon la revendication 35, où :
$R^1$ représente un atome d'hydrogène, un atome de chlore ou un groupe méthyle ;
$R^2$ représente un atome d'hydrogène ou un groupe méthyle ;
$R^3$ représente un atome d'hydrogène ou un groupe méthyle ;
$R^5$ représente un atome d'hydrogène ; et
$R^6$ représente un groupe alkyle contenant 1 à 4 atomes de carbone.

**52.** Composé selon la revendication 35, où :
$R^1$ représente un atome d'hydrogène, un atome de chlore ou un groupe méthyle ;
$R^2$ représente un atome d'hydrogène ou un groupe méthyle ;
$R^3$ représente un atome d'hydrogène ou un groupe méthyle ;
$R^5$ représente un atome d'hydrogène ; et
$R^6$ représente un groupe méthyle.

**53.** Composé selon la revendication 35, où :
$R^1$ représente un atome d'hydrogène, un atome de chlore ou un groupe méthyle ;
$R^2$ représente un atome d'hydrogène ou un groupe méthyle ;
$R^3$ représente un atome d'hydrogène ou un groupe méthyle ; et
$R^5$ et $R^6$ sont identiques ou différents et représentent chacun un groupe méthyle ou éthyle.

**54.** Composé selon la revendication 35, où :
$R^1$, $R^2$ et $R^3$ représentent tous des groupes méthyle ;
$R^5$ et $R^6$ représentent tous deux des atomes d'hydrogène ; et
$R^7$ et $R^8$ sont identiques ou différents et représentent chacun un atome d'hydrogène, un groupe méthyle, un groupe éthyle ou un groupe isopropyle.

**55.** Composé selon la revendication 35, où :
$R^1$, $R^2$ et $R^3$ représentent tous des groupes méthyle ;

$R^5$ représente un atome d'hydrogène ;
$R^6$ représente un groupe méthyle ; et
$R^7$ et $R^8$ sont identiques ou différents et représentent chacun un atome d'hydrogène, un groupe méthyle, un groupe éthyle ou un groupe isopropyle.

**56.** Composé selon la revendication 35, où :
$R^1$, $R^2$ et $R^3$ représentent tous des groupes méthyle ;
$R^5$ et $R^6$ représentent tous deux des groupes méthyle ; et
$R^7$ et $R^8$ sont identiques ou différents et représentent chacun un atome d'hydrogène, un groupe méthyle, un groupe éthyle ou un groupe isopropyle.

**57.** Composés de la revendication 32 choisis parmi :
la 3,4,8-triméthyl-7-(2-morpholinyl)méthoxycoumarine ;
la 3,4-diméthyl-7-(2-morpholinyl)méthoxycoumarine;
la 3,4,8-triméthyl-7-(4-méthyl-2-morpholinyl)méthoxycoumarine ;
la 7-(3-N,N-diméthylaminopropoxy)-3,4,8-triméthylcoumarine ;
la 7-(3-aminopropoxy)-3,4,8-triméthylcoumarine ; et
la 7-(3-aminobutoxy)-3,4,8-triméthylcoumarine ;
et leurs sels pharmaceutiquement acceptables.

**58.** Composition pharmaceutique pour le traitement des troubles cérébro-vasculaires, comprenant une quantité thérapeutiquement efficace d'au moins un composé de formule (I) ou d'un sel pharmaceutiquement acceptable de celui-ci, tel que défini dans l'une quelconque des revendications 32 à 57, en mélange avec un véhicule, diluant ou excipient pharmaceutiquement acceptables.

**59.** Procédé pour préparer un composé de formule (I) selon l'une quelconque des revendications 32 à 57, lequel procédé comprend les étapes de :
(a) réaction d'un composé de formule (IV) :

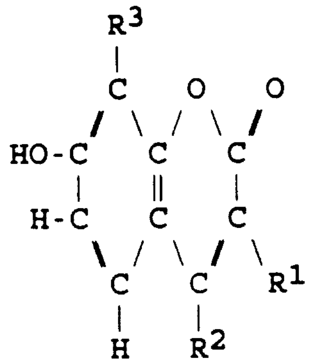

(IV)

(dans laquelle : $R^1$, $R^2$ et $R^3$ sont tels que définis dans la revendication 1) avec un composé de formule (V) :

$Y\text{-}(CH_2)_m\text{-}A'$ (V)

[dans laquelle : m est tel que défini dans la revendication 1 ; Y représente un atome d'halogène, un groupe sulfonyloxy ou un groupe hydroxy ; et A' représente un groupe de formule (II') ou (III') :

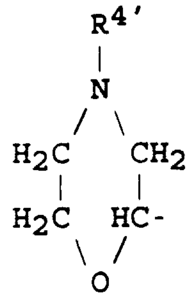

(II')

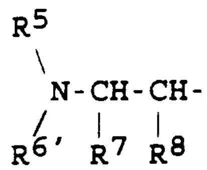

(III')

(dans lesquelles : $R^5$, $R^7$ et $R^8$ sont tels que définis dans la revendication 1 ; $R^{4'}$ représente un groupe amino-protecteur ou un groupe alkyle ayant 1 à 4 atomes de carbone, ledit groupe alkyle étant non substitué ou ayant au moins un substituant choisi parmi les groupes aryle ayant 6 à 10 atomes de carbone, où ledit groupe aryle est non substitué ou est substitué par au moins un des substituants (a) définis dans la revendication 1 ; et $R^{6'}$ représente un groupe amino-protecteur ou un groupe alkyle ayant 1 à 4 atomes de carbone)] ; et

si besoin est, une des étapes (b) ou (c) :

(b) lorsque A' représente ledit groupe de formule (II') et $R^{4'}$ représente un groupe amino-protecteur ou A' représente ledit groupe de formule (III') et $R^{6'}$ représente un groupe amino-protecteur, l'élimination du groupe amino-protecteur ;

(c) lorsque le produit est un composé dans lequel l'un de $R^5$ et de $R^6$ ou les deux représentent un atome d'hydrogène, l'alkylation de l'atome d'hydrogène pour le transformer en un groupe alkyle ayant 1 à 4 atomes de carbone ;

et, facultativement, la salification du produit.

**60.** Procédé pour préparer une composition pharmaceutique pour le traitement des troubles cérébro-vasculaires par mélange d'un composé de formule (I), tel que défini dans l'une quelconque des revendications 1 à 31, ou d'un sel pharmaceutiquement acceptable de celui-ci, avec un véhicule ou diluant pharmaceutiquement acceptables.